# EUROPEAN PATENT SPECIFICATION

(11) **EP 3 503 862 B1**
(45) Date of publication and mention of the grant of the patent: **22.07.2026**
(21) Application number: 17851290.1
(22) Date of filing: 23.08.2017
(51) Int. Cl.: A61H 9/00, A61F 13/08

(54) **THERAPEUTIC COMPRESSION APPARATUS AND METHODS OF USE**
THERAPEUTISCHE KOMPRESSIONSVORRICHTUNG UND VERFAHREN ZUR VERWENDUNG
APPAREIL DE COMPRESSION THÉRAPEUTIQUE ET PROCÉDÉS D'UTILISATION

(30) Priority: 23.08.2016 US 201662378581 P
(43) Date of publication of application: 03.07.2019
(73) Proprietor: Sun Scientific, Inc., Dobbs Ferry, NY 10522 (US)
(72) Inventor: RAVIKUMAR, Sundaram, Dobbs Ferry NY 10522 (US); RAVIKUMAR, Vikram, Dobbs Ferry NY 10522 (US); OSBORNE, Guy, Dobbs Ferry NY 10522 (US); NOLAN, Timothy, Dobbs Ferry NY 10522 (US)
(74) Representative: AWA Denmark A/S
(86) International application number: PCT/US2017/048241
(87) International publication number: WO 2018/052676

(56) References cited:
- WO-A1-2015/198064
- US-A1- 2009 069 731
- US-A1- 2009 124 944
- US-A1- 2009 204 037
- US-A1- 2012 316 480
- US-A1- 2014 171 889
- US-A1- 2015 245 975
- US-A1- 2016 008 204

## Description

### RELATED APPLICATIONS

The present application claim priority from provisional application Serial No. 62/378,581 filed August 23, 20016, and priority from co-pending application and this PCT application is a continuation-in-part of U.S. patent application Serial No. 13/444,600 which is a divisional of U.S. patent application Serial No. 12/855,computer185 entitled "Therapeutic Compression Apparatus" filed on August 12, 2010 and issued as Patent No. 9,033,906.

### BACKGROUND OF THE INVENTION

### I. Field of the Invention

The subject invention is directed generally to a device for applying compression to a limb, and more particularly, to a therapeutic apparatus for applying compression to the leg of an individual in conjunction with the treatment of conditions such as chronic venous insufficiency and lymphedema. The apparatus includes a wrap having a bladder and an inflation means for the bladder with the apparatus capable of applying compression to a limb such as the lower leg and foot of a patient.

### II. Background of the Related Art

Normally, a healthy leg muscle, for example, squeezes the deep veins of the legs and feet to help move blood back to the heart. One-way valves in the deep leg veins keep blood flowing back towards the heart. However, prolonged periods of standing or sitting can cause the walls of the deep leg veins to stretch. Over time, in susceptible individuals, this can weaken the vein walls and damage the valves, causing blood to pool in the veins and increase venous blood pressure. This may result in a condition known as chronic venous insufficiency (CVI).

Treatment of CVI typically involves the use of compression stockings or medical hosiery to decrease chronic swelling. Compression stockings are elastic stockings that squeeze the veins to improve venous circulation and prevent excess blood from flowing backward. Compression stockings can also help to heal skin sores or stasis ulcers that often present in conjunction with CVI. It is also common to employ compression bandages to apply pressure to the leg. In this regard, a bandage is applied with constant tension so as to produce graduated compression with the highest pressure at the ankle. However, the technique is difficult and is often done by highly skilled caregivers.

Highly effective mechanical compression devices have also been developed for treating CVI, which are disclosed, for example, in U.S. Pat. Nos. 7,276,037 and 7,559,908 and U.S. Pat. Application 2012/0316480 and U.S. Pat. Application 2015/024975. These devices include a flexible wrap that carries a manually inflatable air bladder and is adapted to be securely positioned around the leg of an individual to apply localized pressure to a treatment site. The device also includes a fluid-filled wound dressing that can be applied directly to the skin for applying localized pressure and even a medicament to a venous ulcer when it is enveloped by the flexible wrap. While this device is effective for applying localized compression to the leg, it is not configured to apply localized compression to the foot to prevent swelling and further improve venous circulation.

Lymphedema, also known as lymphatic obstruction, is another condition of localized fluid retention and tissue swelling, and is caused by a compromised lymphatic system. Treatment for lymphedema varies depending on the severity of the edema and the degree of fibrosis of the affected limb. The most common treatments for lymphedema are manual compression lymphatic massage, compression garments or bandaging. Elastic compression garments are typically worn by persons with lymphedema on the affected limb following complete decongestive therapy to maintain edema reduction.

Compression bandaging, also called wrapping, involves the application of several layers of padding and short-stretch bandages to the involved areas. Short-stretch bandages are preferred over long-stretch bandages (such as those normally used to treat sprains), as the long-stretch bandages cannot produce the proper therapeutic tension necessary to safely reduce lymphedema and may in fact end up producing a tourniquet effect. During activity, whether exercise or daily activities, the short-stretch bandages enhance the pumping action of the lymph vessels by providing increased resistance for them to push against. This encourages lymphatic flow and helps to soften fluid-swollen areas.

Known methods for CVI and lymphedema treatment, like compression bandaging, have several disadvantages. The bandaging is time consuming and the effectiveness is limited to the skill of the provider. In some instances, bandages can be applied too tightly or too loosely and may slip from their intended position, decreasing their effectiveness. When this occurs, bandages must be taken off and reapplied, further increasing the time of application and decreasing the consistency of application of the therapy.

The effectiveness of many of the current compression therapies is limited by the application of current products. Because current compression therapy is done either with manual wraps or electromechanical systems, they require either a skilled medical processional to apply and/or the need for the patient to be stationary for extended periods of time. Although stockings and/or bandages can be worn by patients and self-administered, they are very difficult for the patient to put on and pose a challenge for unskilled medical professionals to apply consistently and effectively.

Further, many of the current treatment options for CVI and lymphedema cause venous ulcers including the use of current known devices, apparatus, bandages, stocking, hosiery and the like. A venous ulcer is damage and loss of skin above the ankle that is the result of a problem with the veins in the leg. Venous ulcers typically develop on either side of the lower leg, above the ankle and below the calf. They are difficult to heal and often recur.

The veins of the leg are divided into the superficial and deep systems according to their position relative to the fascia. The deep veins, which come together to form the popliteal and femoral veins lie within the fascia and are responsible for the venous return from the leg muscles. Dilated valveless sinusoids also lie within the fascia (more particularly in the soleus and gastrocnemius muscles). The sinusoids fill with blood when the leg is at rest.

The long saphenous vein which runs along the medial side of the leg from foot to groin and the short saphenous vein which runs at the back of the calf from foot to knee are the major vessels of the superficial venous system. These vessels lie outside the fascia and are responsible for the venous return from the skin and subcutaneous fat.

Communicating veins, sometimes called perforators because they perforate the deep fascia, join the two systems. The perforators, like the other veins in the leg, contain valves that permit the flow of blood in one direction only, from the outer or superficial system inwards to the deep veins.

The venous pressure at the ankle of a subject who is lying supine is around 10 mmHg, but on standing this will rise considerably due to an increase in hydrostatic pressure (equivalent to the weight of a vertical column of blood stretching from the point of measurement to the right auricle of the heart).

During walking, as the foot is dorsally flexed, the contraction of the calf muscle compresses the deep veins and soleal sinuses thereby emptying them of blood. As the foot is plantarly flexed, the pressure in the veins falls, the proximal valves close, and the veins are refilled by blood passing through the perforators from the superficial system. During this cycle, in a normal leg, the distal valves of the deep veins and the valves of the perforators will ensure that the expelled blood can go in only one directionupwards, back to the heart.

Blockage or damage to the venous system will cause disruption to normal blood flow, which may manifest itself in a number of different ways according to the site and extent of the damage. If the valves in the superficial system are affected, venous return will be impaired and blood may accumulate in the veins causing them to become distended, leading to the formation of varicosities (varicose veins).

If the function of the perforator valves is impaired, the action of the calf muscle pump will tend to cause blood to flow in the reverse direction into the superficial system increasing the possibility of damage to the superficial vessels.

Following a deep vein thrombosis that results in complete or partial obstruction of a deep vein, the unrelieved pressure produced by the calf muscle pump on the perforator valves may cause these to become incompetent. If this occurs, there will be a large rise in the pressure in the superficial system, which may force proteins and red cells out of the capillaries and into the surrounding tissue. Here, the red cells break down releasing a red pigment that causes staining of the skin, an early indicator of possible ulcer formation.

Venous leg ulcers are generally shallow and red in color. The skin surrounding the ulcer is frequently discolored due to the staining described previously. Incompetent perforating vein valves can also cause malleolar venules to become dilated and appear as fine red threads around the ankle. This condition, called ankle flair, is also diagnostic of a venous ulcer.

Arteries transport oxygen replenished blood from the heart to the rest of the body. Veins return oxygen depleted blood back to the heart. When the veins in the lower extremities of the body have difficulty transporting blood back to the heart, a condition develops called chronic venous insufficiency (CVI), also known as chronic venous disease (CVD). CVI most commonly occurs as the result of a blood clot in the deep veins of the legs, a disease known as deep vein thrombosis (DVT). CVI also results from pelvic tumors and vascular malformations, and sometimes occurs for unknown reasons. When a person is standing or sitting, blood in the veins of the legs flows in an upward direction. When the person walks, the calf muscles and muscles in the feet contract to squeeze the veins and push the blood upward. To keep the blood flowing upward and prevent it from flowing downward, the veins contain one-way valves. CVI occurs when these valves become damaged and allow the blood to leak back downward in the opposite direction. Such valve damage may occur as the result of aging, extended sitting or standing, or a combination of aging and reduced mobility. When the veins and valves become weakened and the blood does not properly flow up to the heart, blood pressure in the veins of the lower extremities can stay elevated for long periods of time, leading to CVI. This condition is more common in older individuals, and if not properly treated, can lead to burst capillaries, local tissue inflammation, internal tissue damage, varicose veins, ulcers, and open sores on the skin's surface.

CVI can diminish the capacity of the venous system and increase the workload of the lymphatic system in the affected area. The lymphatic system must then transport larger volumes of water and protein to reduce the fluid load in the affected tissues of the legs, a situation which is especially difficult for patients with Lymphedema, varicose veins, and other lower extremity pathology.

One non-surgical option often used to help prevent or treat the lower extremity pathologies discussed above is the use of compression stockings. Compression stockings help prevent leg fatigue, ankle and foot swelling, spider veins, and varicose veins. They improve circulation in the legs, especially when used in conjunction with frequent exercise and leg elevation. Compression stockings maintain pressure on the legs while allowing for normal ambulation. Increasing pressure in the tissues beneath the skin reduces excess leakage of fluid from the capillaries and increases absorption of tissue fluid by the capillaries and lymphatic vessels. In addition, the increased pressure decreases the size of the veins, which causes the blood to flow faster and help prevent it from pooling.

Compression stocking tightness typically varies between 15-50 mm HG. The tightness of a given stocking depends on its particular configuration and class. For example, stockings having a compression pressure of 15-20 mm HG are considered light compression stockings. Class I stockings are 20-30 mm Hg, class II stockings are 30-40 mm Hg, and class III stockings are 40-50 mm Hg.

While such compression stockings are a commonly utilized non-invasive treatment of lower leg pathology, the issues they present are numerous. Wearing a tightly fitting stocking can be tedious or time consuming to put on, and may require help from another person if the wearer is injured, elderly, or has some form of disability. In addition, the pressure applied by the stocking generally stays relatively constant during use without any option of increasing or decreasing the tightness level. As compression stockings are repeatedly worn, they lose elasticity and thus tightness over time. Once such prescribed elasticity and tightness is lost, the stocking is of little or no value, and needs to be replaced on account of its looseness, which requires buying a new pair to obtain the desired pressure.

Medical hosiery represents a useful and convenient method of applying compression to normal shaped legs in order to prevent the development or recurrence of leg ulcers. However, these stockings are of limited value in the treatment of active ulceration, being difficult to apply over dressings. In such situations compression bandages currently represent the treatment of choice. Compression bandages apply a pressure to the limb that is directly proportional to bandage tension but inversely proportional to the radius of curvature of the limb to which it is applied. This means, therefore, that a bandage applied with constant tension to a limb of normal proportions will automatically produce graduated compression with the highest pressure at the ankle. This pressure will gradually reduce up the leg as the circumference increases.

As can be readily appreciated, it is cumbersome and difficult to apply uniform tension to the compression bandage as it is applied to the treated limb, and thus this is accomplished only by highly skilled caregivers. Moreover, once secured to the treated limb, care and attention must be given to ensure that the bandage does not slip or become displaced as this will lead to multiple layers forming, which in turn may lead to localized areas of high pressure, which can place the patient in direct risk of skin necrosis.

Mechanical compression treatments have also been proposed. An exemplary compression device is described in U.S. Pat. No. 5,031,604 to Dye. As generally described at col. 2, lines 33 et seq., an arrangement of chambers are provided that circumscribe the leg. An active pneumatic control system controls the pressure in the chambers to squeeze the leg near the ankle and then squeeze sequentially upward toward the knee in order to move blood from the extremity toward the heart. As noted in col. 4, lines 20-59 of U.S. Pat. No. 6,488,643 to Tumey et al., the mechanically produced compression levels may produce ischaemic (i.e., localized tissue anemia) not noted at similar compression levels obtained through bandaging. It may also produce cuffing (i.e., a reduction in leg pulsatile blood flow). The pneumatic control system is also bulky and heavy, which severely limits the mobility of the patient during treatment. Moreover, the pneumatic control system fails to provide a mechanism to ensure that excessive pressure, which can cause necrosis, is not applied to the treated limb. These limitations have resulted in most mechanical compression devices being contraindicated for patients exhibiting deep-vein thrombosis. Consequently, those skilled in the art have to date avoided such mechanical compression devices for the treatment of venous ulcers or edema of the extremities.

Co-owned U.S. Publication No. 2004/0193084, discloses a device for applying pressure to the human leg for use in conjunction with treatment of varicose veins. The device includes a flexible member and at least one air bladder chamber integral thereto that are adapted to securely wrap around the human leg. A tube in fluid communication with the air bladder chamber(s) extends to an air pumping mechanism that operates to inflate the air bladder chamber(s) to a pressurized state. The flexible member preferably includes an opening at the knee joint level to enable a patella to protrude therethrough. In addition, the flexible member preferably extends below knee joint level and is adapted to securely wrap around a lower portion of a leg to provide stability to the leg. Preferably, the air bladder chamber of the device is substantially longer in a first dimension than in a second dimension orthogonal thereto such that the air bladder chamber can be positioned to cover a portion of the human leg that is relatively long in the vertical dimension and narrow in the horizontal dimension.

Co-owned U.S. Pat. No. 7,276,037, discloses an apparatus for applying compression therapy to an extremity of the human body, such as a portion of the human leg. The device includes a flexible member and an air bladder chamber. The flexible member is adapted to wrap around the extremity to secure the air bladder chamber to the extremity. An air pumping mechanism is operated to inflate the air bladder chamber to a pressurized state. One or more fluid-filled pressurized members are provided, each separate and distinct from the flexible member and the air bladder chamber and thus readily moveable relative to the flexible member and the air bladder chamber. The pressurized member(s) is operably disposed between the extremity and the flexible member whereby it applies increased localized pressure to the extremity during use. Preferably, the air bladder chamber is substantially longer in a first dimension than in a second dimension orthogonal thereto such that it can extend longitudinally along the extremity to cover a relatively long and narrow portion of the extremity. The position of the air chamber can be readily adapted to apply local pressure to desired body parts (such as a certain venous channel). The pressurized member(s) can be positioned during use such that it covers a venous ulcer (or other treatment sites) and applies increased localized pressure to the treatment site in order to promote healing.

All current known treatment apparatus, devices, bandages, stockings and hosiery have the problems of stability, maintaining sufficient effective pressure without overpressure complications, maintaining compression and the like. Further all known apparatus, devices, bandages, stockings and hosiery, though especially the current treatment apparatus and devices, are only capable of connecting to one source of compression or inflation means and no universal inflation port of connector is known - wherein a patient could vary treatment through varying the inflation source and inflation means for the treatment apparatus or device.

Other known problems with the current treatment apparatus and devices, bandages, stockings and hosiery is the requirement that a skilled care-giver apply the current treatment apparatus and devices, bandages, stockings and hosiery. Such a skilled care-giver may not be available to all patients, notably those without long-term care insurance or provided a skilled home-health aid. Yet another known problem is leakage of set compression within the treatment apparatus and devices, bandages, stockings and hosiery resulting in an ineffective treatment and ineffective apparatus or device and the like which may be rendered useless to the patient and user. A further problem with the current treatment apparatus and devices, bandages, stockings and hosiery is that the inflation means or source of compression is set up as either manual or mechanical or electrical and cannot be interchanged in that the inflation port or inflation means is not universal and interchangeable. Yet another problem with the current treatment apparatus and devices, bandages, stockings and hosiery is that the inflation means or source is either static or intermittent and again cannot be changed during the treatment with such apparatus or device.

The apparatuses, methods, assemblies and systems of the subject invention provide benefits and advantages that may overcome a number of problems with respect to known compression technologies, particularly the problems that arise due to the difficulty of applying current compression wrap technologies. The subject invention provides an alternative to known technologies that employ tight-fitting therapeutic elastic garments, which cause patients discomfort, and lose their elasticity and therefore their effectiveness over time. Those skilled in the art will readily appreciate that it would be beneficial to provide a therapeutic compression device for treating CVI, **DVT** and lymphedema that is adapted and configured to apply localized compression to the leg and foot to prevent swelling and further improve venous circulation, that may also be self-administered by a patient effectively.

### SUMMARY OF THE INVENTION

The present invention is directed to a therapeutic compression apparatus according to claim 1. Additional features and embodiments of the invention are defined in the dependent claims.

### SUMMARY OF THE DISCLOSURE

The therapeutic compression apparatus comprises: a primary wrap and a secondary wrap and optionally a stirrup. The primary wrap encircles at least a first portion of a limb such as a leg and applies compression thereto. The primary wrap has a horizontal proximal edge for positioning towards for instance a knee of the leg, a horizontal distal edge for positioning towards for instance an ankle of the leg, and first and second peripheral edges perpendicular to the horizontal proximal edge and the horizontal distal edge. The secondary wrap encircles at least a portion of a limb such as a foot of the leg to apply compression thereto. The stirrup is integrated with the primary wrap along the horizontal distal edges for securing the primary wrap to the limb such as a leg, the stirrup being positioned between the primary wrap and the secondary wrap.

The therapeutic compression apparatus may further comprise at least one bladder operatively associated with the primary wrap for applying pressure to a treatment site on the limb such as a leg. The primary wrap may include at least one interior pocket for accommodating the at least one bladder. Alternatively, the at least one bladder may be integral with the primary wrap. One or more means for attaching the primary wrap may be operatively associated along the first and second peripheral edges of the primary wrap for securing the primary wrap around the limb.

The at least one bladder may be adapted and configured to form a predetermined gradient pressure profile when the at least one bladder is filled. The at least one bladder may be one of a wedge-shaped bladder, a cone-shaped bladder, a disk-shaped bladder or a rectangular-shaped bladder. The at least one bladder may also include a plurality of fluid chambers. The therapeutic compression apparatus may further comprise at least one means for adjusting pressure coupled to the at least one bladder for controlling an amount of pressure supplied to the treatment site by the primary wrap.

The secondary wrap may be attached to the stirrup. The secondary wrap may be configured to envelope a limb such as the toes of the foot and/or configured to envelope a heel of the foot. The secondary wrap may also be configured as an adjustable strap around the foot.

The therapeutic compression apparatus may further comprises an adjustable belt along a proximal horizontal edge of the primary wrap for securing the primary wrap around the limb such as for instance the leg. The primary wrap may be formed at least in part of a non-elastic composite material comprising a plurality of distinct layers. In one embodiment, the composite material may comprises three distinct layers: an inner laminate layer, an outer hook-compatible layer, and a middle non-elastic layer provided between the inner and outer layers. The composite material may also be provided with a plurality of stitched darts and gathers for contouring the primary wrap to the limb such as for instance the leg.

The subject invention is also directed to a bladder assembly for a compression apparatus for providing pressure to a limb. The bladder assembly comprises: at least one bladder having first and second flexible walls secured to one another about a peripheral edge thereof to form an air pocket; and at least one spot weld provided in a predetermined location inward of the peripheral edge connecting the first and second walls to one another to define a plurality of chambers within the bladder. The geometric placement of the at least one spot weld determines a pressure profile of the at least one bladder.

An inflation means for inflating the bladder such as the air pocket through at least one inflation port may be provided in the first wall of the bladder assembly. The inflation means may be detachable from the at least one inflation port. At least one pressure valve may be operatively associated with the inflation means for controlling an amount of pressure within the bladder and the air pocket within the bladder. The inflation port includes a check vale so as to maintain a given pressure within the bladder of the therapeutic compression apparatus. The inflation port is universal in that it is configured to be capable of connecting to and accepting a plurality of inflation sources and inflation means such as a manual pump, mechanical pump, electrical pump, battery-operated pump, static pump, intermittent pump, pneumatic pump, negative pressure source and other variations.

A method not forming part of the invention includes the therapeutic treatment apparatus used to treat CVI , DVT and/or lymphedema by applying the primary and secondary wraps around a limb by a patient and inserting an inflation means into an inflation port and inflating the bladders within the primary and secondary wraps and maintaining a certain pressure to treat the CVI, DVT and/or lymphedema.

Another embodiment of the present invention includes an assembly according to the invention includes a pressure mechanism having a flexible member for attachment to a limb and an air chamber which may be pumped up into a desired pressurized state, a separate relatively small pre-filled air bladder, an absorbent foam, sponge or dressing coupled to the pre-filled air bladder, and a suction conduit coupled to a source of negative pressure (suction) and in fluid communication with the absorbent foam, sponge or dressing. In a preferred embodiment, the pre-filled air bladder, the absorbent foam, sponge or dressing and the suction conduit are formed together as a unit.

According to one aspect of the invention, the flexible member of the pressure mechanism is adapted to wrap around a leg or arm and over the pre-filled air bladder in order to secure the pre-filled air bladder and the foam, sponge or dressing to a wound or ulcer in the extremity. Thus, the flexible member is provided with some fixation structure such as a hook and loop closure mechanism. An air pumping mechanism is preferably coupled to the air chamber of the pressure mechanism in order to inflate the air chamber to a pressurized state. The air chamber of the pressure mechanism is preferably designed to apply pressure along a predefined area (e.g., the saphenous vein of a leg) as opposed to around an entire limb.

According to another aspect of the invention, the suction conduit is located either between the pre-filled air bladder and the absorbent foam, sponge or dressing which is adhered to the small air bladder, or the pre-filled air bladder is formed as a donut with a central opening and the suction conduit extends through the central opening. By coupling the suction conduit to a source of negative pressure, exudate from the wound or ulcer is sucked through the foam, sponge or dressing into the suction conduit.

One of the methods not forming part of the invention include locating the pre-filled air bladder and foam, sponge or dressing over a wound or ulcer on a limb, wrapping the flexible member of the pressure mechanism around a limb with the air chamber located over the pre-filled air bladder/absorbent foam, sponge or dressing, and fastening the pneumatic pressure mechanism in place with the fixation structure. When the apparatus is properly located and affixed to the limb, the air chamber is inflated, preferably to 30-40 mm Hg, thereby applying pressure to the limb and more specifically via the pre-filled air bladder to the wound. The suction apparatus is activated by turning on the source of negative pressure, and exudate from the wound or ulcer is pulled through the absorbent foam, sponge or dressing into the suction conduit.

Another embodiment of the present invention includes an apparatus for applying intermittent pressure to a portion of the human body, such as an area of the human leg, which assists with the healing and treatment of various conditions such as venous ulcers or wounds by promoting blood flow into and out of the area and by increasing drainage. The apparatus includes a foot bladder and a leg bladder, each having inflatable chambers that accommodate an entering fluid by inflating. The bladders are fluidly coupled by a fluid conduit, and each is preferably equipped with a means for locating it on a portion of the body. In a preferred embodiment, the foot bladder is positioned on a bottom of a foot and the leg bladder is positioned on a lower portion of a leg. As a person walks while wearing the apparatus, a portion of the foot bladder deflates as the person's foot (heel) strikes the ground due to the external pressure placed on the foot bladder, thereby forcing fluid out of the foot bladder, through the fluid conduit, and into the leg bladder, which raises the pressure therein. As the person's foot rolls from heel to toe in the standard walking motion, the external pressure from the person's weight is removed from the foot bladder, resulting in the pressure of the leg bladder being higher than the pressure in the foot bladder. Fluid thus flows back through the fluid conduit and into the foot bladder, which then inflates again to its original state, such that the pressures of the foot bladder and leg bladder are equalized. This process repeats as a person walks, thereby creating a pumping or kneading force on the leg as the pressure in the leg bladder intermittently increases and decreases, thereby promoting blood flow, fluid drainage, treatment, and healing to various parts of the leg.

These and other aspects of the contacts of the subject invention will become more readily apparent from the following description taken in conjunction with the drawings.

### BRIEF DESCRIPTION OF THE DRAWINGS

So that those having ordinary skill in the art to which the subject invention pertains will more readily understand how to make and use the apparatuses of the subject invention, preferred embodiments thereof will be described in detail herein below with reference to the drawings, wherein:
FIG. 1 is a perspective view of one embodiment of the present invention including a therapeutic compression apparatus according to the subject invention wrapped around a right leg and foot of a user having both a stirrup at a base of the foot and an adjustable strap below the knee, the apparatus having a pocket provided on an internal surface of the primary wrap (shown with hidden lines) for securing a fluid bladder, the bladder being provided with a detachable air pump and pressure gauge, and the primary and secondary wraps being detached from each other;
FIG. 2A is a perspective view of the therapeutic compression apparatus of FIG. 1 in which the primary and secondary wraps are illustrated in an unwrapped state, the interior surface of the wrap is shown, and the secondary wrap is shown to be integrated with the stirrup;
FIG. 2B is perspective view of the therapeutic compression apparatus of FIG. 1 in which the primary wrap and the secondary wrap, are illustrated in an unwrapped state, the interior surface of the primary wrap is shown having a bladder within a pocket of the primary wrap, and the secondary wrap is shown to be integrated with the stirrup;
FIG. 3 is an elevational view of the exterior surface of the an upper section of the primary wrap according to the subject invention having a number of stitched darts and gathers for contouring around a leg, the primary wrap being illustrated to show a majority of the exterior surface of the primary wrap;
FIG. 4 is a cross-sectional view, taken along line 12'-12' of FIG. 3 illustrating the composite material of the primary wrap of the subject invention, which has three discrete layers;
FIG. 5 is an exploded perspective view of a bladder having a plurality of spot welds, a pressure gauge and air pump are also illustrated as detached from the bladder according to the subject invention;
FIG. 6A is an elevational side view of the inflated bladder of FIG. 5 in an inflated condition;
FIG. 6B is a cross-sectional view taken along line 36'-36' illustrating how the strategically positioned spot welds in the bladder serve to join the upper and lower walls of the bladder to form a desired gradient profile;
FIG. 7 is a cross-sectional view of a wedge-shaped bladder in a non-inflated state (left) and an inflated state (right);
FIG. 8 is a cross-sectional view of a cone-shaped bladder in a non-inflated state (left) and an inflated state (right);
FIG. 9 is a cross-sectional view of a disk-shaped bladder in a non-inflated state (left) and an inflated state (right);
FIG. 10 is a cross-sectional view of a rectangular-shaped bladder in a non-inflated state (left) and an inflated state (right);
FIG. 11 is a top plan view of another exemplary embodiment of the present invention including a therapeutic compression apparatus constructed in accordance with the present invention, showing the layout of through-holes, heel pad and ankle pad throughout the compression garment as viewed from the interior of the garment;
FIG. 12 is a bottom plan view of the therapeutic compression apparatus of FIG. 11, showing the weld pattern to connect the outer sheet to the inner sheet defining a bladder within the inner and outer sheets as viewed from the exterior of the garment;
FIG. 13 is an exploded perspective view of the therapeutic compression apparatus of FIG. 11, showing the layout of the outer sheet, the inner sheet and hook and loop closures;
FIG. 14 is a detailed cross-sectional view of the therapeutic compression apparatus taken along line 4-4 of FIG. 11, illustrating the leg compression bladder in a deflated state;
FIG. 15 is a detailed cross-sectional view of the therapeutic compression apparatus taken along line 4-4 of FIG. 11, illustrating the leg compression bladder in an inflated state;
FIGS. 16-17 are front perspective and medial perspective views, respectively, of the therapeutic compression apparatus of FIG. 11 being worn on a lower leg;
FIG. 18 is a detailed cross-sectional view of the therapeutic compression apparatus taken along line 8-8 of FIG. 17, illustrating the leg compression bladder in an inflated state; +
FIG. 19 is a broken schematic view of another embodiment of the present invention including an assembly according to the invention;
FIG. 20A is an isometric view of a compression mechanism of FIG. 19 in accordance with the present invention, showing the mechanism in its unwrapped state;
FIG. 20B is an isometric view of an alternate compression mechanism in accordance with the present invention, showing the mechanism in its unwrapped state;
FIG. 21A is a view of another embodiment of a compression mechanism of FIG 19 in accordance with the present invention which is intended to be wrapped around the lower leg for application of localized pressure to the lower leg in the vicinity of the short saphenous vein and which is shown in its unwrapped state with its body-contacting surface facing out of the page;
FIG. 21B is a view of another embodiment of a compression mechanism of FIG 19 in accordance with the present invention which is intended to be wrapped around the upper leg (e.g., thigh) for application of localized pressure to the upper leg in the vicinity of the long saphenous vein and which is shown in its unwrapped state with its body-contacting surface facing into the page;
FIG. 22 is a broken exploded view of an embodiment of a unit including a pre-filled air bladder, an absorbent foam, sponge or dressing, and a suction conduit where the suction conduit extends into the center of the unit;
FIGS. 23A-23B are broken perspective views of a unit having straps for securing the unit in place;
FIG. 24 is a diagram of another embodiment of the present invention including a compression apparatus of the present invention wherein a leg bladder and a foot bladder are fluidly connected by a fluid conduit;
FIG. 25A is a perspective view of the leg bladder of FIG 24;
FIG. 25B is a top view of the leg bladder of FIG. 25A;
FIG. 25C is a side section view of the leg bladder of FIG. 25A;
FIG. 26 is a side view of the another embodiment of the foot bladder of FIG. 24 secured to the heel of a foot;
FIG. 27 is a side view of another embodiment of the invention showing the foot bladder positioned on a heel of a foot and the leg bladder positioned about a lower portion of a leg;
FIG. 28A is a perspective view of the invention showing the foot and leg bladders, the fluid conduit, and a one way valve connected to the fluid conduit the flexible member wrapped around a leg ;
FIG. 28B is a partially cut-away perspective view of the flexible member of FIG. 28A wrapped around a leg with the leg bladder sandwiched between the leg and the flexible member;
FIG. 29 is a schematic view of another embodiment of the present invention of a therapeutic compression apparatus including a valve for an inflation means or source which is joinable to the compression apparatus;
FIG. 29B is an exploded view of the embodiment of the present invention of a therapeutic compression apparatus as shown in FIG. 29A;
FIG. 30A is a close-up view of the valve mechanism of the compression apparatus of FIG. 29;
FIG. 30B is a close-up view of the valve mechanism of the compression apparatus of FIG. 29;
FIG. 31A is one embodiment of a pump of the present invention for a therapeutic compression apparatus;
FIG. 31B is a side view of the embodiment of a pump of the present invention for a therapeutic compression apparatus as shown in FIG. 31A;
FIG. 31C is a top view of the embodiment of a pump of the present invention for a therapeutic compression apparatus as shown in FIG. 31A;
FIG. 32A is another embodiment of a pump of the present invention for a therapeutic compression apparatus;
FIG. 32B is a blow up of the check valve of the embodiment of a pump of the present invention for a therapeutic compression apparatus as shown in FIG. 32A;
FIG. 33 is another embodiment of a pump of the present invention for a therapeutic compression apparatus;
FIG. 34 is a cross-sectional inner view of the embodiment of a pump of the present invention for a therapeutic compression apparatus as shown in FIG. 33;
FIG. 35 is a cross-sectional inner view of another embodiment of a pump of the present invention for a therapeutic compression apparatus;
FIG. 36 is a cross-sectional inner view of another embodiment of a pump of the present invention for a therapeutic compression apparatus;
FIG. 37 is another embodiment of the present invention of an arm therapeutic compression apparatus;
FIG. 38 is another embodiment of the present invention of an arm therapeutic compression apparatus wherein two separate therapeutic compression apparatus are joined by optional tabs;
FIG. 39 is a schematic of another embodiment of the present invention of a therapeutic compression apparatus including an electric inflation mechanism; and
FIG. 40 is one embodiment of the present invention of a therapeutic compression apparatus including an electric inflation mechanism as shown in FIG. 39.

### DETAILED DESCRIPTION OF THE PREFERRED EMBODIMENTS

Preferred embodiments of the subject invention are described below with reference to the accompanying drawings, in which like reference numerals represent the same or similar elements. One of ordinary skill in the art would appreciate that while the apparatuses discussed herein relate to compression therapy of the leg and foot, the scope of the invention is not limited to those exemplary applications and may be sized and shaped for the anatomical portion for which compression therapy is needed.

The subject invention provides compression to a patient's limbs, including the extremities, including for example, the lower leg and foot or an arm and a hand, in a manner that is simpler and more convenient than current systems. Any limb or body part may be compressed by the instant therapeutic compression apparatus such as for instance a foot, calf, thigh, knee, leg, hip, buttocks, waist, torso, ribs, shoulder, arm, hand, fingers, neck, head or the like.

The subject invention provides system for providing compression and preventing swelling of a limb such as for instance the foot using a non-elastic binder and bladder which can be used for compression. The bladder is provided within a non-elastic wrap and creates compression in a manner that allows for consistent measuring of the pressure supplied, as well as safe, comfortable, convenient, effective, self-application by the patient.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, exemplary methods and materials are now described.

It must be noted that as used herein and in the appended claims, the singular forms "a", "an," and "the" include plural referents unless the context clearly dictates otherwise. Thus, for example, reference to "a stimulus" would include a plurality of such stimuli and reference to "the signal" would include reference to one or more signals and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may differ from the actual publication dates which may need to be independently confirmed.

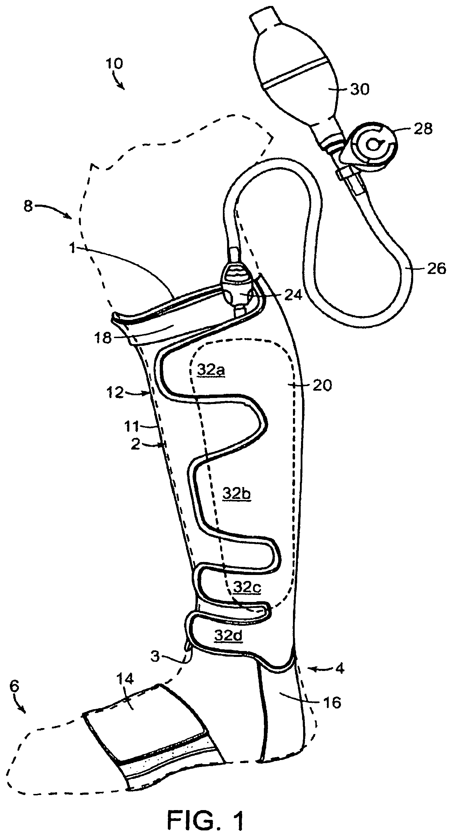

Referring now to FIG. 1, there is illustrated an exemplary embodiment of a therapeutic compression apparatus 10 according to the subject invention in which the exterior surface 11 of the primary wrap 12 of the therapeutic compression apparatus 10 is shown in a wrapped state around a patient's right leg. In this embodiment the therapeutic compression apparatus 10 is adapted and configured to supply compression of a lower leg 2 and foot 6 of a patient. The therapeutic compression apparatus 10 consists of a primary wrap 12 which encircles the lower leg 2 and may be utilized for applying compression to the lower leg 2. The primary wrap 12 has an associated bladder 22 provided on an inner surface 9 of the primary wrap 12. As shown in FIG. 1, the primary wrap 12 encircles the lower leg 2, and when the bladder 22 is filled or inflated, compression is applied to a desired treatment location. Notably, the primary wrap 12 is made of a non-elastic material which allows for increased localized compression of the treatment site. The therapeutic compression apparatus 10 also includes either a permanent or detachable pressure gauge 28 which is capable of being detached at the inlet or inflation port 24. A pump 30 is provided for manually pumping air into the bladder 22 through the tube 26. Other inflation means beyond a manual pump may be employed. Additional features of the primary wrap 12, secondary wrap 14, and bladder 22 will be described in turn herein.

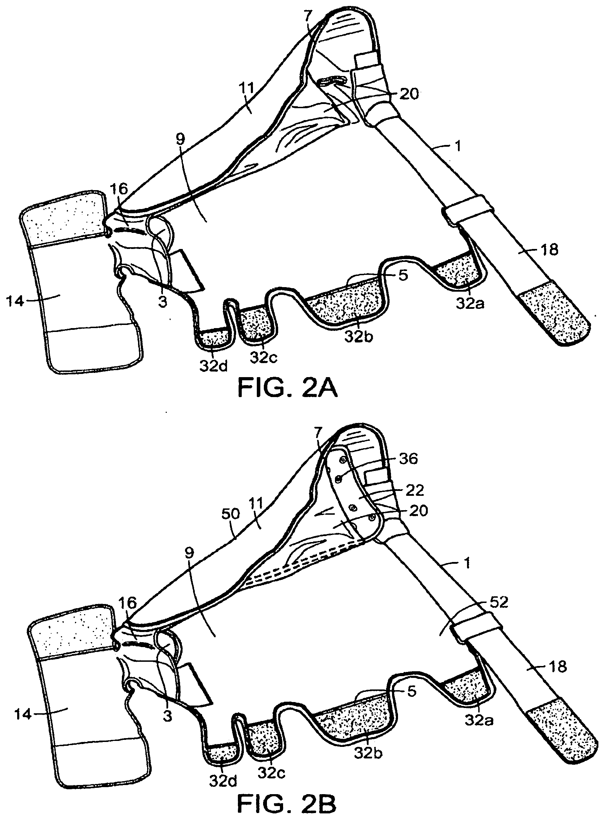

The primary wrap 12 may be attached to a patient's limb such as a lower leg 2, for example, by encircling the wrap about the lower leg 2 and attaching it at the peripheral edges 5 and 7 with any number of connecting structures. Hook and loop fastening tabs, such as connecting tabs 32a, 32b, 32c, 32d illustrated in FIGS. 1 and 2A-2B can be used to connect the opposing peripheral edges 5 and 7 of the primary wrap 12 and secure it about the lower leg 2. Hook and loop fastening tabs, buckles, straps, snaps or other known structures for fastening the primary wrap 12 to itself may also be used to attach the primary wrap 12 around the patient's limb. The number and position of the connecting tabs 32a, 32b, 32c, 32d may be selected based on the anatomical location of the patient's therapeutic site so that the primary wrap 12 is secured comfortably without causing bunching or sagging of the primary wrap 12 material and causing discomfort to the patient.

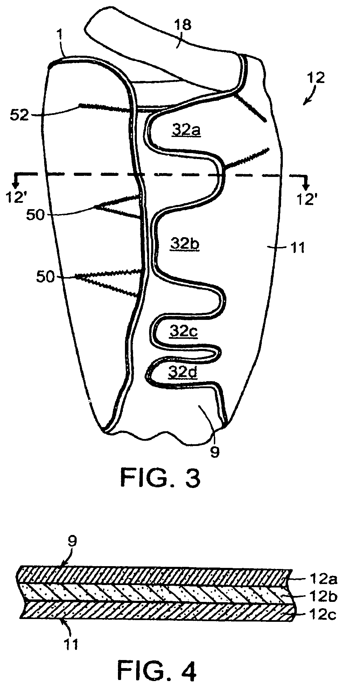

At a horizontal proximal end 1 of the primary wrap 12 in this instance towards the knee 8, an optional garter or adjustable belt 18 may be provided to further secure the primary wrap 12 about the lower leg 2. The adjustable belt 18 may be connected around the leg 2 using a connecting tab, buckle or other known connecting structures. As shown in FIG. 3, the primary wrap 12 is also manufactured with a number of stitched darts 50 and gathers 52 which are strategically placed to contour the primary wrap 12 around the specific limb being treated.

The primary wrap 12 is preferably made of a composite material that is non-elastic and has one or more wicking layers. Because the material is non-elastic, the primary wrap 12 remains stiff and does not stretch when the bladder 22 is filled, or inflated, for example. Referring to FIG. 4, the composite material of the primary wrap 12 has three discrete layers 12a, 12b, and 12c as shown. The inner layer 12a is in contact with the patient's skin and includes an interior surface 9 that is non-elastic and is preferably made of a wicking, non-hook and loop compatible material. The wicking feature of this inner layer 12a pulls perspiration and other unwanted moisture away from the limb being treated. The non-hook and loop-compatible nature of the inner layer 12a is desirable so that the primary wrap 12 will not get caught on the connecting tabs 32a, 32b, 32c, 32d, if hook and loop fastening tabs, are used, for example. The non-hooking material against the patient's skin also reduces irritation, which may be caused from a hooking material. The outer layer 12c has at least a portion which is hook compatible in order for the connecting tabs 32a, 32b, 32c, 32d to be secured at the peripheral edge of the primary wrap 12 about the limb. The bottom surface of the outer layer 12c is the exterior surface 11 of the primary wrap 12 and is also non-elastic and breathable. The inner layer 12b, located between the inner and out layers 12a and 12c, is typically made of a breathable, laminate material.

Referring to FIGS. 2A-2B, a stirrup 16 is also provided to help to secure the primary wrap 12 in place around the limb which in this instance is the base of the foot 6. As best seen in FIG. 1, the stirrup 16 in this example encircles a portion of the foot 2, and is connected to the horizontal distal edge 3 of the primary wrap 12 at two points on either side of the primary wrap 12 near the ankle 4. The stirrup 16 is located between the primary wrap 12 and the secondary wrap 14. As shown in FIGS. 2A-2B, the stirrup 16 is typically one continuous piece of elastic material connected to and integrated with the secondary wrap 14.

The secondary wrap 14 of the therapeutic compression apparatus 10 encircles the limb in this instance the foot 6 and, like the primary wrap 12, may be similarly utilized for both applying compression to the limb such as foot 6 or alternatively as a protective garment for wound care dressings. The secondary wrap 14 helps to prevent swelling of the limb such as the foot 6. The swelling may occur on its own, or as a result of compression of the lower leg 2. The secondary wrap 14 may be optionally made of a continuous piece of material with the stirrup 16, as shown in FIGS. 2A-2B. Alternatively, as shown in FIG. 1, the secondary wrap 14 may be provided independently from the primary wrap 12.

The secondary wrap 14 is typically formed of an elastic material, but may also be formed of a non-elastic material or a combination of the two. The secondary wrap 14 may be a single piece of connected material. Alternatively, the secondary wrap 14 may also be secured about the foot 6 or another limb or body part by any number of mechanical securing devices such as one or more hook and loop fastening tabs as shown in FIG. 1, or by straps, buckles, snaps, and the like. In this embodiment, the secondary wrap 14 provides compression to the foot 6 by pulling and tightening a hook and loop fastening tab, thereby applying pressure around the foot 6.

The secondary wrap 14 may have a number of configurations depending on the therapeutic needs of the patient. The secondary wrap 14 may be open-toed to expose the toes of the patient's foot 6 as shown in FIG. 1. Alternatively, the secondary wrap 14 may be configured as a close-toed boot. In addition, the secondary wrap 14 may have an open heel as shown in FIG. 1 or have a closed heel similar to a boot (not shown).

The primary wrap 12 supplies compression to a patient's limb by non-elastically holding at least one bladder 22 around the treatment site. In one embodiment, for example, localized pressure is provided by the therapeutic compression apparatus 10 near the saphenous vein of the lower leg 2. As shown in FIG. 2B, the primary wrap 12 covers the bladder 22 associated with the primary wrap 12. The fluid within the bladder 22 may be a liquid, a gas or a gel. The bladder 22 serves to provide compression to the leg 2 when the bladder 22 is filled within the therapeutic compression apparatus 10.

The bladder 22 may be inserted into one or more pockets 20 provided within the primary wrap 12 for storing the bladder 22 at a location where compression will be primarily applied. In this embodiment, the bladder 22 is detachable from the therapeutic compression apparatus 10. In another embodiment, the bladder 22 is permanently integrated within the primary wrap 12 and not detachable (not shown).

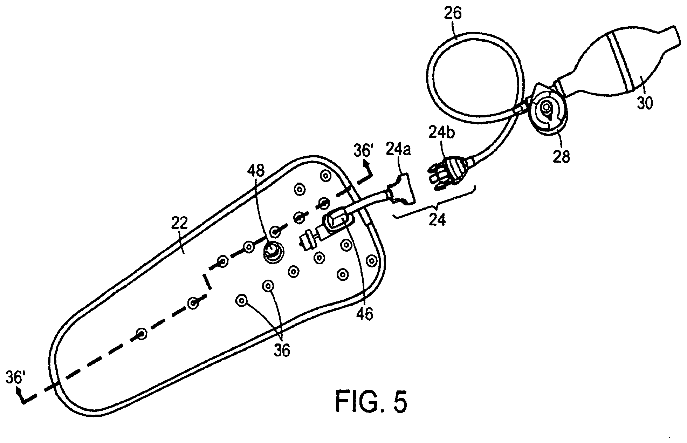

According to the subject invention, the bladder 22 may have a number of additional features for monitoring, setting and adjusting the pressure required for a desired therapeutic regimen. In an exemplary embodiment illustrated in FIGS. 1 and 5, the bladder 22 includes a pressure gauge 28. The pressure gauge 28 is a pressure measuring instrument, such as a manometer, which measures the pressure differential between a closed pressure applied within the bladder 22 and an open pressure at the other of the pressure gauge 28. The increase in the measured pressure depends on the density of the liquid used and the diameter of the tubing.

The pressure gauge 28 works in conjunction with the fluid or air pump 30 which pumps air into the bladder 22 through an inflation tube 26 at the inflation inlet 24a. The pump 30 may be a manual pump or an electronic pump for providing air to the bladder 22. An overflow valve 46 may also be provided and limits the amount of air capable of entering the bladder 22, along with a one-way valve 48 for releasing air from within the bladder 22, thereby lowering the pressure within the bladder 22. As shown in FIG. 5, the pressure gauge 28 and pump 30 may be detachably provided from the bladder 22 at a connector 24b.

In another embodiment, the bladder 22, itself, may serve as its own pressure gauge, in which the distention of the bladder 22 as it inflates indicates an amount of pressure within the bladder 22. In this instance, the pressure within the bladder 22 is pre-calibrated. Alternatively, more than one bladder 22 can be used, or a bladder 22 having multiple chambers can be used such that the distention of one or more of the bladders 22 or bladder chambers signifies the internal pressure. The subject invention provides pressure within the bladder 22 typically within the range of 20-50 mm Hg.

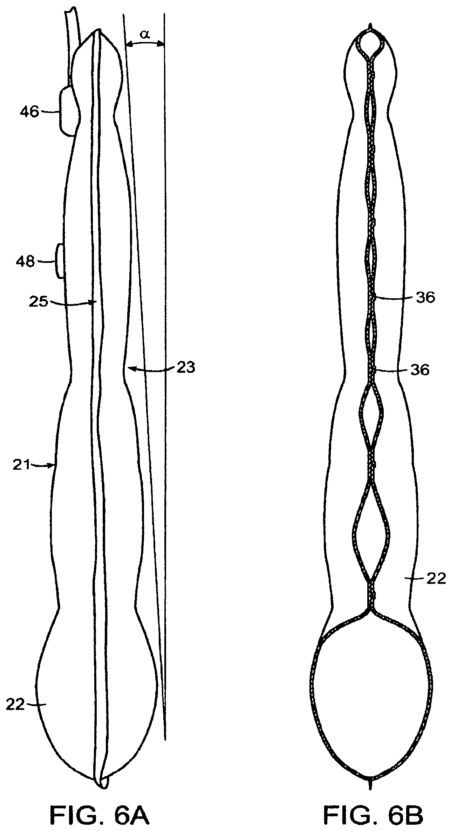

A number of different embodiments of a bladder 22 can be used in the therapeutic compression apparatus 10 of the subject invention. FIG. 5 shows a perspective view of one embodiment of a bladder 22 having a plurality of spot welds 36 according to the subject invention. The spot welds 36 are strategically placed within the bladder 22 in a predetermined pattern based on the desired gradient profile relative to the compression needed at the patient's treatment site. FIG. 6A is a side view of an inflated bladder 22 illustrated in FIG. 5 inflated to form an angle relative to the horizontal axis of the bladder 22. The bladder 22 has a first side wall 21 and a second side wall 23 which are sealed together along a peripheral edge seam 25. Spot welds 36 are strategically positioned to join the first and second side walls 21 and 23 to one another. These spot welds 36 enable the bladder 22 to change the gradient profile and take on a number of configurations when inflated. The geometric placement of the spot welds 36 within the bladder 22 allows increased inflation of certain portions of the bladder 22, and can create one or more fluid chambers within the bladder 22. FIG. 6B is a cross-sectional view of an inflated bladder 22 taken along line 36'-36'. When the bladder 22 is used within a primary wrap 12, the bladder 22 inflates more at one end of the primary wrap 12 than the other, creating a gradient compression profile, as shown in FIG. 5. This configuration is particularly useful when compression is needed to improve fluid movement (blood, lymph, etc) within the body.

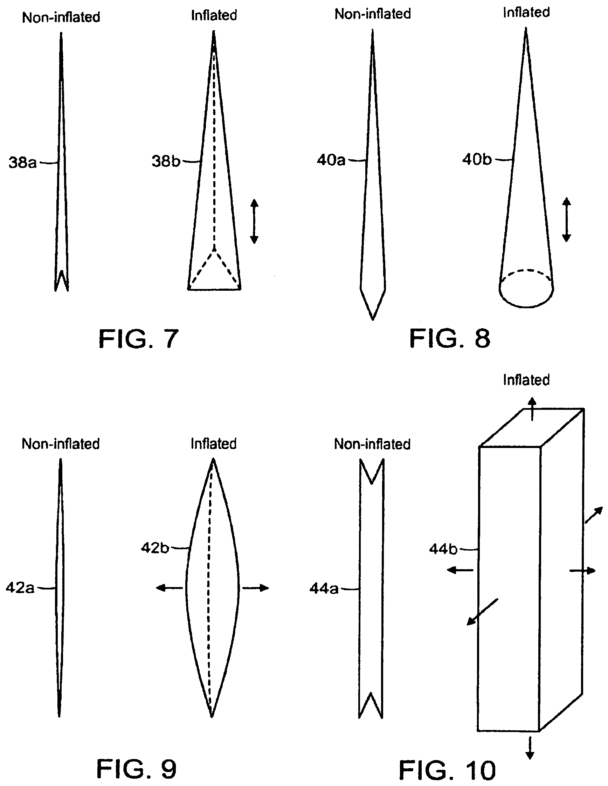

**In** addition to the bladder 22 having spot welds 36 illustrated in FIGS. 5 and 6B, several other bladder configurations shown in FIGS. 7-10 may be used within the therapeutic compression apparatus 10 of the subject invention. FIG. 7 is a cross-sectional view of a wedge-shaped bladder in a non-inflated state 38a (left) and an inflated state 38b (right) which may be used in the therapeutic compression apparatus 10 according to the subject invention. The wedge-shaped bladder 38b provides a comfortable and efficient gradient profile to the lower leg 2 when inflated and positioned within the primary wrap 12. The wedge-shaped bladder 38b has a pyramidal shape, as illustrated in FIG. 7 when inflated. One of the three pyramidal sides may be rigid, to prevent distention of the wedge-shaped bladder 38b in a direction away from the desired treatment area. Alternatively, the side closest to the treatment area may be attached or connected to a rigid material placed in a pocket 20 of the primary wrap 12 to prevent distention of the wedge-shaped bladder 38b in a direction away from the desired treatment area. The wedge-shaped bladder 38b is preferable to accommodate the normal anatomy where the ankle 4 is thinner than the lower leg 2. Thus, when the wedge-shaped bladder 38b is placed on the leg 2, the thinner portion is positioned towards the knee 8 and the thicker end is positioned towards the ankle 4. Referring now to FIG. 8, the cone-shaped bladder 40b, is similar to the wedge-shaped bladder 38b, and forms a cone when inflated. The inflated cone-shaped bladder 40b is preferable for use in normal anatomy in which the ankle 4 is thinner than the lower leg 2.

Turning to FIG. 9, a cross-sectional view of a disk-shaped bladder in a non-inflated state 42a (left) and an inflated state 42b (right) is shown. The disk-shaped bladder 42b is formed from two walls and has a disk or saucer shape when inflated. The rectangular-shaped bladder shown in FIG. 10 in a non-inflated state 44a (left) and an inflated state 44b (right) provides added benefits over the disk-shaped bladder 42b. The rectangular-shaped bladder 44b is also known as a three-dimensional bladder that allows for compression without bulging or distention in a direction away from the treatment area. The rectangular-shaped bladder 44b inflates uniformly throughout its length and width. This uniform inflation reduces the bulging that may occur at the center of the disk-shaped bladder 42b illustrated in FIG. 9. The walls of the rectangular-shaped bladder 44b can be elastic or inelastic. Alternatively, a combination of both inelastic and elastic walls may be used. One or more portions of the walls of the rectangular-shaped bladder 44b may be formed of a rigid material or attached to a rigid material placed within the pocket 20 of the primary wrap 12 in order to avoid distention.

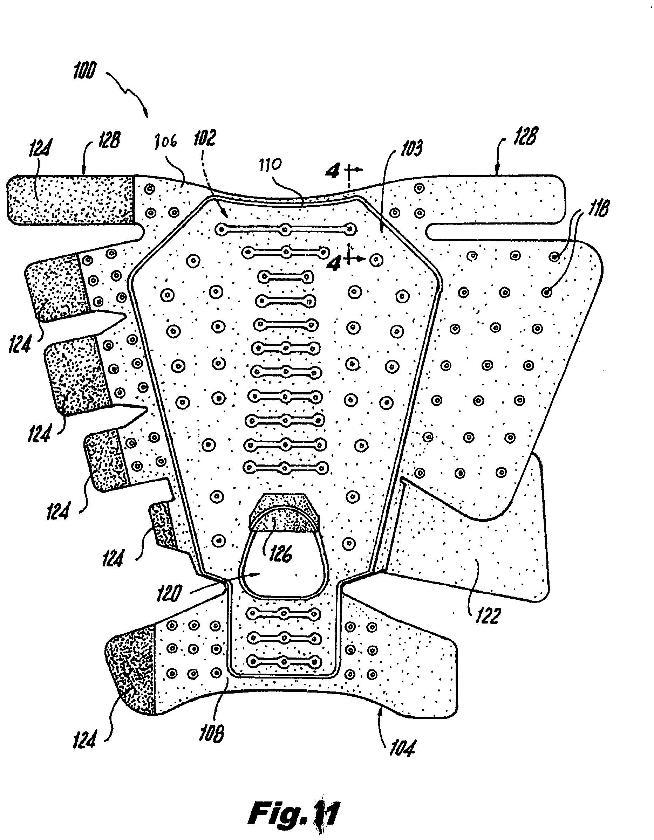

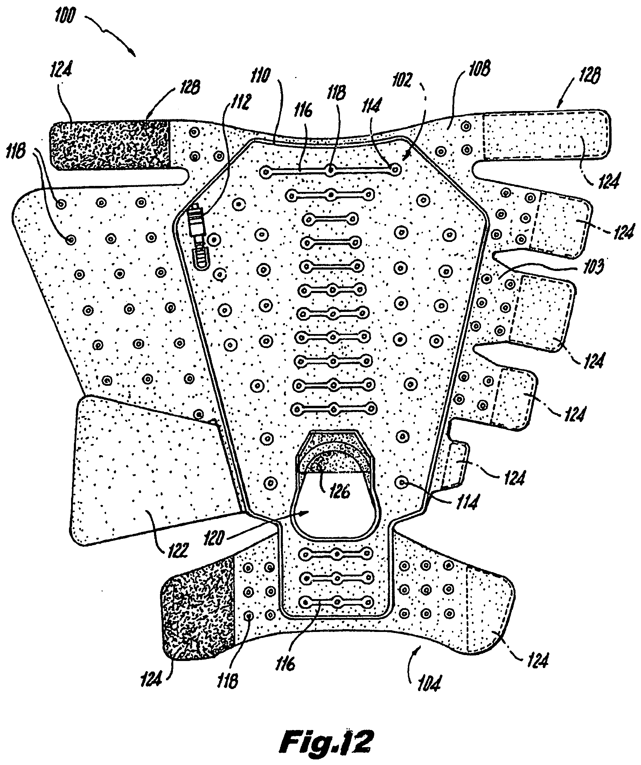

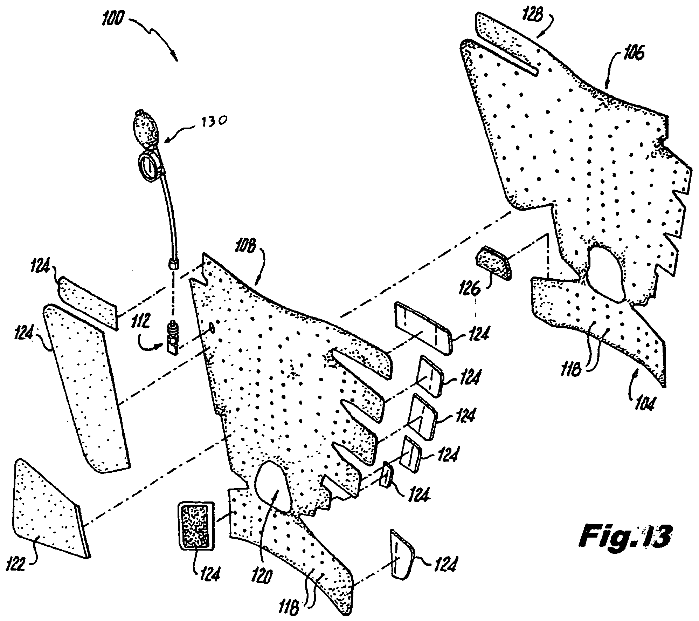

Referring to FIGS. 11-13, an exemplary embodiment of the therapeutic compression apparatus 100 constructed in accordance with the present invention, showing compression bladder 102 integrally formed in therapeutic compression apparatus 100. Therapeutic compression apparatus 100 is configured and adapted to wrap around a patient's limb such as in this instance a lower leg through the use of primary or primary wrap 103 and secondary or foot wrap 104 which are formed out of continuous outer sheet 108 and inner sheet 106. Therapeutic compression apparatus 100 is a wrap member with a proximal end portion (top as oriented in FIGS. 11 and 12) and opposed distal end portion (bottom as oriented in FIGS. 11 and 12) which is configured and adapted to conform around a patient's lower leg and provide compression through the inflation of bladder 102. Inner sheet 106 and outer sheet 108 are made out of a nylon laminated polyurethane sheet which are configured and adapted to be RF welded together. However, any other suitable materials which are weldable or otherwise joined while being airtight can be used. Continuous peripheral weld line 110 forms an airtight boundary of integrally formed bladder 102. In this exemplary embodiment, bladder 102 is a single continuous bladder throughout the primary wrap 103 and secondary wrap 104. However, it is envisioned that secondary wrap 104 can have an independent bladder either separately inflatable or inflatable through a one-way valve or other desired inflation/deflation configuration. Secondary wrap 104 can also be configured and adapted to provide a differing pressure from lower leg wrap 103. In an exemplary embodiment, bladder 102 located in secondary wrap 104 is configured and adapted to be located along the underside of a patient's foot. Bladder 102 in secondary wrap 104 can be adjusted as desired to provide compression to the desired part of a patient's foot.

In this embodiment, hook and loop fasteners 124 are provided along the edge of inner and outer sheets in order to ease adjustment and secure therapeutic compression apparatus 100 on a patient's limb such as for example a lower leg and foot. It is envisioned that the therapeutic compression apparatus 100 can also be secured to a patient's lower leg by other means, such as zippered, buttoned, or be cuff shaped by other such suitable means. Further, it is also envisioned that hook and loop closures 124 can be replaced by material similar to that of ankle strap 122 described below and be welded/sewn/attached to bladder 102 for improved comfort.

In this embodiment inflation means is a device 130 which is a hand pump capable of attaching to inflation port 112 to inflate bladder 102. It can be appreciated that a mechanical or automatic inflation pump (not shown) can also be attached to inflation port 112 to inflate and deflate bladder 102 to provide pulsating pressure to a user's lower leg. A number or variety of inflation means can be employed such as a manual pump, hand pump, foot pump, mechanical pump, electrical pump, battery-operated pump, static pump, intermittent pump, varying pump, automatic pump, pneumatic pump, negative pressure pump, suction pump or vacuum, pulsing pump, or any other known or developed source of inflation so as to provide a certain pressure within the bladder so to provide compression in use by the patient. A vent valve (not shown) can also be incorporated into therapeutic compression apparatus 100 or with inflation means 130 to allow a user to selectively deflate bladder 102. Further, a check valve or relief valve is incorporated with either inflation means 130 or bladder 102 to prevent over-inflation once a maximum pressure is detected. Examples of relief valves are described in U.S. Pat. No. 7,276,037 and U.S. Pat. No. 7,850,629.

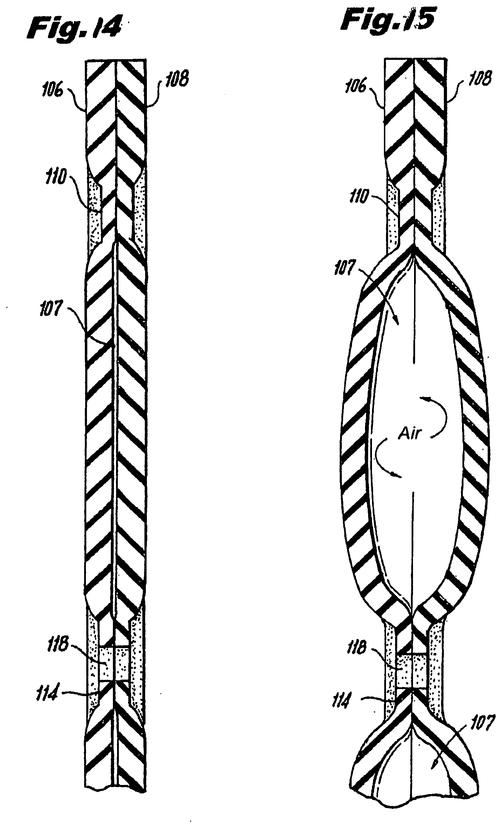

Referring now to FIGS. 14-15, by forming bladder 102 to be integral within inner sheet 106 and outer sheet 108, the location and desired preconfigured compression gradient profile can be obtained cost-effectively. A number of different embodiments of bladder configurations can be used in the therapeutic compression apparatus of the subject invention such as those configurations described above. FIGS. 11 and 12 show therapeutic compression apparatus 100 having bladder 102 with a plurality of spot welds 114 therein. Spot welds 114 are strategically placed within bladder 102 in a predetermined pattern based on the desired gradient profile relative to the compression needed at the patient's treatment site. Spot welds 114 enable bladder 102 to define the gradient profile when inflated through inflation port 112. The geometric placement of spot welds 114 within bladder 102 allows increased inflation of certain portions of bladder 102, and can create one or more fluid chambers within bladder 102. This configuration is particularly useful when compression is needed to improve fluid movement (e.g., blood, lymph, etc.) within the body. Further, linear weld lines 116 allow for better compression along the back of a patient's calf by increasing tension applied to the back of the calf of a patient. This increased tension can generate a more effective calf compression in order to increase venous flow. Linear weld lines 116 located laterally along the back of the calf create a ribbed portion, which keeps the inflated profile of therapeutic compression apparatus 100 compact which can further increase ambulation and reduce interference with a patient's clothes. In another embodiment secondary wrap 104 can also be made from an elastic garment without bladder 102.

It can be appreciated that depending on the location of the therapeutic compression apparatus, different pressure gradients may be utilized. Examples of other bladder pressure gradient profiles are described in U.S. patent application Ser. No. 12/911,563 and U.S. patent application Ser. No. 12/855,185.

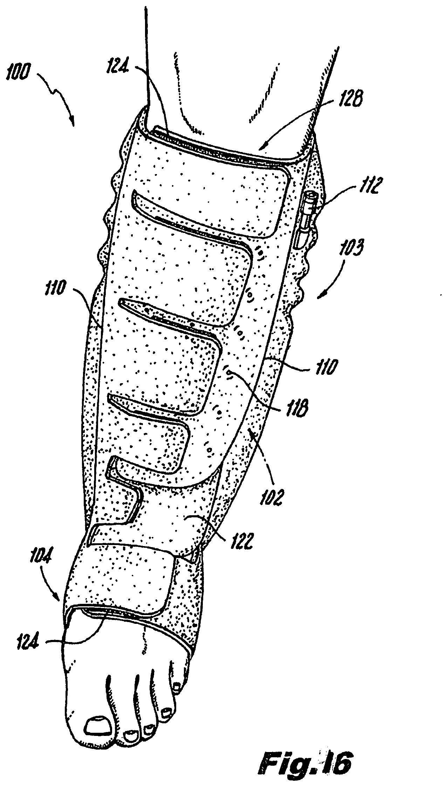

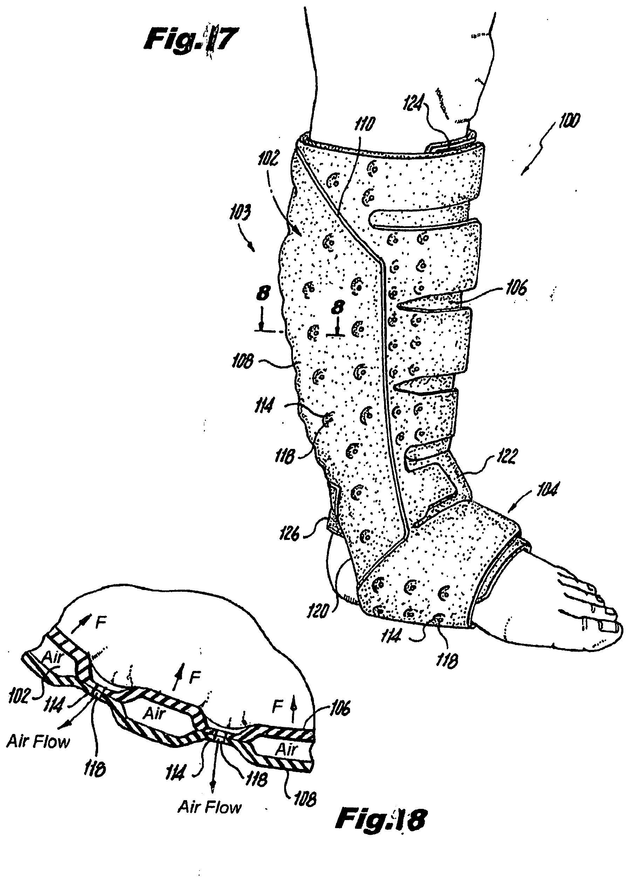

Referring now to FIGS. 16-17, once therapeutic compression apparatus 100 is secured around a patient's lower leg, bladder 102 is not able to shift out of place, thus increasing comfort and reducing fitting issues on the patient. In order to increase the ease of ambulation by a patient, in an exemplary embodiment, ankle cushion 126 can be attached adjacent heel port 120 to prevent the occurrence of a pinch point and reduce pressure on a patient's Achilles tendon. In combination with ankle cushion 126, ankle strap 122 can be used. In an exemplary embodiment, ankle strap 122 can include non-elastic foam which prevents a pinch point at the bottom of lower leg wrap 103 and the upper part of secondary wrap 104. A further advantage to providing ankle strap 122 is that bladder 102 proximate ankle strap 122 is pulled tight against a patient's leg and improves compression near the heel of a patient. Ankle strap 122 is advantageously wrapped around the patient's ankle and foot prior to affixing hook and loop fasteners 124. In order to improve comfort, through-holes 118, as seen in FIG. 18, are located throughout therapeutic compression apparatus 100 in order to allow for ventilation about a patient's leg during extended wear of therapeutic compression apparatus 100. For the sake of clarity, not all of the through-holes 118 are identified with reference characters in the Figures.

In accordance with an exemplary embodiment, inner sheet 106 further includes a layer (not shown) that has a first elastic modulus, inner sheet 106 has a second elastic modulus. The first elastic modulus is less than the second elastic modulus in a transverse direction relative to the proximal and distal end portions of therapeutic compression apparatus 100 to wrap therapeutic compression apparatus 100 around the leg when the leg compression bladder is inflated. In an exemplary embodiment, inner sheet 106 includes a secondary sheet (not shown) disposed on an inner surface thereof, to directly contact the lower leg in use. The secondary sheet can be a fabric layer, which is elastic in a first direction and inelastic in a second direction to curl the wrap member around the leg when the leg compression bladder is inflated.

In another exemplary embodiment, upper leg strap 128 is configured and adapted to improve wearability of therapeutic compression apparatus 100 by locating a portion of bladder 102 above the widest portion of the calf of a patient and provides stability of therapeutic compression apparatus 100 by preventing therapeutic compression apparatus 100 from slipping down the lower leg of a patient which would make therapeutic compression apparatus 100 ineffective in providing calf compression.

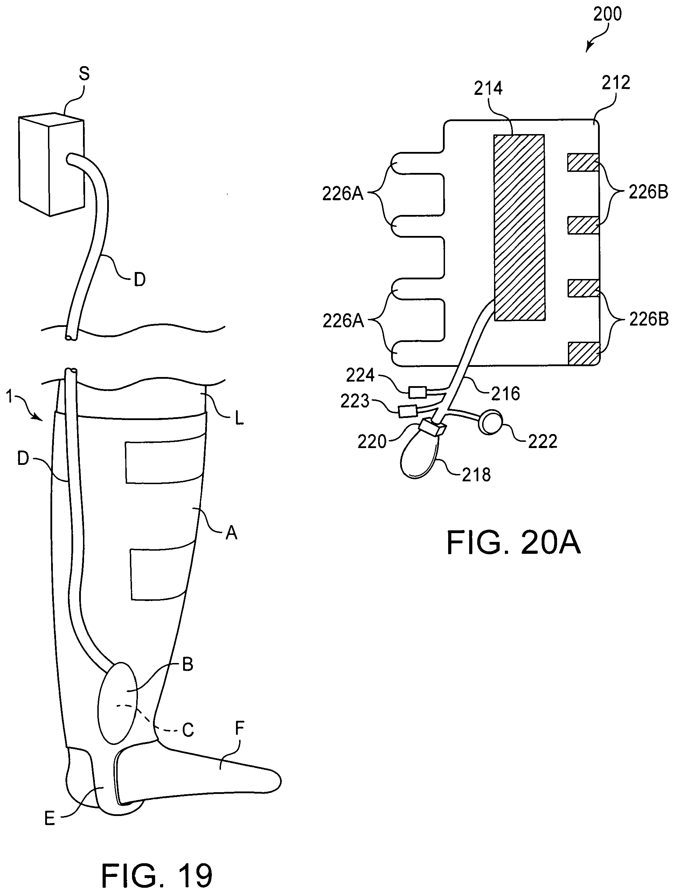

Turning to FIG. 19, another embodiment of the present invention is shown including a medical assembly 150 is provided. The medical assembly generally includes a compression mechanism A, a pre-filled air bladder B, an absorbent foam, sponge or dressing C, and a suction conduit D which is coupled to a suction apparatus S. The pre-filled air bladder B, absorbent foam, sponge or dressing C, and the suction conduit D may be provided as a unit. As shown, the compression mechanism A is wrapped around the leg L of a patient and includes an optional foot strap E which may be wrapped around the foot F of the patient. The suction conduit D is of desired length to couple the assembly 1 to the suction source S.

Turning to FIG. 20A, another embodiment of the therapeutic compression apparatus 200 (corresponding to compression mechanism A of FIG. 19) is provided for applying pressure to the limb of a patient such as for instance the lower leg of the human body. The therapeutic compression apparatus 200 preferably includes a flexible wrap member 212 and one or more inflatable air bladder chambers 214 (preferably, a single air chamber as shown). The inflatable air bladder chamber 214 is preferably secured to the flexible wrap member 212 in its unwrapped state. For example, the flexible wrap member 212 may comprise two layers of elastomeric material with the air bladder chamber(s) 214 affixed between these two layers by nylon threads or other suitable fastening means. Alternatively, the flexible member 212 may include pockets into which the air bladder chamber(s) 214 are removably inserted and securely held therein. In yet another alternative embodiment, the air bladder may be glued or welded to the inside surface of the member 212. The elastomeric material of the member 212 may be realized from nylon, polyurethane, cotton, or other suitable material. A tube 216, which is in fluid communication with the air bladder chamber(s) 214, extends to a pumping bulb 218. The pumping bulb 218, which is preferably made of rubber, includes a valve 220 that regulates the pumping of air into the air bladder chamber(s) 214 via the tube 216. Air is pumped into the air bladder chamber(s) 214 by squeezing the pumping bulb 218. In this manner, the air bladder chamber(s) 214 are placed into a pressurized state. Preferably, a pressure gauge 222 is operably coupled to the air bladder chamber(s) 214 to provide a visual indication of the pressure level therein. An automatic pressure relief valve 223 and a manual pressure relief valve 224 may be operably coupled to the air bladder chamber(s) 214, for example via the tube 216. The automatic pressure relief valve 223 automatically vents the air in the chamber(s) 214 to the ambient environment when the internal pressure reaches a certain threshold maximum pressure. In the preferred embodiment, this threshold maximum pressure is between 30 to 40 mmHg, and most preferably around 40 mmHg; however it can be varied based upon the desired treatment. In this manner, the pressure inside the chamber(s) 214 cannot exceed the threshold maximum pressure, thereby reducing the danger of necrosis and other complications that arise from excessive pressure. The manual pressure relief valve 224 vents the air in the chamber(s) 214 to the ambient environment when manually actuated by the patient (or caregiver). In this manner, it facilitates quick and easy control over the internal pressure of the air chamber(s) 214. In alternative embodiments, the manual pressure relief valve 224 and possible the automatic relief valve 223 may be integrated into a common package.

The air chamber 214 is substantially longer in a first dimension (e.g., the vertical dimension of FIG. 20A) than in a second dimension orthogonal thereto (e.g., the horizontal dimension of FIG. 20A) such that the air chamber 214 can be positioned to extend substantially longitudinally along the lower leg to apply local pressure along its length (the vertical dimension). Such local pressure is substantially constant along the length of the chamber 214. In the illustrative embodiment, the air chamber 214 is disposed such that it runs along the calf of the lower leg, which enables the air chamber 214 to apply local pressure to the short saphenous vein of the patient when securely wrapped around the patient's lower leg and inflated. However, the flexible member 212 and air chamber 214 may be adapted such that they are disposed along another portion of the lower leg (e.g., a portion of the leg below the knee), which enables the air chamber 214 to apply local pressure to such portion of the lower leg when inflated.

The flexible member 212 may include a strap (not shown) that extends around the heal (and/or other parts) of the foot when in use. This strap limits the upward travel of the flexible member 212 when in use. It may also have suspender hooks or slots (not shown) that allow for suspenders to be mated thereto which support the mechanism 210 by a band that wraps around the knee or thigh. The suspenders limit downward travel of the flexible member 212 when in use. These features reduce the travel of the flexible member 212 along the length of the leg such that its desired position is maintained during use. The flexible member 212 may be positioned not only on the foot but also on other limbs or body parts such as the calf, thigh, hip, buttocks, torso, ribs, arm, hands, fingers, shoulder, neck, head or the like.

In alternative embodiments, the flexible member 212 and air chamber 214 may be adapted such that they are disposed along a portion of the upper leg (e.g., a portion of the thigh), which enables the air chamber 214 to apply local pressure to such portion of the upper leg when inflated. For treatment of the upper leg, the flexible member 212 may define an opening (not shown) at the knee joint level to enable the patella (knee cap) to protrude therethrough. In this configuration, the flexible member 212 may extend below the knee joint level and securely wraps around portions of the lower leg to provide stability to the leg. It may also have suspender hooks or slots (not shown) that allow for suspenders to be mated thereto in order to support the mechanism 210 by a waist band when in use. The suspenders limit downward travel of the flexible member 212 when in use such that the flexible member 212 maintains its desired position.

Preferably, the flexible member 212 includes multiple hook and loop closure mechanisms 226A, 226B (e.g., VELCRO^{™} members). In the preferred embodiment, the flexible member includes four hook and loop closure mechanisms as shown in FIG. 20A. These multiple closures enable the flexible member 212 and the air chamber(s) 214 to be securely wrapped around a portion of the human leg. If desired, other suitable fastening means may be used to secure the flexible member and the air chamber(s) to the human leg. For example, the flexible member may be adapted to form a sleeve-like shape with a zipper running along its length dimension. Alternatively, the zipper may be omitted such that sleeve-like flexible member is slid over the patient's leg until it is disposed in the desired position.

Preferably, pressure in the air chamber(s) is reduced/removed (e.g., the air chamber(s) are deflated) by user manipulation of the manual relief valve 224 or check valve, and the pneumatic compression mechanism is removed from the leg by manually detaching the hook and loop closures and unwrapping the flexible member 212 from around the leg.

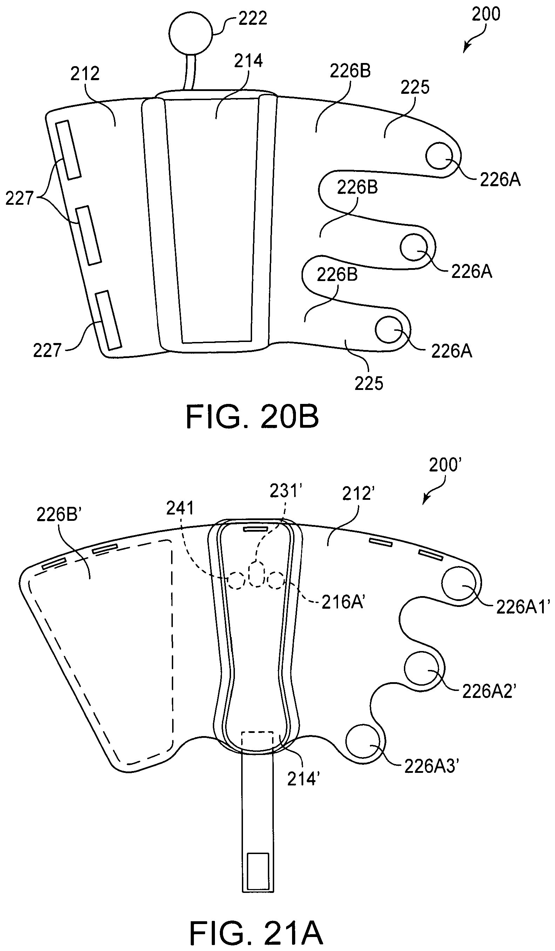

FIG. 20B illustrates an alternative compression mechanism 210 (corresponding to compression mechanism A of FIG. 19) which includes a flexible member 212 and one or more pre-inflated (pre-filled) and sealed air chambers 214 (preferably, a single air chamber as shown). The pre-inflated sealed air chamber 214 is preferably secured to the flexible member 212 in its unwrapped state. For example, the flexible member 212 may comprise two layers of elastomeric material with the air bladder chamber(s) 214 affixed between these two layers by nylon threads or other suitable fastening means. Alternatively, the flexible member 212 may include pockets into which the sealed air chamber(s) 214 are removably inserted and securely held therein. In yet another alternative embodiment, the air chamber may be glued or welded to the inside surface of the member 212. Regardless, a pressure gauge 222 is operably coupled to the air chamber(s) 214 to provide a visual indication of the pressure level therein. The flexible member 212 also includes multiple straps 225 having hook and loop closure mechanisms 226A, 226B (e.g., VELCRO^{™} members), and multiple openings 227 for receiving the straps. In the preferred embodiment, the flexible member includes three straps 225 and three openings 227. In use, the straps are threaded through the openings and back on themselves such that the hook and loop closure mechanisms 226A and 226B engage each other. By pulling the straps tight and affixing them using the closure mechanisms, more or less pressure may be applied to the pre-filled chamber; thereby applying more or less pressure to the limb on which the compression mechanism is applied. The amount of pressure applied may be read by the pressure gauge 222 which may have numerical or other visible indications.

Turning now to FIG. 21A, an alternate embodiment of a pneumatic compression mechanism (corresponding to compression mechanism A of FIG. 19) is shown. The compression mechanism 210' includes a flexible member 212' and one or more inflatable air chambers 214' (preferably, a single air bladder as shown). The inflatable air chamber 214' may be formed from two walls that are bonded together, preferably by heat sealing, about a flange portion in a manner similar to the fluid-filled members 230 as described herein. The air chamber 214' is secured to the inside surface of the flexible member 212' preferably by gluing, welding or other suitable fastening means. Alternatively, the flexible member 212' may comprise two layers of elastomeric material with the air chamber(s) 214' affixed between these two layers by nylon threads or other suitable fastening means. The flexible member 212' is preferably realized from nylon, polyurethane, cotton, or other suitable material. A connector 216A' is in fluid communication with the air chamber(s) 214' via a port (not shown) which preferably extends through the bottom side of the air bladder chamber 214'. The connector 216A' mates to an inflation tube (not shown) for fluid connection to the pumping bulb as described above or other inflation mechanisms. An automatic pressure relief valve 323' and a manual pressure relief valve are in fluid communication with the air chamber(s) 214' via additional ports (not shown) which preferably extend through the bottom side of the air bladder chamber(s) 214'.

Preferably, the flexible member 212' includes multiple hook and loop closure mechanisms (e.g., VELCRO^{™} members) which enable the flexible member 212' (and the air chamber(s) 214' secured thereto) to be securely wrapped around a portion of the human leg. In the exemplary embodiment of FIG. 20A, the flexible member 212' includes three hook buttons 226A1', 226A2', 226A3' disposed on the body-contacting side of member 212' that mate to a larger loop panel section 226B' disposed on the opposite side of member 212'. If desired, other suitable fastening means may be used to secure the flexible member 212' and the air bladder chamber(s) 214' to the human leg. For example, the flexible member 212' may be adapted to form a sleeve-like shape with a zipper running along its length dimension. Alternatively, the zipper may be omitted such that sleeve-like flexible member 212' is slid over the patient's leg until it is disposed in the desired position. The flexible member 212' may also include cut-outs (not shown) which provide enhanced flexibility of the member 212'.

During use, air is pumped into the air chamber(s) 214' by actuation of the pumping bulb (or other inflation mechanism). The air chamber 214' is substantially longer in a first dimension (e.g., the vertical dimension of FIG. 21A) than in a second dimension orthogonal thereto (e.g., the Horizontal dimension of FIG. 21A) such that the air chamber 214' can be positioned to extend substantially longitudinally along the leg to apply local pressure along its length (the Vertical dimension). Such local pressure is substantially constant along the length of the chamber 214'.

In the exemplary embodiment shown, the air chamber 214' has a length of 33.02 cm (13.00 inches) (Vertical dimension) and width of 9.76 cm (3.77 inches) and 8.915 cm (3.51 inches) (horizontal dimension) at its top and bottom ends, respectively, as shown. The width of the chamber 214' tapers as it extends away from the top and bottom ends to a minimal width, which is located relatively closer to the bottom end as shown. It will be appreciated that the air chamber 214' may take other shapes and sizes.

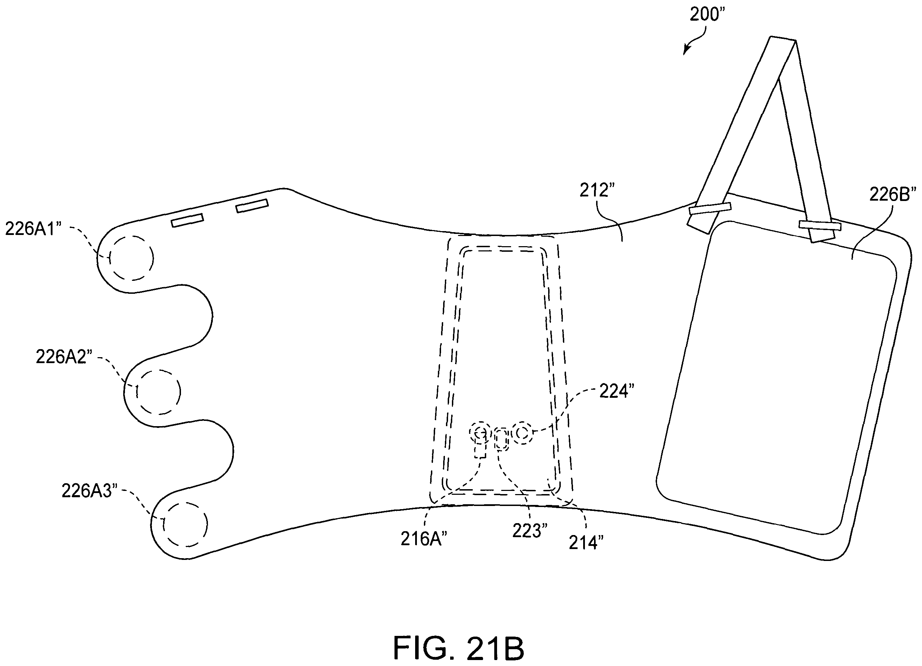

In another alternative embodiment as shown in FIG. 21B, a flexible member 212" and air chamber 214" of a compression mechanism 210" (corresponding to compression mechanism A of FIG. 19) are adapted such that they are disposed along a portion of the upper leg (e.g., a portion of the thigh), which enables the air chamber 214" to apply local pressure to such portion of the upper leg when inflated. The structure and operation of the elements of the mechanism 210" are analogous to the mechanism 210' as described above with respect to FIG. 21A and thus description of such elements (which are labeled with like numbers) are omitted for simplicity of description.

In the exemplary embodiment shown, the flexible member 212" is contoured to conform to the upper leg when wrapped around it. The air bladder chamber 214" has a length of 29.845 cm (11.75 inches) (Vertical dimension) and maximum width of 15.24 cm (6.00 inches) (Horizontal dimension) at its bottom end as shown. The width of the chamber 214" tapers as it extends away from the bottom end to the top end as shown. It will be appreciated that the air chamber 214" may take other shapes and sizes.

It will be appreciated that the chambers of either of the embodiments of FIGS. 21A and 21B may be pre-filled and sealed as described above with reference to FIG. 20B. It will also be appreciated that the air chambers which are arranged to receive air can be inflated using a manual pumping bulb (as shown in FIG. 20A), or can be inflated by an electric air pump (not shown) which can use batteries or AC wall current to pump air into the chamber(s). Any known source of air or fluid may be employed whether manual, mechanical, electrical, battery-operated or any other power sourced pump or pressure creator. The inflation means may be a manual pump, hand pump, foot pump, mechanical pump, electrical pump, battery-operated pump, static pump, intermittent pump, varying pump, automatic pump, pneumatic pump, negative pressure pump, suction pump or vacuum, pulsing pump, or any other known or developed source of inflation so as to provide a certain pressure within the bladder so to provide compression in use by the patient.

Other embodiments of the compression mechanism are possible such as a combination of a sealed fluid-filled bladder, an absorbent foam, sponge or dressing, and a suction conduit such as for example corresponding to bladder B, foam, sponge or dressing C and at least a portion of suction conduit D of FIG. 19).

The fluid-filled member 238 may be formed in many of numerous ways. In one arrangement, the fluid-filled member includes two walls 242A, 242B that are bonded together, preferably by heat sealing, about a flange portion (not shown). The two walls define a chamber 246 therebetween that is filled with fluid. The fluid held in the chamber 246 can be a gas (such as air), a liquid (such as water), or a gel. The fluid inside the chamber 246 may be loaded with one or more therapeutic agents, such as antibiotics, growth factor, absorbents. In such configurations, the bottom wall 242B is realized from a semi-permeable material that allows the therapeutic agents retained in the chamber 246 to migrate through to the treatment site while maintaining the desired internal pressure in chamber 246. Such fluid might also be a gel compound that retains heat and/or cold such that is useful for hot and/or cold therapy of the treatment site

The fluid-filled member 238 preferably has an oval shape with a length on the order of 10.16 to 15.24 cm (four to six inches), a width on the order of 5 to 10.16 cm (two to four inches), and a height on the order of 0.635 to 1.905 cm ( one-quarter to three-quarters of an inch). Enough fluid is preferably provided to prevent the walls of the member 238 from sagging and touching each other, although some sagging can be tolerated. The walls of fluid-filled member 238 may be formed from polyurethane, polyvinylchloride, nylon, or other plastic(s) known in the art and are of sufficient thickness (i.e., are sufficiently strong) such that the fluid-filled member 238 will not burst when 40mm Hg is applied to it by the compression mechanism. It will be appreciated that the fluid-filled member 238 may take other shapes and sizes, and may be formed from other materials.

Attached to the fluid-filled member 238 is the absorbent foam, sponge or dressing 245 (hereinafter referred to as "the sponge"). The sponge 245 has the ability to absorb exudate from the wound or ulcer. In addition, the sponge preferably has an open-cell structure which aids in wicking the exudate from the wound or ulcer. The sponge 245 is preferably of a similar size and shape as the fluid-filled member. The sponge 245 is preferably fixed by adhesive to the bottom wall 242B of the fluid-filled member such that that the sponge 245 and the fluid-filled member 238 cannot be easily separated from each other. Alternatively, the sponge may be lightly affixed to the fluid-filled member 242B by an adhesive film such that the sponge may be peeled off and replaced. As another alternative, the fluid-filled member 238 may be provided with a circumferential holding flap for the sponge 245 which may be inserted and into and removed from the flap as desired. If desired, the circumference of the sponge may be sealed either by injecting sealant into the sponge or by collapsing the open-cell structure of the sponge. Sealing the circumference of the sponge can help reduce air from entering the sponge when suction is applied to the unit as described below.

Some embodiments in the unit 230 may include a suction conduit 247 for example provided in between the fluid-filled member 238 and the sponge 245. More particularly, suction conduit 247 may be a tube having a bifurcated distal end 249 which defines a series of holes 251. The holes 251 may be oriented downwardly (towards the sponge 245 so that when a source of negative pressure is applied to the suction conduit 247, that negative pressure is applied to the wound or ulcer via the sponge 245. As a result, exudate can be wicked and suctioned away from the wound or ulcer via the sponge 245 and into the conduit 247. In another embodiment, the bifurcated distal end 249 of the suction conduit 247 extends only partially around the periphery of the unit 230. The proximal end of the conduit may couple to a bottle or other reservoir (not shown) which is also coupled to a source of negative pressure S (shown in FIG. 19). Alternatively, the proximal end of the conduit may terminate in a fluid coupling 259 which in turn can be coupled to an elongate conduit (extension tube) which is coupled to the bottle or reservoir and source of negative pressure. In this manner the conduit may be easily detached from the unit so that the patient can walk or be transported away from the source of suction while pressure is still being applied to the wound or ulcer.

The suction conduit 247 may be held between the fluid-filled member 238 and the sponge 245 preferably by an adhesive (not shown) which is applied to the top and bottom of the distal end 249 of the conduit 247, and which binds the distal end 249 to the sponge 245 on its bottom and to the fluid-filled member 238 on its top. Alternatively, the suction conduit 247 may be heat sealed or laser-sealed to the fluid-filled member 238 and/or the sponge 245. In embodiments where the sponge 245 is removable from the fluid-filled member 238, the conduit 247 is preferably adhered only to the bottom of the fluid-filled member 238.

Another embodiment of a unit incorporating a pre-filled air bladder, a sponge and a suction conduit (corresponding to bladder B, foam, sponge or dressing C and suction conduit D of FIG. 19) may be different from unit 230' of the previous exemplary embodiments in that the fluid-filled member 238' in this embodiment defines a central hole 239 (i.e., it is shaped like a donut), and the distal end 249' of the suction conduit 247' is not bifurcated and does not include a series of holes, but rather extends through the central hole of the fluid-filled member 238' and has an open end 251' located at sponge 245'. Preferably, the suction conduit 247' is held in place in the central hole 239 by adhesive (not shown) which binds the distal end 249' of the suction conduit 247' to the walls of the fluid-filled member 238' which define the central hole 239. Alternatively, the suction conduit 247' can be held in place by friction or by other means such that it can be detached from the pre-filled air bladder and sponge unit. As with the previous embodiments, the sponge and fluid-filled member may be strongly or lightly adhered to each other, or the sponge may be removable from a flange element of the fluid-filled member which holds the sponge. In addition, the sponge may be circumferentially sealed.

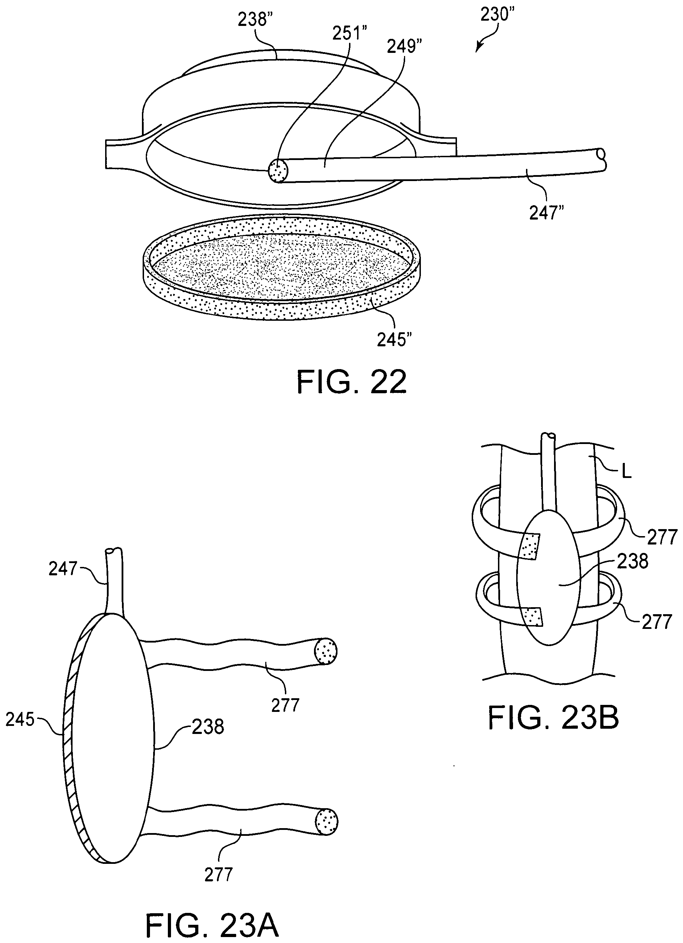

A third embodiment of a unit including a pre-filled air bladder, a sponge, and a suction conduit (corresponding to bladder B, foam, sponge or dressing C and suction conduit D of FIG. 11) is seen in FIG. 22. Unit 230" is similar to units 230 and 230' disclosed above as previous embodiments, except that the suction conduit 247" is oriented horizontally between the fluid-filled member 238" and the sponge 245" and includes a distal end 249" having an open end 251". The open end 251" is preferably centrally located in the unit 230", and preferably is oriented in a downward direction (i.e., the top half of the conduit 247" extends further than the bottom half). The suction conduit 247" is held between the fluid-filled member 238" and the sponge 245" preferably by an adhesive (not shown) which is applied to the top and bottom of the distal end 249" of the conduit 247", and which binds the distal end 249" to the sponge 245" on its bottom and to the fluid-filled member 238" on its top. In embodiments where the sponge 245" is removable from the fluid-filled member 238", the conduit 247" is preferably adhered only to the bottom of the fluid-filled member 238". If desired, the sponge 245" may be circumferentially sealed.

In accord with one aspect of the invention, the units 230, 230' and 230" may be provided with additional means for holding the unit in place over the wound or ulcer. Thus, the periphery of the unit might be provided with a biocompatible adhesive. The adhesive could be provided on the sponge, or the fluid-filled member might be provided with a flange (not shown) having a biocompatible adhesive. Alternatively, and as shown in FIGS. 23A and 23B, straps 277 may be provided as the means for holding or securing the unit in place. In one embodiment, the straps are formed from the same material and integrally with the fluid-filled member 238, 238', 238" and extend from one side of the fluid-filled member. The straps are adapted to be sufficiently long so as to encircle the limb L. The ends of the straps are provided with an adhesive which is used to couple the straps to the other side of the fluid-filled member. Alternatively, the fluid-filled member may be provided with slots (not shown) into which the straps may be strapped for tightening. In another embodiment, the straps are separately formed and attached to the fluid filled member. Again, the ends of the straps may be provided with an adhesive for coupling the straps to the other side of the fluid-filled member. Alternatively, the fluid-filled member may be provided with slots (not shown) into which the separately formed straps may be strapped for tightening.

In use, a patient or practitioner will select a unit 230, 230', 230", and locate the unit sponge face down over a wound or ulcer. If the unit includes straps, the unit can be strapped in place on the limb. Alternatively, if the unit includes an adhesive ring, the unit is located such that the adhesive ring is preferably located on healthy skin as opposed to the wound or ulcer. Once the unit is located, an appropriate compression mechanism 10, 110, 210, 210', 210" or other embodiments is wrapped over the limb with the air chamber 14, 214, 214', 214", 114 pr other embodiments located over the unit 230, 230', 230". When desired, the air chamber is pressurized (or pressure is applied by fixation of the wrap) so that pressure is applied by the compression mechanism to the limb and to the unit. Application of pressure to the unit applies specific pressure to the wound or ulcer and aids in the healing process. Also, when desired, suction is applied to the wound or ulcer by applying a source of negative pressure via the suction tube 247, 247', 247" to the unit. Where the sponge is circumferentially sealed, the amount of suction required to move exudates is lessened. The suction and pressure may be applied together or alone (alternatingly), or one or the other may be constantly applied and the other turned on and off as needed. When applied together, positive mechanical pressure is applied to the wound or ulcer by the compression mechanism and the unit, while negative air pressure is applied to the wound or ulcer via the sponge by the source of negative pressure, thereby removing exudates and further aiding in the healing process. In addition, if desired, a pressure sensor (not shown) may be incorporated with unit 230, 230', 230" and electrically coupled to the suction source S (typically via a wire--not shown--running along suction tube 247, 247', 247"), where the suction source S has an on/off control (not shown). In this manner, the source S of negative pressure can be turned on and off as a function of the mechanical pressure applied by the compression mechanism and the unit as sensed by the sensor (not shown). Thus, if the pressure is above a desired threshold, the suction can be turned on, and if the pressure is below that or a lower threshold the suction can be turned off; or if desired, if the pressure is above a desired threshold the suction can be turned off, and if the pressure is below that or a lower threshold the suction can be turned on. The pressure sensor (not shown) can be located between the fluid-filled member 238, 238', 238" and the sponge 245, 245', 245", or in the sponge, or on a surface of the fluid-filled member. Alternatively, the pressure sensor (not shown) can be associated with the air chamber 14, 114, , 214, 214', 214" or other embodiments of the flexible member 12, 112, 212, 212, 212', 212" or other embodiments.

Where the sponge is separable from the fluid-filled chamber, the sponge may be replaced at desired intervals. If not separable, the entire unit may be replaced as desired. Where the unit has a fluid coupling, the suction tube may be detached prior to replacement of the unit. This unit can be placed under the therapeutic compression apparatus 10, 100, 200, 300, 400 and assembly 310 so that the negative pressure is coupled with the compression for treatment of CVI, **DVT** and/or lymphedema.

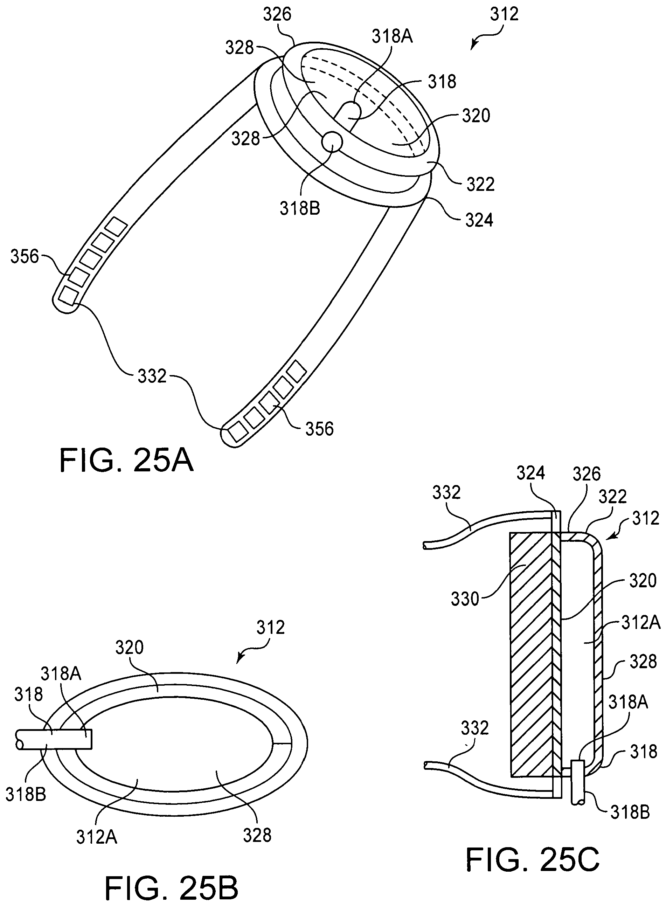

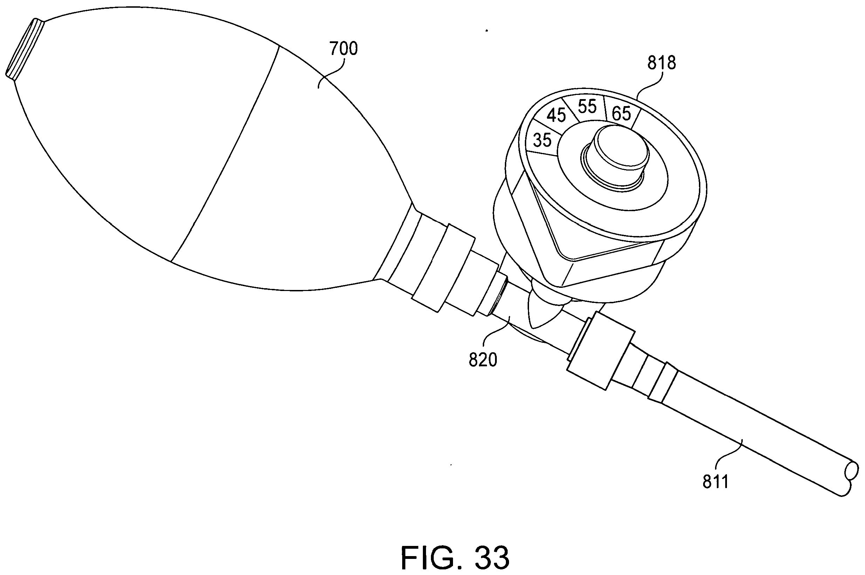

For purposes of explanation and illustration, and not limitation, another exemplary embodiment of the present invention is shown in FIGS. 18-24 including a therapeutic compression apparatus 200 and, or aspects thereof, in accordance with the invention is shown in FIGS. 18-24 and is designated generally by reference character 200. Another exemplary embodiment in accordance with the invention, or aspects thereof, is shown in FIGS. 25-34 and is designated generally by reference character 300 and the assembly by 310.

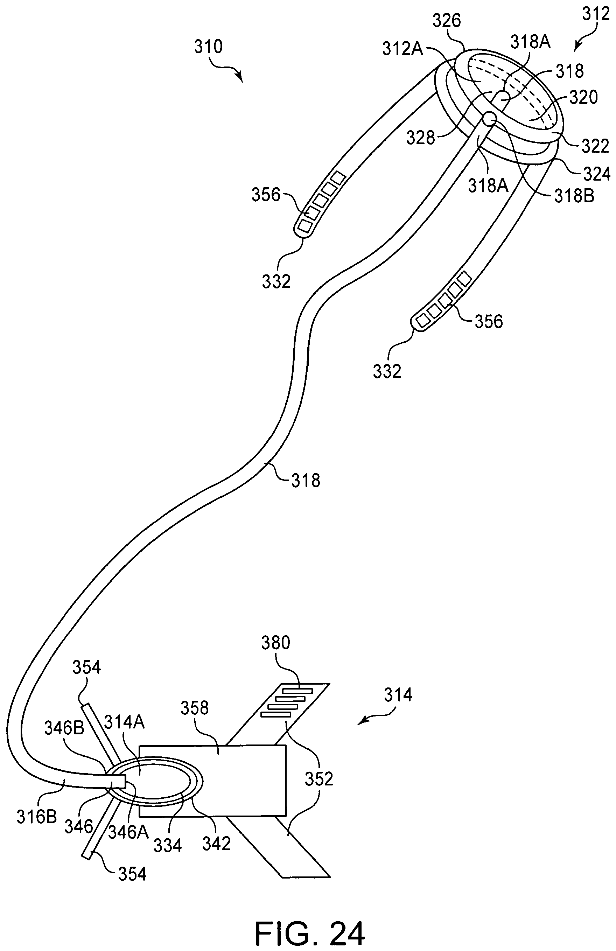

Turning to FIG. 24, shown is another embodiment of the therapeutic compression apparatus 300 including an inflation means for providing intermittent pressure to a portion of a human body, such as a leg, which promotes blood flow and fluid drainage to treat various conditions, including venous ulcers or wounds. In this embodiment the apparatus utilizes a primary bladder 312 and a secondary bladder 314, each having inflatable chambers 312a, 314a that accommodate an entering fluid by inflating. The bladders 312, 314 are fluidly coupled by a fluid conduit 316, which attaches to the primary bladder 312 and the secondary bladder 314 at respective ends 316a, 316b, thereby allowing fluid to flow back and forth between the primary bladder 312 and the secondary bladder 314. The intermittent deflation of one of the two bladders 312, 314 causes an intermittent inflation and pressure increase in the other of the two bladders 312, 314 as described in the preferred and alternative embodiments further discussed below

The primary bladder 312 is best seen with reference to FIGS. 25A-25C. The primary bladder 312 has a lower wall 320 and an upper wall 322 bonded together, preferably by heat sealing, on a flange portion 324 of the lower wall 320. The lower wall 320 and the upper wall 322 define the inflatable chamber 312a therebetween. The upper wall 322 extends orthogonally from the flange portion 324 to form a sidewall 326, and then curves to form a top wall 328 which is generally parallel to the lower wall 320. The lower wall 320 including the flange portion 324, and the upper wall 322 are flexible such that the lower wall 320 may bend to conform to a surface contour of a leg. A bottom layer 330 of material is optionally provided on the lower wall 320 of the primary bladder 312. The bottom layer 330 is preferably a semi-permeable material (e.g., a sponge) that absorbs sweat, puss, or other fluids and provides a more delicate contact surface to the wound. If desired, an adhesive film may be provided on the flange portion 324 to help secure the primary bladder 312 to the leg.

In one embodiment, the primary bladder 312 has a nozzle or connector element 318 extending through the upper wall 322 with a first end 318a inside the inflatable chamber 312a and a second end 318b outside of the inflatable chamber 312a. The nozzle 318 receives the fluid conduit 316 at the first end 316a of the fluid conduit 316 (FIG. 24). The second end 318b of the nozzle 318 may alternatively be located flush with the sidewall 326 rather than outside of the inflatable chamber 312a so long as an airtight seal is provided between the nozzle 318 and the upper wall 322. The nozzle is preferably provided with an internal valve (not shown) which is open so long as the fluid conduit 316 is inserted in the nozzle 318, but which closes and prevents fluid from escaping when the fluid conduit 316 is removed from the nozzle 318. As shown in FIG. 25C, the nozzle 318 preferably extends through the sidewall 3326 portion of the upper wall 322 parallel with the lower wall 320 so that when the primary bladder 312 is placed over a wound on a leg, the nozzle 318 extends in a direction principally parallel with a surface of the leg. Providing the nozzle 318 parallel with the surface of the leg will prevent it from creating a bulge or protrusion orthogonal to the surface of the leg, which may create additional pressure on the region of the leg directly underneath the nozzle 318 when the primary bladder 312 is attached to the leg as further discussed below. In addition, maintaining the nozzle 318 parallel with the surface of the leg will facilitate the attachment of the fluid conduit 316 with the primary bladder 312 in a direction principally parallel with the surface of the leg, a configuration that more readily allows clothing to be worn over the primary bladder 312.

In another embodiment (not shown) the primary bladder 312 does not contain a nozzle 318. Instead, the first end 316a of the fluid conduit 316 is integrally formed with the primary bladder 312 such that the fluid conduit 316 is inseparable from the primary bladder 312. An optional one-way valve 68 is provided as described below. If not provided, then the system is a closed system that is pressurized during a manufacturing process, and thus does not require the primary bladder 312 or any other component of the apparatus 10 to be pressurized prior to its operation.

Referring to FIGS. 25A-25C, the primary bladder 312 is optionally equipped with a plurality of straps 332 which are designed to extend around the leg in order to secure the primary bladder 312 to a bottom portion o223f the leg. The straps 332 may contain Velcro sections 356 (FIG. 25A) that overlap and attach to each other after wrapping around the leg. The straps 332 may also have an elastic quality to produce axially directed compression forces when they are wrapped around the leg. In one embodiment, the straps 332 are wrapped with tension over the inflatable chamber 312a after passing around the leg such that when the VELCRO^{™} sections 356 are connected, the straps 332 exert a continuous compressive force on the inflatable chamber 312a. In other embodiments (not shown) the straps 332 may be secured horizontally around the leg or crisscrossed over the primary bladder 312 to secure the primary bladder 312 to the leg and to provide resistance to the inflatable chamber 312a inflating outward, thus increasing the pressure on the wound area when additional fluid is forced into the inflatable chamber 312a as further discussed below.

The lower wall 320, upper wall 322, and bottom layer 330 of the fluid primary bladder 312 may be formed in a variety of shapes and from a variety of materials, but flexible plastic or rubber is preferred so that the fluid primary bladder 312 may bend to accommodate the specific contour and curves of a given leg. The size of the primary bladder 312 may be varied, and different sizes may be chosen depending on the size and location of the wound. The upper wall 322 of the fluid primary bladder 312 is preferably made from a material such as a flexible and resilient plastic (e.g., polyurethane, polyvinylchloride (PVC), or polypropylene) that changes shape in order to permit the volume of the inflatable chamber 312a to increase as additional fluid enters therein. The material of the upper wall 322 should be non-rigid so that as pressure increases inside the inflatable chamber 312a, the pressure is not absorbed by the upper wall 322, but rather, is transmitted through the wall to the leg. The operation of the primary bladder 312 as part of the overall apparatus 10 is discussed in more detail below.

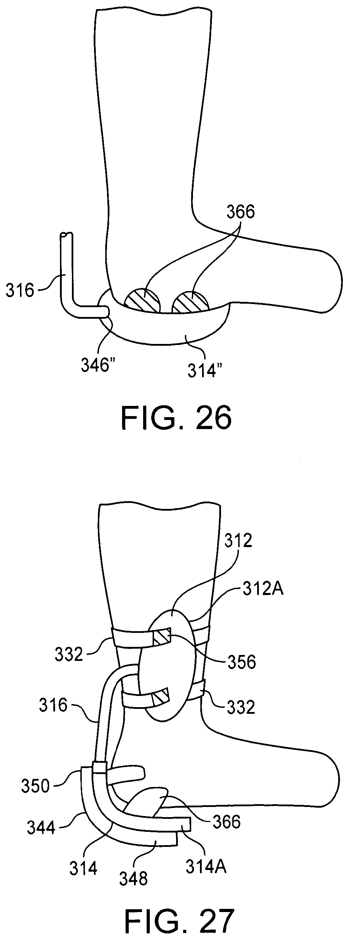

The fluid secondary bladder 314 is best seen with reference to FIG. 26. The secondary bladder 314 has a lower wall 334 with a flange 342, and an upper wall 336 that forms a sidewall 338 and a top wall 340. The fluid secondary bladder 314 may be formed in the same manner as the primary bladder 312. The lower wall 334 and upper wall 336 are bonded together, preferably by heat sealing on the flange 342 of the lower wall 334. The upper wall 336 and lower wall 334 define the inflatable chamber 314a. The upper wall 336 extends orthogonally from the flange 342 to form the sidewall 338 and curves to form the top wall 340, which is generally parallel to the lower wall 334. The lower wall 334, including the flange 342 and the upper wall 336 are flexible such that the lower wall 334 may bend to conform to a surface contour of an ankle.

In one embodiment as shown in FIG. 27, the secondary bladder 314 is worn in a bent configuration that conforms to the heel. The size and shape of the inflatable chamber 314a of the secondary bladder 314 may vary significantly, and is by way of example and not limitation between about 7.62 to 15.24 cm (three and six inches) long, about 3.81 to 7.62 cm (one and a half to three inches) wide, and when inflated between about 0.635 to 1.27 cm (one quarter and one half inch) in height. The shape of the inflatable chamber 314 preferably conforms to the shape of a foot or heel. A bottom layer 344 of material is optionally provided to the lower wall 334 of the secondary bladder 314. If the fluid secondary bladder 314 is worn without any footwear covering it, then the bottom layer 344 may provide added cushioning for the heel and/or may provide a non-slip surface having a higher coefficient of static friction than the lower wall 334. The fluid secondary bladder 314 may also be worn upside down wherein the inflatable chamber 314a is operably disposed on the exterior thereof, and the bottom layer 344 is disposed on the interior thereof in direct contact with the heel. The bottom layer 330 is optional in either of these configurations as the primary function of the secondary bladder 314, as discussed in further detail below, is to pump fluid into the primary bladder 312 and receive fluid back from the primary bladder 312 during the normal walking motion.

In one embodiment, the secondary bladder 314 may have a nozzle or connector element 346 extending through the upper wall 336 with a first end 346a inside the inflatable chamber 314a, and a second end 346b outside of the inflatable chamber 314a. The nozzle 346 receives the fluid conduit 316 at the second end 316b of the fluid conduit 316 (FIG. 24). The nozzle 346 is preferably provided with an internal valve (not shown) which is open so long as the fluid conduit 316 is inserted in the nozzle 346, but which closes and prevents fluid from escaping when the fluid conduit 316 is removed from the nozzle 346. The nozzle 346 preferably extends through the sidewall 338 portion of the upper wall 336 parallel with the lower wall 334 so that when the secondary bladder 314 is bent into a configuration with the nozzle 346 extending in an upward direction principally parallel with the back of the leg.

In another embodiment the secondary bladder 314 does not contain a nozzle 346. Instead, the second end 316b of the fluid conduit 316 is integrally formed with the secondary bladder 314 such that the fluid conduit 316 is inseparable from the secondary bladder 312. As described above, an optional one-way valve 368 is provided as described below. If not provided, then the system is a closed system that is pressurized during a manufacturing process, and thus does not require the secondary bladder 314 or any other component of the apparatus 10 to be pressurized prior to its operation.

In the bent configuration described above as shown in FIG. 27 the inflatable chamber 314a of the secondary bladder 314 can be described as being divided into a compression section 348 and a connecting section 350. As the secondary bladder 314 is attached to a heel of a foot (further discussed below), the compression section 348 of the inflatable chamber 314a is operably disposed directly beneath the heel while the connecting section 350 is oriented in a direction principally parallel with the direction of the leg (FIG. 27). Positioning the nozzle 346 such that it extends in the direction of the connecting section 350 perpendicular to the compression section 348 prevents the nozzle 346 from being compressed as a user steps down on the compression section 348 while walking. In addition, positioning the nozzle 346 in a direction perpendicular to the compression section 348 allows the fluid conduit 316 to attach to the secondary bladder 314 parallel with the leg and prevents the fluid conduit 316 from being compressed by the ankle or leg while a person is walking.

The secondary bladder 314 is preferably equipped with at least two front and/or rear straps 352, 354 which wrap around the foot to secure the fluid secondary bladder 314 to a bottom portion of the foot. The straps 352, 354 preferably include Velcro sections 360 that overlap and attach to each other after wrapping around the foot. The front straps 352 extend from a front flap 358 that is attached to the lower wall 334 or flange 342 of the secondary bladder 336 by heat seal, glue, stitching, or other equivalent means. The front straps 352 extend away from the front flap 358. The rear straps 354 connect directly to either the flange 342 or the lower wall 334 of the secondary bladder 314 by heat sealing, glue, stitching, or other equivalent means. The rear straps 354 start at a rear portion of the secondary bladder 314 and may be pulled up towards the top of the foot in front of the leg, and wrapped over the top of the foot. The rear straps 354 also have VELCRO^{™} sections (not shown) that overlap and attach to each other on the top of the foot. In one embodiment, the front and rear straps 352, 354 are wrapped with tension over the top of the foot such that a continuous compressive force is exerted on the inflatable chamber 314a as it is positioned securely to the heel. The rear straps 354 should not be pulled so tightly that the nozzle 346 extending upward from the connecting section 350 is compressed.

Different embodiments of a secondary bladder may include a crescent-shaped with inflatable chamber 314a' and nozzle 346, or a rectangularly shaped with inflatable chamber 314a" and nozzle 346" . The secondary bladders 314' and 314" attach to the heel such that the bulk of each inflatable chamber 314a', 314a" is operably disposed underneath the heel and bottom of the foot. In such embodiments, the inflatable chamber is not divided into a compression section 348 and a connecting section 350 because most if not all of the inflatable chamber is compressed when the heel strikes the ground.

In all embodiments, the amount of fluid in the secondary bladder is chosen so that the primary bladder can accommodate all of the fluid of the secondary bladder when that fluid is forced out of the secondary bladder by the walking motion. Preferably, the system is arranged such that the primary bladder generally applies a constant pressure of 30-40 mm Hg to a wound site, and when fluid is forced out of the secondary bladder due to the walking motion, the pressure in the primary bladder is increased by an additional 10-20 mm Hg. While it is possible to tolerate higher intermittent pressures (e.g., 80 mm Hg), a maximum pressure of 50-60 mm Hg is preferred.

In the configuration of FIG. 26 and the other possible embodiments described above, the fluid conduit 316 can be affixed to the inflatable chamber 314a', 314a" of the secondary bladder 314', 314". Alternatively, the secondary bladder 314', 314" can be provided with a nozzle or connector element 346', 346" which extends through the upper wall with a first end inside the inflatable chamber, and a second end outside of the inflatable chamber. The second end of the nozzle is preferably disposed behind the heel so that the nozzle 346', 346" will not be crushed by the foot in a standing position or during the walking motion.

As shown in FIG. 26, secondary bladder 314" may be provided with front and rear straps 352", 354", which are shortened and are provided with adhesive sections 366 located at the ends of the straps 352, 354 for attachment directly to the foot (or sock).

In the embodiments of the present invention in which the fluid conduit 316 is integrally formed with and permanently connected to the bladders 312, 314, the apparatus 10 is a closed system. Such embodiments do not require the pressurization of either of the bladders 312, 314 or of the fluid conduit 316 prior to their use. Rather, during the manufacturing process of apparatus 10, the ends 316a, 316b of the fluid conduit 316 are fluidly coupled to and form airtight seals with the inflatable chambers 312a, 314a. During the heat sealing of one or both of the inflatable chambers 312a, 314a, the amount of fluid trapped in the system is controlled. Alternatively, if a one-way valve/nozzle 368 is provided, then after the fluid conduit 316 is permanently attached to the bladders 312, 314, pressurization of the therapeutic compression apparatus 300 and assembly 310 may take place by means of the one-way valve/nozzle 368 such as a check valve or relief valve. The one-way valve/nozzle 368 is used to pressurize the therapeutic compression apparatus 300 and assembly 310 to a desired pressure by connection to a fluid source such as an air pump. Once the desired pressure is reached (which may be below the ambient air pressure), the fluid source is removed from the one way valve 368, which closes. In such embodiments, nozzles 318, 346 are not necessary because the therapeutic compression apparatus 300 and assembly 310 are a closed system. Fluid flows freely between the bladders 312, 314 through the fluid conduit 316 (with the pressure in each of the bladders 312, 314 varying as the therapeutic compression apparatus 300 and assembly 310 is operated in the manner discussed below), but no additional fluid is allowed into the apparatus 10. The apparatus 10 is secured to the body by attaching the foot and primary bladders 314, 312 to the foot and leg as discussed herein. The one way valve 368 such as a check valve or relief valve may alternatively be located on either of the inflatable chambers 312a, 314a of the bladders 312, 314 rather than on the fluid conduit 316.

In other embodiments, the fluid conduit 316 is detachably connected to the nozzles 318, 346 of the primary bladder 312 and secondary bladder 314. In such embodiments, the ends 316a, 316b of the fluid conduit may be inserted through the nozzles 318, 346 to fluidly couple the primary bladder 312 with the secondary bladder 314 in an airtight manner. The air-tight connection between the fluid conduit 316 and the nozzles 318, 346 may be accomplished by male and female threaded surfaces, bayonet locks, or other equivalent means known in the art. The nozzles 318, 346 may contain two way valves (not shown) for pressurizing either or both of the inflatable chambers 312a, 314a prior or subsequent to attaching the fluid conduit 316. The fluid conduit 316 is preferably made from rubber or plastic and by way of example and not by way of limitation has a diameter in the range of 0.635 to 1.27 cm (1/4 to 1/2 inch), a length in the range of 5.08 to 30.48 cm (two to twelve inches), and walls having a thickness in the range of 0.3175 to 1.27 cm ( 1/8 to 1/2 inch). This wall thickness is recommended to prevent or minimize expansion of the fluid conduit 316 as the pressure varies therein throughout the operation of the therapeutic compression apparatus 300 and assembly 310 (i.e., the tube preferably can withstand pressures of 60 mm Hg and above without expanding).

In embodiments in which the fluid conduit 316 is permanently attached to the bladders 312, 314, the therapeutic compression apparatus 300 and assembly 310 are assembled by simply attaching the foot and primary bladders 314, 312 to the foot and leg as described herein. No additional set-up is necessary.

In embodiments in which the fluid conduit 316 is detachably connected to the bladders 312, 314, the apparatus may be assembled by either first attaching the foot and primary bladders 314, 312 to the foot and leg, and then connecting the fluid conduit 316 to the nozzles 318, 346, or by first connecting the fluid conduit 316 to the foot and primary bladders 314, 312, and then connecting the foot and primary bladders 314, 312 to the foot and leg. In such embodiments, a fluid is supplied into either the inflatable chamber 312a of the primary bladder 312 or the inflatable chamber 314a of the secondary bladder 314 (or both) through the nozzles 318, 346 from a fluid source such as an air pump. As fluid is supplied, the bladder(s) 312, 314 will inflate and pressurize. When the fluid source is removed from the nozzles 318, 346, the valves in the nozzles will maintain the pressure in each of the bladders 312, 314. The fluid conduit 316 may then be pinched at one of the two ends 316a, 316b while the other of the two ends 316a, 316b is coupled to one of the nozzles 318, 346. The fluid conduit 316 opens the valve within the nozzle as it enters therein. The pinched end of the fluid conduit 316 is then coupled to the other nozzle, which opens that nozzle's valve, and the apparatus 10 is then ready for operation. The fluid used inside of the therapeutic compression apparatus 300 and assembly 310 may be air, liquid, or a combination of both depending on the fluid source desired, the pressure desired, and the specific materials used.

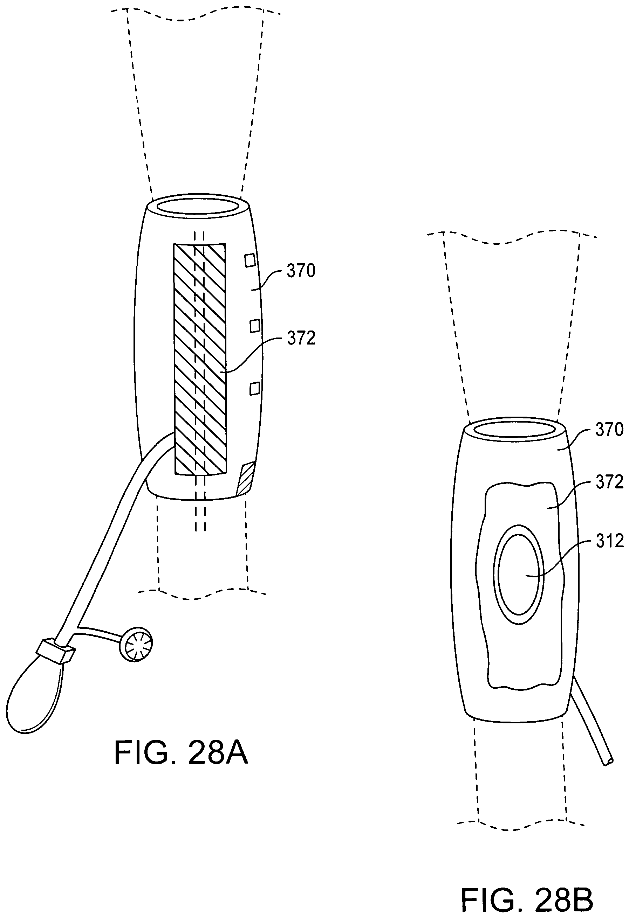

In yet another embodiment of the present invention, the primary bladder 312 is positioned on the leg by means of a flexible leg wrap apparatus 370 such as the one disclosed in the commonly owned U.S. Pat. No. 7,276,037. The leg wrap apparatus 370, shown in FIGS. 28A-28B, has an inflatable air bladder chamber 372 secured to a flexible wrap member 373. The flexible member 373 is designed to wrap around the leg in a wrapped configuration with the air bladder chamber 372 being operably disposed on an interior surface of the flexible member 373 such that the flexible member 373 exerts pressure over a large area of a leg. In this embodiment, no straps or other attachment means need be connected to the primary bladder 312 because the flexible member 373 serves the dual function of positioning the primary bladder 312 on the leg (the primary bladder 312 being sandwiched between the inflatable air bladder chamber 372 and the leg) and providing additional pressure to other areas of the leg.

In one embodiment, the inflatable chambers 312a, 314a of the leg and secondary bladders 312, 314 are prefilled such that when they are secured to the leg and foot and used in conjunction with the leg wrap apparatus 370, the leg wrap apparatus causes the primary bladder 312 to substantially deflate when a patient is lying down or has the foot elevated while walking (i.e. when the foot is not compressing the secondary bladder 314). In such embodiments, it is preferred that the fluid displaced from the primary bladder 314 to the secondary bladder 312 cause the inflatable chamber 312a of the secondary bladder 312 to inflate to approximately one-half to three-quarters of its maximum volume. In these embodiments, the leg wrap apparatus 370 alone puts pressure on the saphenous vein of the patient while the patient is lying down or has his or her foot elevated. When the secondary bladder 314 is compressed during the standard walking motion, fluid is displaced from the secondary bladder 314 back to the primary bladder 312, increasing the pressure therein. This intermittent on and off compression will help to promote circulation to an ulcer bed disposed on the patient's leg underneath the primary bladder 312 and leg wrap apparatus 370.

The inflatable air bladder 372 is optionally provided with a pressure gauge and an automatic pressure relief valve coupled to the inflatable air bladder chamber 372 to vent air from the chamber 372 to the ambient environment when the internal pressure reaches a threshold maximum pressure. For example, the therapeutic compression apparatus 300 and assembly 310 may be attached to the leg and foot as described above in any of the embodiments. The flexible member 373 is then wrapped with tension around the leg and over the primary bladder 312 with the air bladder chamber 372 touching the inflatable chamber 312a and exerting a pressure thereon (FIGS. 28A and 28B). If during operation of the therapeutic compression apparatus 300 and assembly 310, the pressure in the inflatable air bladder 372 becomes higher than the threshold maximum, then the automatic pressure relief valve releases air from the chamber 372, which reduces the tension in the flexible member 373. The flexible leg wrap apparatus 370 may be used in conjunction with all embodiments of the apparatus 10 disclosed herein.

Once the therapeutic compression apparatus 300 and assembly 310 is assembled and fastened to a person as discussed above, the therapeutic compression apparatus 300 and assembly 310 operates as a person walks. During the standard heel to toe motion of walking, the compression section 348 of the secondary bladder inflatable chamber 314a is squeezed between the heel and the ground, which puts external pressure on the compression section 348 forcing all or most of the fluid out thereof, (i.e. the compression section 348 of the inflatable chamber 314a of the secondary bladder 314 deflates as a heel strikes the ground). Fluid is thus pushed Up through the connecting section 350 of the inflatable chamber 314a, into and through the fluid conduit 316, and up into the primary bladder 312. The inflatable chamber 312a of the primary bladder 312 inflates to accommodate the entering fluid. As the inflatable chamber 312a inflates, the sidewall 326 of the inflatable chamber 312a presses against the straps 332 of the primary bladder and/or the flexible member 373 of the leg wrap apparatus 370. The straps 332 and/or flexible member 373, which are securely fastened to the leg with tension and preferably extend over the top of the inflatable chamber 312a, provide resistance to the sidewall 326 flexing or bowing outward, which limits the volume increase of the inflatable chamber 312a. The additional fluid entering the inflatable chamber 312a thus causes an increase in the pressure therein. This increased pressure is transmitted through the lower wall 320 and/or bottom layer 330 to the leg.

When the person's foot rolls from heel to toe, the external pressure from the person's weight is removed from the heel. At that point, the pressure in the fluid conduit 316 and the inflatable chamber 312a of the primary bladder 312 is greater than the pressure in the inflatable chamber 314a of the secondary bladder 314. Because in equilibrium the pressures will be equal, a portion of the fluid in the primary bladder 312 flows back through the fluid conduit 316 into the inflation chamber 314a of the secondary bladder 314, which expands back to its original state, until the pressure therein equals the pressure in the primary bladder 312 and the fluid conduit 316. This process repeats as the person walks, which creates a pumping or kneading force on the wound area of the leg over which the primary bladder 312 is placed as the pressure in the primary bladder 312 increases and decreases, thereby promoting blood flow, drainage, treatment, and healing to various parts of the leg.

The pressure in the inflatable chamber 312a of the primary bladder 312 is impacted by a number of factors. It should be noted that if the primary bladder 312 is pressurized before placing it on the leg, then the pressure inside the inflatable chamber 312a will increase when it is placed on the leg prior to the straps 332 being secured because the volume of the inflatable chamber 312a decreases slightly as the lower wall 3320 curves to conform to the shape of the leg. The straps 332 further increase the pressure on the inflatable chamber 312a as they are secured thereto. In addition, in embodiments in which the fluid conduit 316 is detachable, the pressure in the apparatus 10 may be varied by pressurizing one or both of the primary bladder 312 and secondary bladder 314, or by using a longer or wider fluid conduit 316, which increases the internal volume of the apparatus 10. The pressure may also vary as a function of the tightness with which the straps 332 are wrapped around the leg and/or placed over the inflatable chamber 312a, and the tension with which the straps 352, 354 are wrapped around the foot and/or placed over the inflatable chamber 314a. For example, a greater tension of the straps around the leg and/or inflatable chamber 312a of the primary bladder 312 produces a greater inwardly directed (minimum) compressive force on the leg. The operation of the apparatus 10 to inflate the inflatable chamber 312a will produce additional pressure on the leg as the inflatable chamber 312a inflates, encounters resistance from the straps and continues to fill with fluid without a corresponding increase in volume.

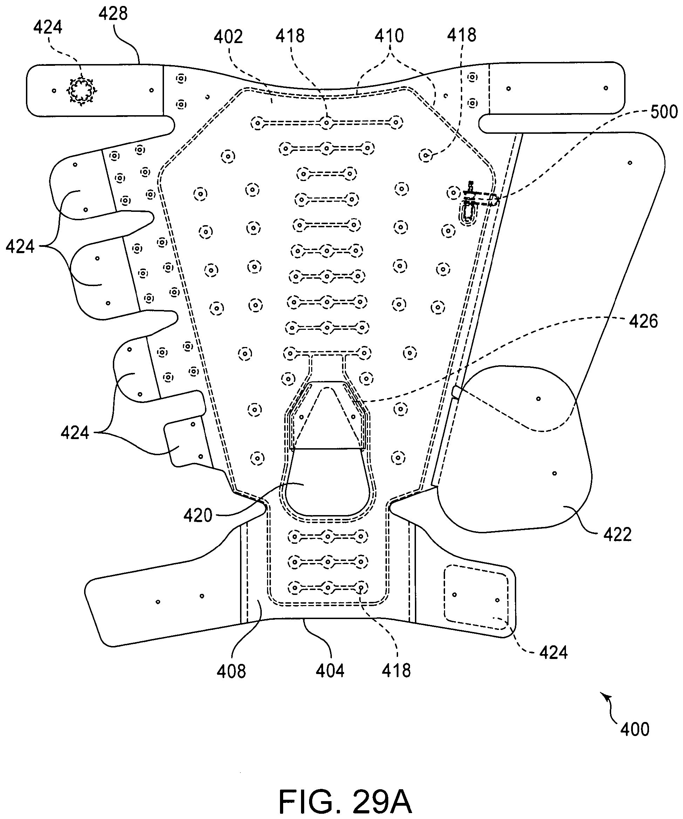

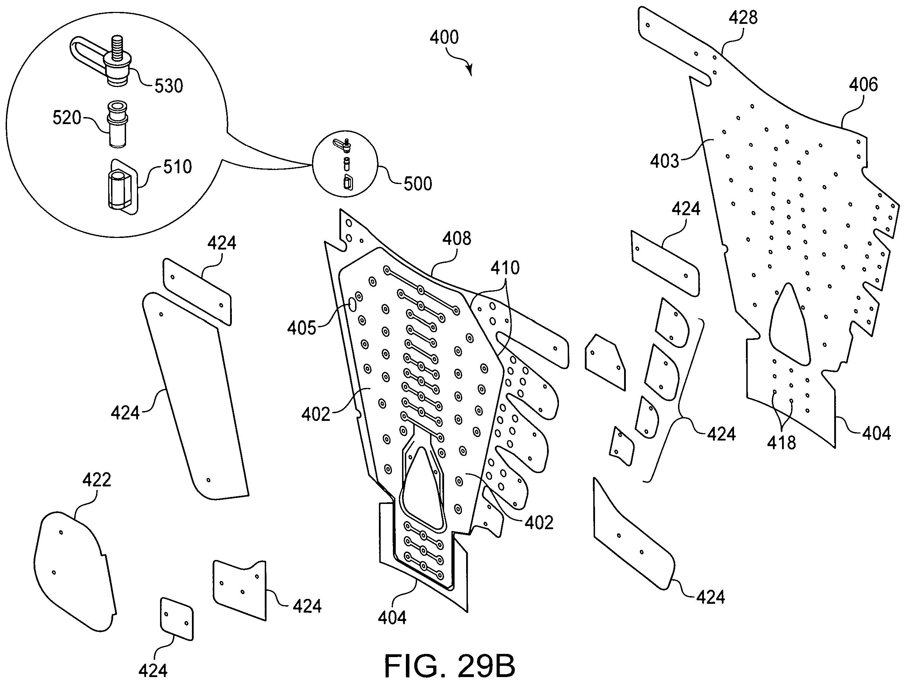

Yet another embodiment of the present invention is shown in FIG. 29 of a therapeutic compression apparatus 400 constructed in accordance with the present invention, showing compression bladder 402 integrally formed in therapeutic compression apparatus 400. In this instance the compression bladder 402 is one bladder within both the primary and secondary wraps 403, 404. Therapeutic compression apparatus 400 is configured and adapted to wrap around a patient's limb such as for instance the lower leg through the use of primary wrap 403 (such as a calf wrap) and a secondary wrap 404 (such as a secondary wrap 404), each of said wraps which are formed out of continuous outer sheet 408 and inner sheet 406. Therapeutic compression apparatus 400 is a wrap member with a proximal end portion (top as oriented in FIG. 29A and 29B) and opposed distal end portion (bottom as oriented in FIG. 29A and 29B) which is configured and adapted to conform around a patient's limb such as for instance a lower leg and provide compression through the inflation of bladder 402, which may be a primary bladder or the sole bladder in the therapeutic compression apparatus 400. In one embodiment of the present invention inner sheet 406 and outer sheet 408 are made out of a nylon laminated polyurethane sheet which are configured and adapted to be RF welded together. However, any other suitable materials which are weldable or otherwise joined while being airtight can be used. Continuous peripheral weld line 410 forms an airtight boundary of integrally formed bladder 402. In this exemplary embodiment, bladder 402 is a single continuous bladder throughout the primary wrap 403 and secondary wrap 404. However, it is envisioned that secondary wrap 404 can have an independent bladder either separately inflatable or inflatable through a one-way valve or other desired inflation/deflation configuration. Secondary wrap 404 can also be configured and adapted to provide a differing pressure from primary wrap 403. In an exemplary embodiment, bladder 402 located in secondary wrap 404 is configured and adapted to be located along the underside of a patient's foot. Bladder 402 in secondary wrap 404 can be adjusted as desired to provide compression to the desired part of a patient's foot.

Hook and loop fasteners 424 are provided along the edge of inner and outer sheets in order to ease adjustment and secure therapeutic compression apparatus 400 on a patient's lower leg and foot. It is envisioned that therapeutic compression apparatus 400 can also be secured to a patient's lower leg by other means, such as zippered, buttoned, or be cuff shaped by other such suitable means. Further, it is also envisioned that hook and loop closures 424 can be replaced by material similar to that of ankle strap 422 described below and be welded/sewn/attached to bladder 402 for improved comfort.

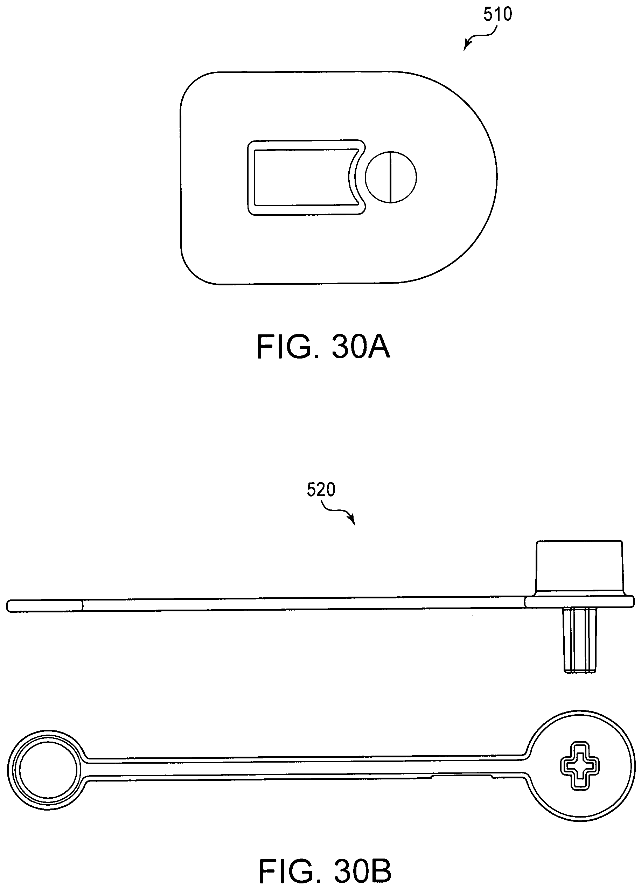

In one embodiment of the present invention inflation means 700 is a hand pump which can attach to inflation port 500 to inflate bladder 402. It can be appreciated that a mechanical or automatic inflation pump (not shown) can also be attached to inflation port 500 to inflate and deflate bladder 402 to provide pulsating pressure to a user's lower leg. A number or variety of inflation means can be employed not limited to a manual pump, hand pump, foot pump, mechanical pump, electrical pump, battery-operated pump, static pump, intermittent pump, varying pump, automatic pump, pneumatic pump, negative pressure pump, suction pump or vacuum, pulsing pump, or any other known or developed source of inflation so as to provide a certain pressure within the bladder so to provide compression in use by the patient. A vent valve is also be incorporated into therapeutic compression apparatus 400 or with inflation means 700 to allow a user to selectively deflate bladder 402. Further, a relief valve is also incorporated with either inflation means 700 or bladder 402 to prevent overinflation once a maximum pressure is detected. Examples of relief valves are described in U.S. Pat. No. 7,276,037 and U.S. Pat. No. 7,850,629. Further examples are shown in FIGS. 30A and 30B.

In another embodiment of the present invention the therapeutic compression apparatus 400 can be formed by first forming bladder 402 to be integral within inner sheet 406 and outer sheet 408, the location and desired preconfigured compression gradient profile can be obtained cost-effectively. A number of different embodiments of bladder configurations can be used in the therapeutic compression apparatus of the subject invention such as those configurations described above. In another embodiment therapeutic compression apparatus 400 may have a bladder 402 with a plurality of spot welds 414 therein. Spot welds 414 may be strategically placed within bladder 402 in a predetermined pattern based on the desired gradient profile relative to the compression needed at the patient's treatment site. Spot welds 414 enable bladder 402 to define the gradient profile when inflated through inflation port 500. The geometric placement of spot welds 414 within bladder 402 allows increased inflation of certain portions of bladder 402, and can create one or more fluid chambers within bladder 402. This configuration is particularly useful when compression is needed to improve fluid movement (e.g., blood, lymph, etc.) within the body. Further, linear weld lines 416 allow for better compression along the back of a patient's calf by increasing tension applied to the back of the calf of a patient. This increased tension can generate a more effective calf compression in order to increase venous flow. Linear weld lines 416 located laterally along the back of the calf create a ribbed portion, which keeps the inflated profile of therapeutic compression apparatus 400 compact which can further increase ambulation and reduce interference with a patient's clothes. Secondary wrap 404 can also be made from an elastic garment without bladder 402.

It can be appreciated that depending on the location of the therapeutic compression apparatus being placed on the patient's body part or limb, different pressure gradients may be utilized. Examples of other bladder pressure gradient profiles are described in U.S. patent application Ser. No. 12/911,563 and U.S. patent application Ser. No. 12/855,185.

Once therapeutic compression apparatus 400 is secured around a patient's limb such as for instance a lower leg, bladder 402 is not able to shift out of place, thus increasing comfort and reducing fitting issues on the patient. In order to increase the ease of ambulation by a patient, in an exemplary embodiment, ankle cushion 426 can be attached adjacent heel port 420 to prevent the occurrence of a pinch point and reduce pressure on a patient's Achilles tendon. In combination with ankle cushion 426, ankle strap 422 can be used. In an exemplary embodiment, ankle strap 422 can include non-elastic foam which prevents a pinch point at the bottom of lower leg wrap 403 and the upper part of secondary wrap 404. A further advantage to providing ankle strap 422 is that bladder 402 proximate ankle strap 422 is pulled tight against a patient's leg and improves compression near the heel of a patient. Ankle strap 422 is advantageously wrapped around the patient's ankle and foot prior to affixing hook and loop fasteners 424. In order to improve comfort, through-holes 418, as seen in FIG. 29, are located throughout therapeutic compression apparatus 400 in order to allow for ventilation about a patient's leg during extended wear of therapeutic compression apparatus 400. For the sake of clarity, not all of the through-holes 418 are identified with reference characters in the Figures.

In accordance with an exemplary embodiment, inner sheet 406 further includes a layer (not shown) that has a first elastic modulus, inner sheet 406 has a second elastic modulus. The first elastic modulus is less than the second elastic modulus in a transverse direction relative to the proximal and distal end portions of therapeutic compression apparatus 400 to wrap therapeutic compression apparatus 400 around the leg when the leg compression bladder is inflated. In an exemplary embodiment, inner sheet 406 includes a secondary sheet (not shown) disposed on an inner surface thereof, to directly contact the lower leg in use. The secondary sheet can be a fabric layer, which is elastic in a first direction and inelastic in a second direction to curl the wrap member around the leg when the leg compression bladder is inflated.

In another exemplary embodiment, upper leg strap 428 is configured and adapted to improve wearability of therapeutic compression apparatus 400 by locating a portion of bladder 402 above the widest portion of the calf of a patient and provides stability of therapeutic compression apparatus 400 by preventing therapeutic compression apparatus 400 from slipping down the lower leg of a patient which would make therapeutic compression apparatus 400 ineffective in providing calf compression.

The inflation means or mechanism for each of the various embodiments of the present invention may include a hand pump, electric pump, battery-operated pump, remote controlled pump, air pump, gas pump, or any other known inflation means. A number or variety of inflation means can be employed such as a manual pump, hand pump, foot pump, mechanical pump, electrical pump, battery-operated pump, static pump, intermittent pump, varying pump, automatic pump, pneumatic pump, negative pressure pump, suction pump or vacuum, pulsing pump, or any other known or developed source of inflation so as to provide a certain pressure within the bladder so to provide compression in use by the patient. FIGS. 30A and 30B include various valves to be used in the present invention. Further, the inflation means could include a means to monitor or regulate the inflation. The inflation means could include programming such that the bladders are inflated and deflated to a set pressure at intervals or at set times throughout the day or night when the compression apparatus is in use worn on the patient. For instance, by way of example only, the inflation means could be set to 40 mm-Hg at 9 am and then set to deflate to 20 mm-Hg at 11 am and then set to inflate to 30 mm-Hg at 12 pm and so on throughout the day and night for each patient individually. In one embodiment of the invention the dial 610 includes graphics of pressure amount such as "35, "45', "55' and "65" or lettering such as "A", "B", "C", "D" which each would correspond to a certain pressure such as 25 mm-Hg, 35 mm-Hg, 45 mm-Hg and 55 mm-Hg. The specific pre-determined pressure to correspond with the graphic is endless and not limited by the examples herein.

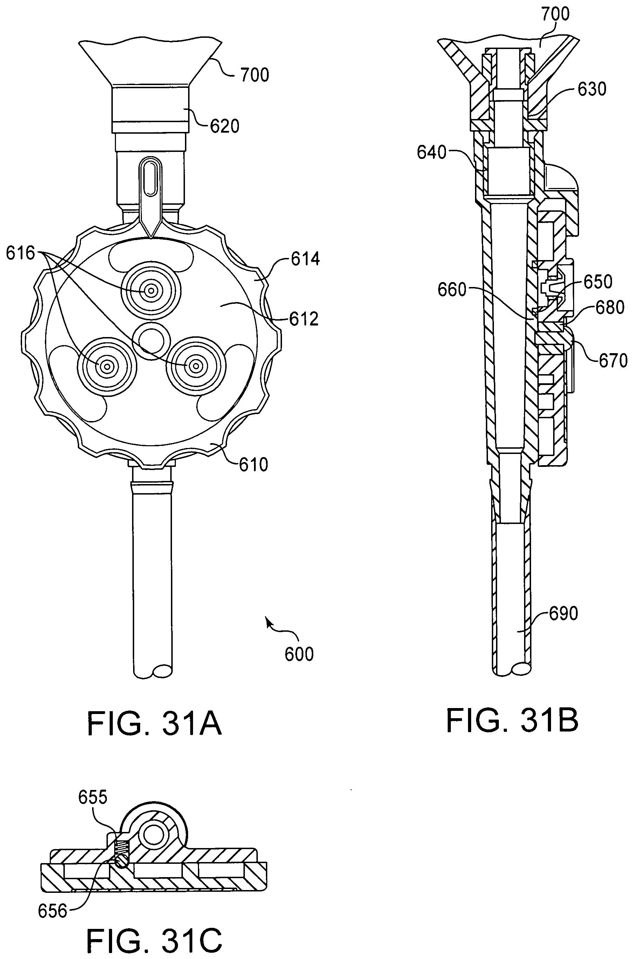

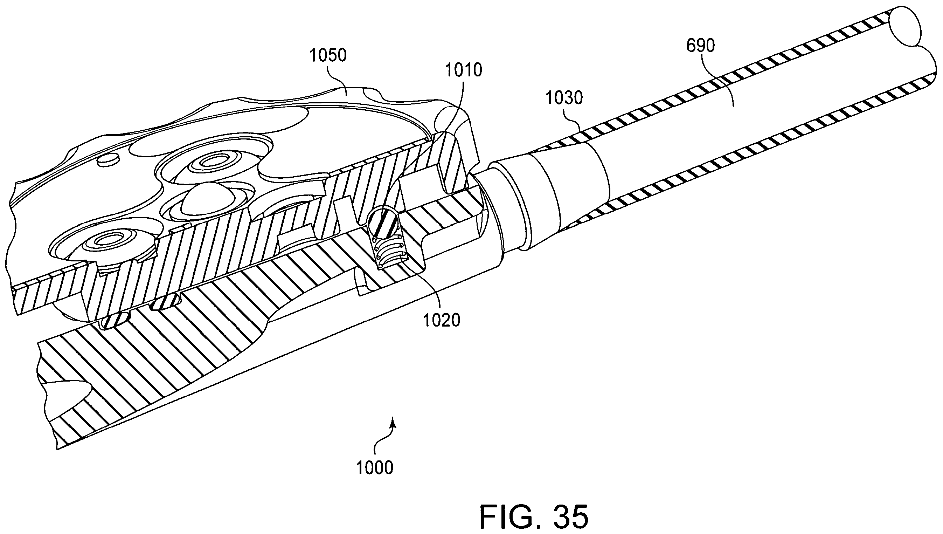

Further, the therapeutic compression apparatus may be deflated by the valve cap or in another embodiment has a button or a switch to deflate the primary and/or secondary bladder and thus release the pressure. In any embodiment where there is one bladder running the length of both the primary wrap and secondary wrap, the term "primary and/or secondary bladder" shall mean the sole primary bladder or compression bladder (402). In another embodiment as shown in FIGS. 31A-31C and 35, the switch may have a plurality of integrated umbrella valves so that one umbrella valve is set and closed to maintain the pressure within the primary and/or secondary bladder, while a second umbrella valve would release a certain amount of air or fluid within the primary and/or secondary bladder so as to release the pressure such as while the patient is walking (pressure increases on the calf with each step) or flying (pressure increases based on altitude), and a thirds umbrella valve which would release all the air or fluid in the primary and/or secondary bladder and thus release all pressure and deflate the therapeutic compression apparatus. For instance by way of example only, the first umbrella valve is set in a closed position so that when activated this umbrella valve maintains the amount of air or fluid in the primary and/or secondary bladder and thus maintains the set pressure, say for instance at 45 mm-Hg, the second umbrella valve is set to release the air or fluid within the primary and/or secondary bladder if the pressure within exceeds 45 mm-Hg and bring the pressure down to 45 mm-Hg (such as when in high altitude or other increases in pressure) and then maintain the pressure at 45 mm-Hg, and a third umbrella valve is set to open and release all the air or fluid within the primary and/or secondary bladder and thus release all pressure when activated by the patient so as to deflate the primary and/or secondary bladder and the therapeutic compression apparatus. By way of another example, the dial may include graphics such as (A) "Walk" wherein the set pressure amount is maintained while the patient walks and the pressure spikes and returns over and over in time as the umbrella valve remains in the closed position, (B) then a graphic of "Air" wherein the set pressure amount will be maintained by this umbrella vale occasionally releasing pressure as the pressure increases over the set amount or value so that the umbrella valve is activated to release air or fluid within the primary and/or secondary bladder and release the pressure yet then close and stay closed to maintain the set pressure amount, and (C) "Release" or "Deflate" wherein the pressure will be released and the air or fluid within the primary and/or secondary bladder released to deflate and this umbrella valve is always in the open position. In this embodiment there are three umbrella valves with one set to always open the primary and/or secondary bladder to release pressure completely, one set to always close to maintain air or fluid in the primary and/or secondary bladder to maintain pressure, and a third set to open or release at a predetermined or set pressure point. In all of the embodiments referring to umbrella valve the umbrella valve may also be a switch (manual or otherwise) or a digital switch or any other known means to open, close or partial release air or fluid within a bladder and thereby maintain, change or release pressure therein.

The inflation means and valves shown in FIGS 30A and 30B include an inflation port having an elbow connector 510 which is welded to the compression apparatus to allow air to flow into it. A check valve or relief valve is inserted into the elbow connector 510. A dust cap 520 or valve cap is included to prevent dust clogging the check valve and if inserted in a reverse configuration the inflated bladder is deflated manually. This is spring loaded so that the inflation means can inflate the bladder and once the inflation means is removed the spring seals the bladder such that the pressure is not released until manually or automatically decreased or deflated. The check valve may also be non-spring activated and activated by any other known means. The inflation means may include a bulb, motorized, batter-operated or electric means. The inflation means may include the dials shown or a simpler dial or one valve. Further embodiment are shown in FIGS. 31-38.

The inflation means 700 shown in FIGS. 31A and 31B and 31C include a hand pump 700. The inflation means 600 includes a tube 690 connected to a pump assembly including a valve dial 614 and in this embodiment three umbrella valves 616, as well as a label 612 which the user can use to see the various pressure amounts for the therapeutic compression apparatus 10, 100, 200, 300, or 400. The valve dial 614 is connected to the hand pump bulb 700 by a valve base 620. The hand pump bulb 700 may be a squeeze bulb or any other known hand pump or ball. As shown in FIG. 31B, which is a cross section of FIG. 31A, the valve dial 614 may include a check valve which is a safety component such that the user does not overinflate the compression apparatus and cause harm to himself or herself. The check valve 630 may be joined to the valve base 620 via any known means, such as for example only, adhesive 640. The valve dial 614 may further include an O-ring 650 and a lubricant, such as by way of example only grease 660, applied to the O-ring 650. Further, the valve dial 614 may include a drive screw 670 and a washer 680. The inflation means 600 may further include, as shown in FIG. 31C, a detent spring 655 and a detent ball 656. The detent spring 655 and detent ball 656 has an audible "click" and provides a tactile and audible way for the user to know the pressure is changing.

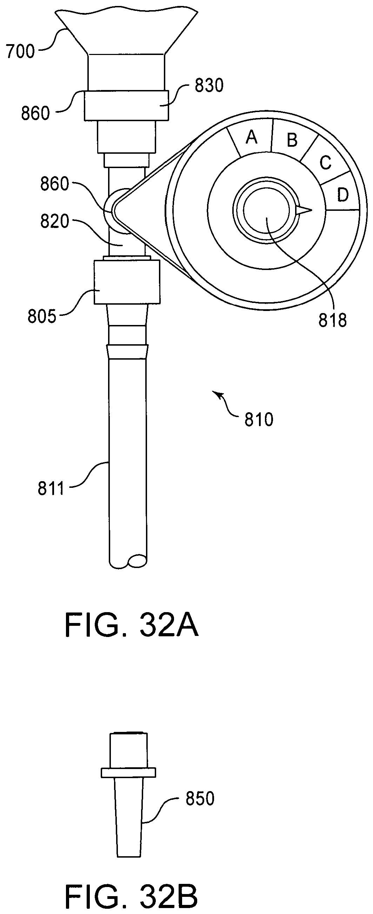

Another embodiment of the inflation means is shown in FIGS. 32A and 32B, 33 and 34. The inflation means 810 includes a tube 811 and a dial 818. The dial 818 is a monometer type and the arrow moves around based on the pressure via the bulb 700. The dial 818 is connected to a check valve 830 so that the user cannot overinflate the compression apparatus. The dial 818 is connected to the tube 811 and the check valve 830 via any known joining means, and in this embodiment is shown by way of example only, as a t-connector 820.

Another embodiment of the inflation means (not shown) is includes a plunger assembly. The plunger assembly includes an umbrella valve (similar to the umbrella valves 616 shown in FIG. 31A). The umbrella valve includes an O-ring assembled into a slot to prevent air leakage out of the spring hole. The plunger assembly has a predetermined height so as to control relief pressure in the dial 618 or dial valve 610. The plunger assembly further includes at least one or multiple relief slots which allow air pressure to access the umbrella valve itself.

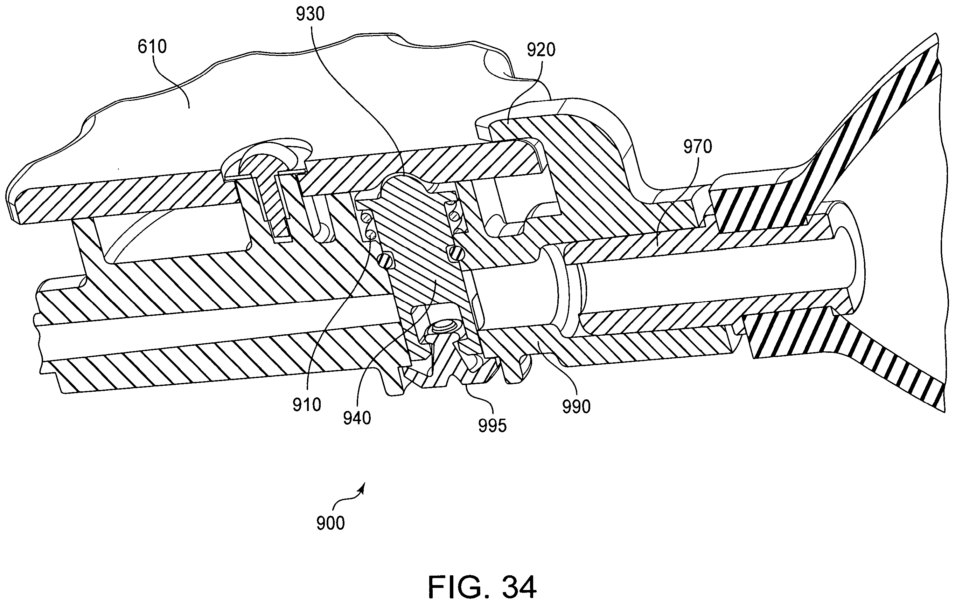

FIG. 34 is a cross-section view of another embodiment of the inflation means wherein the dial 610 includes a recess 930 in varying increments such that the umbrella valve 910 stretches and has different relief pressures. A pointer 920 is included in this embodiment which resists the spring load and holds the dial 610 down at a precise height. The compression spring 910 drives the valve retainer upwards within the inflation means 900. The dial 610 has the pointer 920 located above the slide 940. The compression spring 910 and O-ring 990 are located along the slide 940. The umbrella valve 10 is connected below the slide 940. The inflation means further includes a washer 950 and a screw drive 960. Also shown are the check valve 980 and the valve base 980. A distal end of the blub 700 is also shown.

Another embodiment of the inflation means 1000 is shown in FIG. 35 including a dial 1050 having below a detent ball 1010 and a detent spring 1020. The detent ball 1010 and detent spring 1020 act as an audible and tactile manner for the user to know the pressure change. The insertion tip 1030 is shown joined to the tube 690. In this embodiment the three umbrella valves may be set so that one is always in the open position to release the air or fluid in the primary and/or secondary bladder and thus release the pressure for a deflated position of the bladder(s), the second umbrella valve may be set in a closed position so as to maintain the air or fluid in the primary and/or secondary bladder and maintain the pressure, and the third umbrella valve is at a set pressure amount or value such that as the pressure increases the umbrella valve opens and releases the excess pressure and then the umbrella valve closes to maintain the set pressure amount or value.

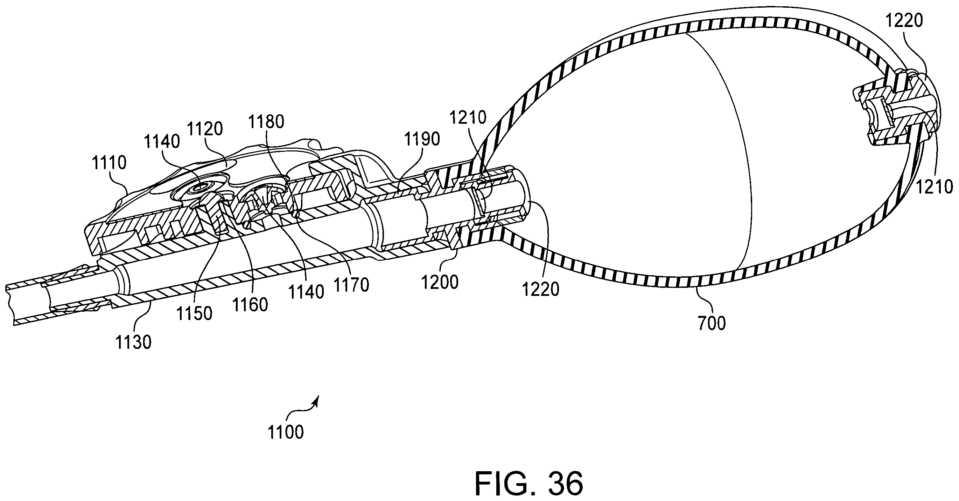

Another embodiment of the inflation means 1100 is shown in FIG. 36. The dial 1110 includes a label 1120 for the user to visualize the pressure amounts in use. The dial 1610 further includes at least one, and in this embodiment cross-section shown as two or three, umbrella valves 1140. In this embodiment the three umbrella valves may be set so that one is always in the open position to release the air or fluid in the primary and/or secondary bladder and thus release the pressure for a deflated position of the bladders, the second umbrella valve may be set in a closed position so as to maintain the air or fluid in the primary and/or secondary bladder and maintain the pressure, and the third umbrella valve is at a set pressure amount or value such that as the pressure increases the umbrella valve opens and releases the excess pressure and then the umbrella valve closes to maintain the set pressure amount or value. Further included are a drive screw 1150 and washer 1160. An O-ring 1170 is included and lubricated via grease 1180. The valve body 1130 is joined to a connected bulb 700 via any known connecting or joining means, and in this embodiment by way of example only, is an adhesive 1190. The check housing 1220 includes the check valve and a silicon disk 1210.

In another embodiment of the present invention the compression apparatus may be a stand-alone thigh compression or thigh compression portion added to the leg and foot compression apparatus of the various other disclosed embodiments. The thigh compression apparatus includes an inner layer and an outer layer. The outer layer has joined to it an inflation port which is capable of connecting or joining to an inflation means. The inner layer includes a plurality of fasteners. In one embodiment the thigh compression apparatus includes, by way of example only, hook and loop fasteners along the edge to ease adjustment and secure therapeutic compression apparatus on a patient's thigh. Other uses of the thigh compression apparatus may be used such as on the back, calf, arm, stomach, torso, shoulder and other body parts, such that the designation as the "thigh" compression apparatus is not limited to only use of such apparatus on the thigh of a patient. It is envisioned that compression apparatus can also be secured to a patient's thigh or other body part by other means, such as zippered, buttoned, or be cuff shaped by other such suitable means.

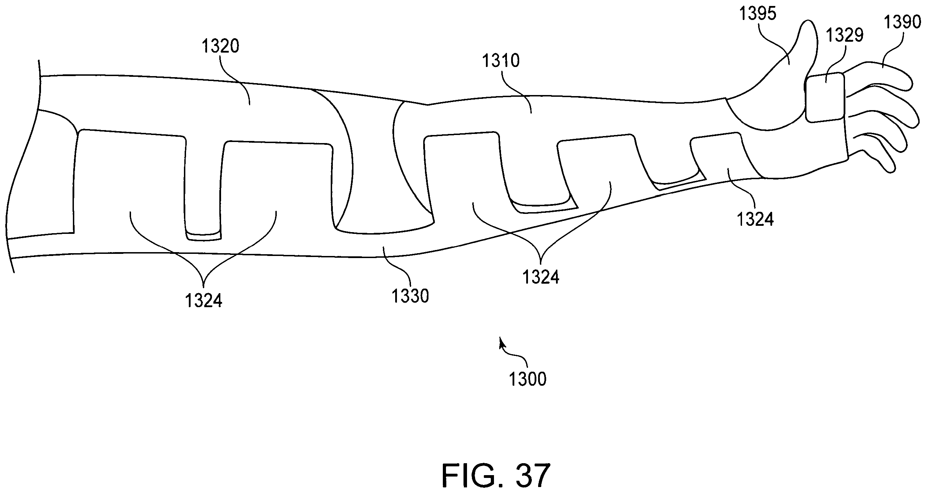

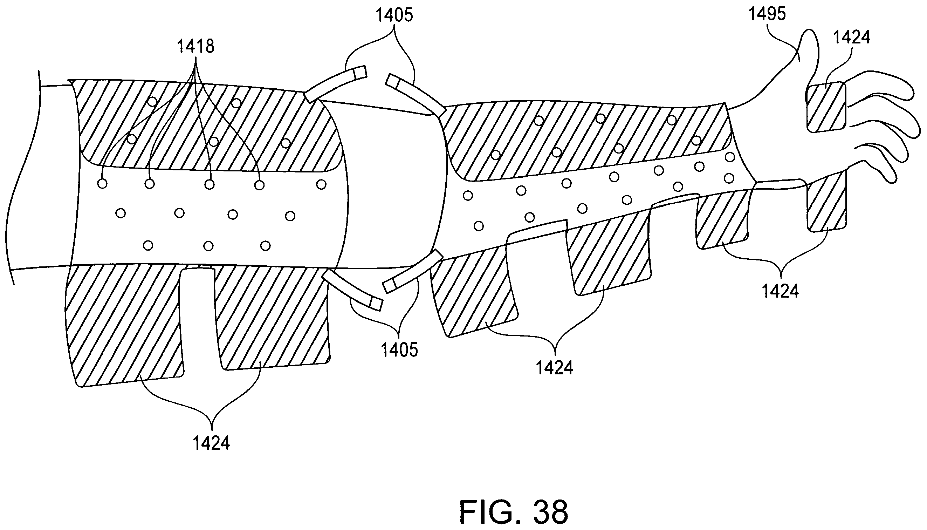

FIGS. 37 and 38 show yet another embodiment of the present invention with the compression apparat is being configured to be used on a patient's arm. The arm compression apparatus 1300 includes a layer 1310 and an inflation port 1305 which is capable of connecting or joining to an inflation means. The outer layer 1310 includes a plurality of fasteners 1324 which may fasten around the patient's thumb 1395 and fingers 1390. In one embodiment the thigh compression apparatus 1300 includes, by way of example only, hook and loop fasteners 1324 along the edge to ease adjustment and secure therapeutic compression apparatus 1300 on a patient's arm. Other uses of the arm compression apparatus 1300 may be used such as on the back, shoulder, torso, calf, arm, stomach and other body parts, such that the designation as the "arm" compression apparatus is not limited to only use of such apparatus 1300 on the arm of a patient. It is envisioned that compression apparatus 1300 can also be secured to a patient's arm or other body part by other means, such as zippered, buttoned, or be cuff shaped by other such suitable means. In order to improve comfort, through-holes 1313, as seen in FIG. 38 are located throughout compression apparatus 1300 in order to allow for ventilation about a patient's arm, or other body part, during extended wear of compression apparatus 1300. For the sake of clarity, not all of the through-holes 1313 are identified with reference characters in the Figures. The compression apparatus 1300 may include a portion 1330 for use around the patient's elbow as shown in FIG. 37 and may include an aperture. In another embodiment as shown in FIG. 38 two smaller compression apparatus (compared to the apparatus in FIG. 29A) are used on the lower arm and the upper arm of the patient with a plurality of tabs secondary bladder capable of connecting to each other such as via hook and loop closure or VELCRO^{™} or any other known connecting means such as buckles, straps, buttons, snaps, zippers and other combinations. As shown in FIG. 38, the at least two therapeutic compression apparatus 1400 which are capable of connecting or joining to an inflation means and capable of connecting to each other via tabs 1405. The outer layer 1410 includes a plurality of fasteners 1424 which may fasten around the patient's thumb 1495 and fingers. Other configuration may be employed so that the patient may apply pressure and compression to parts of the leg, arm, wrist, hand, shoulder, waist, torso, chest and the like.

The inventive therapeutic compression apparatus may be included in a kit having various wound dressings and/or bandages. The wound dressings and/or bandages may be disposed of on a more frequent basis and the inventive therapeutic compression apparatus is applied in conjunction or combination with the wound dressings and/or bandages. In one embodiment the therapeutic compression apparatus is used over or on top of the wound dressing applied to the skin.

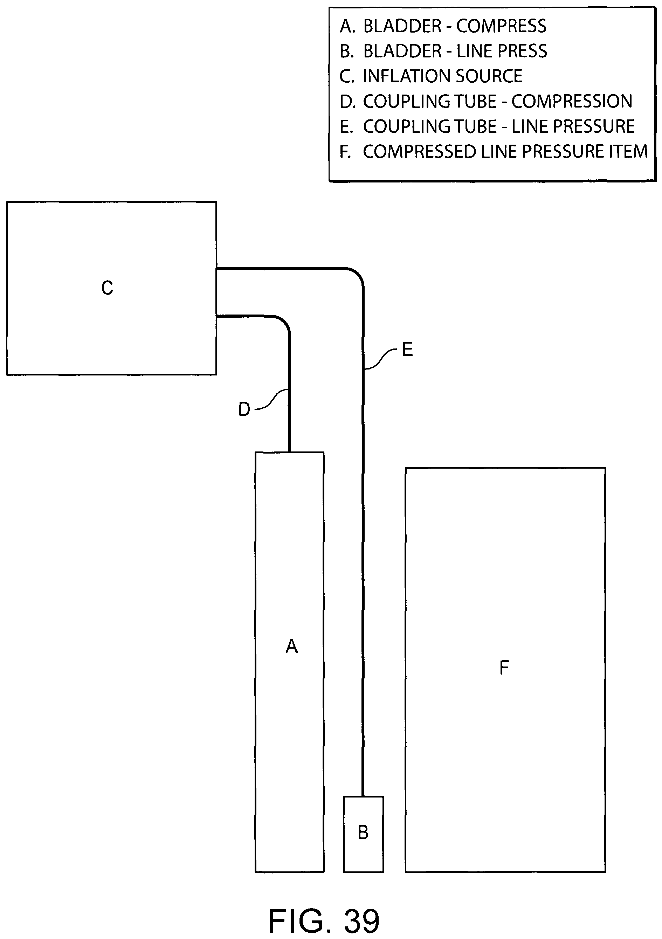

Another embodiment of the present invention includes a method of applying a measured compression amount with feedback. As shown in FIG. 39, Compression Bladder A is inflated by Inflation Source C - the nature of Compression Bladder A is such that the amount of compression is determined by the amount of inflation medium (typically air) pumped into A from C. In this design, Inflation Source C is also coupled with Bladder B, which has a fixed volume of air. When Compression Bladder A inflates, it will squeeze Bladder B as it compresses Compressed Item F. Inflation Source C is able to read the line pressure from the Coupling Line E to determine the interface pressure from Bladder B - in this design, Inflation Source C can be calibrated to provide only the amount of inflation medium necessary into Compression Bladder A as determined by matching the desired interface pressure from Bladder B. Other configurations may be employed so that feedback may be obtained from the inflation means and compression apparatus.

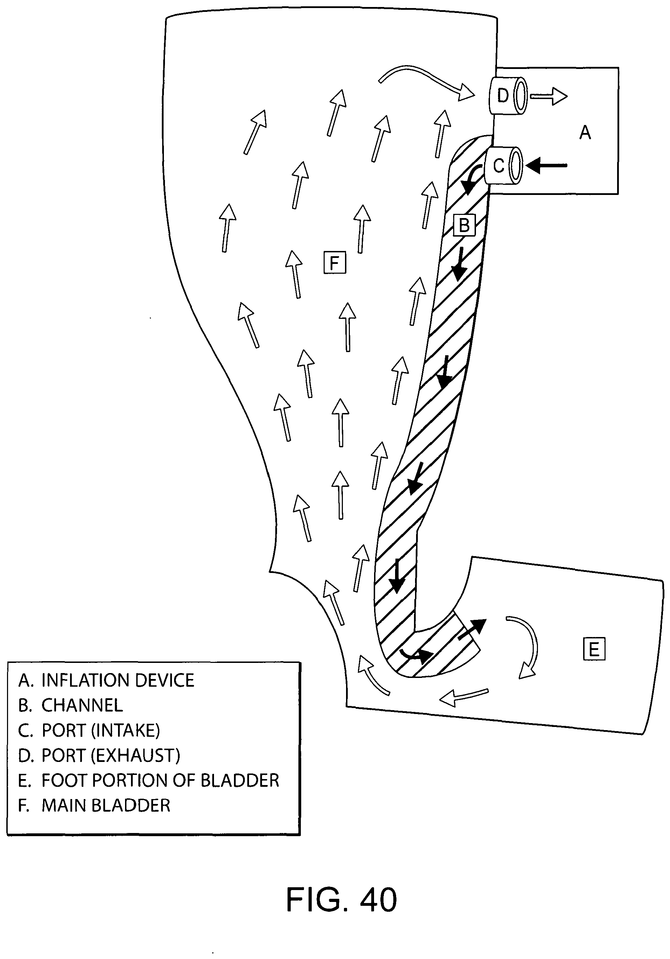

Another embedment of the present invention includes a Sequential Gradient Compression with Single Chamber. As shown in FIG. 40, the compression apparatus includes an inflation bladder to apply not only gradient compression but sequential (filling up of the foot portion first and making its way upwards) compression. In this embodiment as shown in FIG. 40, Inflation Device A is coupled to the device in two places (Intake Port C and Exhaust Port D). By inflating and providing air that goes directly into Channel B, the Foot Bladder Portion E inflates first, before the Main Bladder F. Air Then exits out of Exhaust Port D. In this design, the inflation can be intermittently provided for gradient and sequential compression or inflation can be held at a constant level to provide just the gradient profile.

Another embodiment of the present invention include an electric or other automated inflation means such that the bladder is inflated to a set volume or by reading the back pressure of which is being filled in. A pressure cycling function may be included. Further, an embodiment may have an inflation means such that the inflation maintains in the bladder(s) even after the inflation means is removed. Such inflation means may be integral to the compression apparatus itself or may be removable. Such inflation means may include an integrated circuit and/or wireless capability for tracking of usage, pressure, compliance by the patient in regard to maintaining certain pressures recommend by a physician or part of such patient's treatment plan, and other health data such as standing pressure and moving or working pressure, pedometer (number of steps), heartbeat, blood pressure and any other possible monitoring of the patient. Depending on the feedback obtained the inflation means may be programmed to increase or decrease the pressure without manual changing by the patient. Further, the inflation means may be configured so that the physician or other treatment professional may increase or decrease the pressure remotely based on the feedback. Other combinations may be included such as manual changing of the dial or inflation means in combination with automated means or electric means or digital means.

The dials shown are non-digital by way of non-limiting example only but a digital means may also be employed. A motorized pump and digital display may be used. The valve may include digital or electric means to change or modify pressure at a set rate or intervals or based on feedback from the monitoring means. The apparatus may include various sensors and monitors.

In use the therapeutic compression apparatus 10, 100, 200, 300 or 400 may be placed by the patient, practioner or care-giver on the chosen limb, such as for instance the lower leg on the calf and foot and the secondary wrap, or the foot wrap is fastened around the foot of the patient. The opening aperture for the ankle is set by the patient for comfort. Moving in an upward position from the foot then the patient, practioner or care-giver fastens or secures the fastening tabs (such as 32a, 32b, 32c, 32d, and 424, etc.) up to the knee. If there are additional straps located on the proximal end of the primary bladder or calf or leg bladder (near the knee) the first strap should be closed or secured in a tight fashion so that the therapeutic compression apparatus fits snugly but not too tight and the second strap near the distal end of the secondary wrap (near the foot) should be closed or secured in a tight fashion so that the therapeutic compression apparatus fits snugly but not too tight. The patient, practioner or care-giver then removes the valve cap from the valve located on the therapeutic compression apparatus such as for instance on the primary wrap. The patient, practioner or care-giver then selects a pressure amount of value on the dial of the inflation means depending on the treatment and whether the patient will be walking, sitting, lying down or traveling in a vehicle, train or airplane. Once the pressure amount or value is chosen on the dial (such as a given pressure amount such as "35" mm-Hg or a text such as "Walk" or "Air" or "Travel" or "Low" or "Medium" or "High"), the corresponding umbrella valve or switch is activated such that the pressure is thereafter maintained (closed position) or modified so as to maintain the pressure as it changes with the activity or altitude when in use). The patient, practioner or care-giver then inserts an end of the inflation means into the valve on the therapeutic compression apparatus and the air or fluid is increased to inflate the primary and/or secondary bladder and thus achieve a desired pressure amount or valve. Again this inflation means may be a hand pump, electric pump, battery-operated pump, remote controlled pump, air pump, gas pump, or any other known inflation means. A number or variety of inflation means can be employed such as a manual pump, hand pump, foot pump, mechanical pump, electrical pump, battery-operated pump, static pump, intermittent pump, varying pump, automatic pump, pneumatic pump, negative pressure pump, suction pump or vacuum, pulsing pump, or any other known or developed source of inflation so as to provide a certain pressure within the bladder so to provide compression in use by the patient. Depending on the inflation means employed such inflation means may be removed and the valve cap replaced and the pressure will not decrease except as noted in the "Air" or "Walk" position. At any point in use the patient, practioner or care-giver can deflate the primary and/or secondary bladders by either inserting the valve cap so it depresses the valve spring and thus release the air or fluid in the primary and/or secondary bladder and decrease the pressure, or the patient, practioner or care-giver can reinsert the inflation means and select the "Deflate" or "Release" and the corresponding umbrella valve will be in the open position so as to release the air or fluid in the primary and/or secondary bladder and decrease the pressure until a deflated state is achieved for the bladder and the therapeutic compression apparatus. The therapeutic compression apparatus can be reinflated and deflated over and over again when in use.

The present invention has been illustrated and described with respect to specific embodiments thereof, which embodiments are exemplary and illustrative of the principles of the invention and are not intended to be exclusive or otherwise limiting embodiments. For instance, while in the foregoing embodiments the therapeutic compression apparatus are described as having inflatable bladders, the therapeutic compression apparatus may additionally include integrally formed or attached (e.g., by adhesive, radio-frequency welding, etc.) compression members that are not configured for inflation and/or deflation. For instance, additional compression members may be implemented using any of a variety of preformed and/or prefilled cushioning materials such as foam cushions and/or air, gel, or other fluid filled non-inflatable cushions, provided such compression members generate sufficient compression in combination with integral compression bladders. Further, while particular shapes, sizes, and materials have been described for purposes of illustration, it will be recognized that any of a variety of shape or size can be used, and the materials described are not exclusive but merely illustrative. Also, as noted above, while the bladder shown is inflated with air, it will be appreciated that any other fluid or medium such as liquid or gel can be used. Moreover, as also noted, it will be understood that bladders may be configured to have multiple pneumatically independent and/or pneumatically coupled bladder sections, and may also be configured to have various contours or lobulations.

The therapeutic compression apparatus described herein can be used for any suitable condition treatable by compression therapy and the like. For example, therapeutic compression apparatus in accordance with the present invention can be used for compression of the venous system for the treatment of venous ulcers, CVI, DVT, for the treatment of lymphedema (where it is circulation of fluids in the lymph system rather than in the venous system that is promoted), and the like.

The therapeutic compression apparatus of the instant invention described herein solves many problems with the prior art and in the industry and treatment of patients. The therapeutic compression apparatus may be applied on the patient's body part by the patient without the need or requirement of a skilled care-giver as required by current devices and apparatus. It further is capable of maintaining sufficient effective pressure without overpressure complications, maintaining compression and the like.

The therapeutic compression apparatus of the instant invention includes a universal inflation port which is configured to be capable of connecting to more than one source of compression or inflation means such that the patient could vary treatment through varying the inflation source and inflation means for the treatment apparatus or device. For instance, a patient using The therapeutic compression apparatus of the instant invention can alternate between a manual or mechanical or electrical inflation means or source of inflation and pressure. Further, the patient can alternate between static or intermittent inflation and pressure when using the inventive therapeutic compression apparatus

The therapeutic compression apparatus of the instant invention also reduces the problem is leakage of set compression within the treatment apparatus and devices, bandages, stockings and hosiery and instead promotes a more effective treatment for CVI, DVT and/or lymphedema and other treatments.

While the subject invention of the present disclosure has been described with respect to preferred and exemplary embodiments, those skilled in the art will readily appreciate that various changes and/or modifications can be made to the invention without departing from the scope of the invention as described herein. There have been described and illustrated herein several embodiments of an intermittent pressure apparatus and a method of installing and operating same. While particular embodiments of the invention have been described, it is not intended that the invention be limited thereto, as it is intended that the invention be as broad in scope as the art will allow and that the specification be read likewise. Thus, while particular shapes and sizes of inflatable bladders and straps have been disclosed, it will be appreciated that other shapes, sizes, and attachment means may be used as well. It will also be understood that while Velcro and adhesive means have been disclosed for helping to secure the bladders to the leg and foot, other types of attachments such as hooks, snaps, or wraps may be used. In addition, it will be appreciated that while the fluid conduit may be detachably connected to the bladders using mating threaded portions or bayonet locks, other means of attachment known in the art may be used. It will therefore be appreciated by those skilled in the art that yet other modifications could be made to the provided invention without deviating from its scope as claimed.

## Claims

1. A therapeutic compression apparatus (10, 100, 200, 300, 400) comprising:
a primary wrap (12, 103, 403) having a proximal end and a distal end of the primary wrap (12, 103, 403);
a secondary wrap (14, 104, 404) having a proximal end and a distal end wherein the proximal end of the secondary wrap (14, 104, 404) is connected to the distal end of the primary wrap (12, 103, 403) creating an at least one bladder (22, 102, 402) within the primary wrap (12, 103, 403) and/or secondary wrap (14, 104, 404);
an inflation means (600, 700, 810, 900, 1000, 1100) capable of inflating the at least one bladder (22, 102, 402) to multiple set pressure amounts as reflected on a dial (610, 614, 818, 1030, 1110) of a switch, the inflation means (600, 700, 810, 900, 1000, 1100) connected to the at least one bladder (22, 102, 402) via a single inflation port (24, 112, 500) on the primary wrap (12, 103, 403), creating a closed system configured to prevent overinflation of the at least one bladder (22, 102, 402) wherein the inflation means (600, 700, 810, 900, 1000, 1100) includes at least two check valves (630, 830, 980, 1220) that prevent overinflation by releasing excess pressure in the at least one air bladder and the switch having an overinflation umbrella valve which is manually or automatically capable of opening to release excess pressure above a chosen set pressure amount on the dial (610, 614, 818, 1030, 1110)_and closing once the excess pressure is released to maintain the set amount of pressure in the at least one bladder (22, 102, 402); and
wherein the single inflation port (24, 112, 500) is a universal inflation port configured to be universally capable of connection to both a static and intermittent inflation means to provide static and/or intermittent compression to a body part of a user when the at least one bladder (22, 102, 402) is inflated by the inflation means (600, 700, 810, 900, 1000, 1100) with the single inflation port (24, 112, 500) including a check valve as part of the closed system configured so that when the inflation means is disconnected the single universal inflation port check valve closes and maintains the set pressure within the at least one air bladder (22, 102, 402) until released either manually or automatically.

2. The therapeutic compression apparatus of claim 1 wherein the primary wrap (12, 103, 403) includes (a) an inner sheet (106) configured and adapted to be disposed facing a limb when in use and (b) an outer sheet (108) joined to the inner sheet (106) with the at least one bladder (22, 102, 402) defined between the inner and outer sheets (106, 108).

3. The therapeutic compression apparatus of claim 1 wherein the at least one bladder (22, 102, 402) is configured to have one chamber (114, 214, 214', 214") capable of sequential gradient pressure when connected to the inflation means (600, 700, 810, 900, 1000, 1100) and wherein the switch is a three-way switch configured to be capable of changing among a first valve or switch, a second valve or switch and a third valve or switch with the first valve or switch configured to be capable of holding a pressure created by the inflation means (600, 700, 810, 900, 1000, 1100) within the at least one bladder (22, 102, 402) creating an inflated state of the bladder (22, 102, 402), the second valve or switch configured to be capable of releasing a set amount of pressure within the at least one bladder (22, 102, 402) creating a partially deflated state of the at least one bladder (22, 102, 402), and the third valve or switch configured to be capable of releasing all the pressure within the at least one bladder (22, 102, 402) so as to create a fully deflated state of the at least one bladder (22, 102, 402).

4. The therapeutic compression apparatus of claim 1 wherein the inflation means (600, 700, 810, 900, 1000, 1100) includes a real-time pressure measurement mechanism.

5. The therapeutic compression apparatus of claim 1 further comprising an elbow connector (510) and a valve cap (520) wherein the valve cap (520) is capable of releasing a pressure created by the inflation means (600, 700, 810, 900, 1000, 1100) within the at least one bladder (22, 102, 402).

6. The therapeutic compression apparatus of claim 1, wherein the single universal inflation port check valve is operatively connected to the at least one bladder to protect from overinflation.

7. The therapeutic compression apparatus of claim 1 further comprising at least two tabs (32a, 32b, 32c, 32d) connected either to the proximal end of the primary wrap (12, 103, 403) or the distal end of the secondary wrap (14, 104, 404) wherein the tabs include a connecting means configured to be capable of connecting at least two therapeutic compression apparatus (10, 100, 200, 300, 400) together along a plane.

8. The therapeutic compression apparatus of claim 1 for applying pressure to a leg of the human body wherein the at least one bladder (22, 102, 402)_includes a foot bladder (314, 314', 314") having a first inflatable chamber (314a, 314a', 314a") that accommodates a fluid entering therein by inflating and a leg bladder (312) having a second inflatable chamber (312a) that accommodates the fluid entering therein by inflating and a fluid conduit (316) fluidly coupling the foot bladder (314, 314', 314") and the leg bladder (312) such that a fluid may travel from the foot bladder (314, 314', 314") to the leg bladder (312) through the fluid conduit (316), and from the leg bladder (312) to the foot bladder (314, 314', 314") through the fluid conduit (316), the apparatus further including a first means for locating the leg bladder (312) on the leg of the human body and a second means for locating the foot bladder (314, 314', 314") on a foot of the human body.

9. The therapeutic compression apparatus of claim 8, wherein the first means for locating is a first attachment member wrapping around the leg to secure the leg bladder (312) to the leg wherein the first attachment member is either a plurality of straps attached to the leg bladder (312) or is a flexible member wrapping around the leg and over the leg bladder (312) wherein the leg bladder (312) is sandwiched between the leg and the flexible member and wherein the second means for locating is a second attachment member wrapping around the foot to secure the fluid foot bladder (314, 314', 314") to the foot and wherein the second attachment member is either a plurality of straps attached to the foot bladder (314, 314', 314") and have an adhesive or hook and loop closure for attaching the straps to the foot.

10. The therapeutic compression apparatus of claim 9, wherein the first attachment member includes a secondary air bladder, the flexible member has an encircling configuration that wraps around the leg, and an open unwrapped configuration, and the secondary air bladder is supported by the flexible member in the encircling configuration, the secondary air bladder capable of inflation to a pressurized state which supplies compressive forces that are directed radially inward to apply compressive forces to the leg when supported by the flexible member in the encircling configuration.

11. The therapeutic compression apparatus of claim 8, wherein the foot bladder (314, 314', 314") and the leg bladder (312) are arranged such that when the fluid is forced out of the foot bladder (314, 314', 314"), the leg bladder (312) applies an extra pressure to the leg and wherein the extra pressure is between 10 and 20 mm Hg extra pressure.

12. The therapeutic compression apparatus of claims 1 or 8 wherein the inflation means (600, 700, 810, 900, 1000, 1100) is selected from the group consisting of manual pumps, static pumps, intermittent pumps, electrical inflation pumps, battery inflation pumps, gas powered inflation pumps, static pneumatic compression pumps, intermittent pneumatic pressure pumps, and the combination thereof.

13. The therapeutic compression apparatus of claims 1 or 8, wherein the single universal inflation port check valve is either set to open at a predetermined pressure or is configured to open at a user selectable pressure.

14. The therapeutic compression apparatus of claims 1 or 8, further comprising a vent valve operatively connected to at least one bladder (22, 102, 312, 314, 402) for deflation.

15. The therapeutic compression apparatus of claims 1 or 8 further comprising a securing means (32a, 32b, 32c, 32d, 122, 124, 226A, 226B, 226A1', 226A2', 226A3', 226B', 356, 422, 424, 1324, 1424) to secure the therapeutic compression apparatus onto the body part of the user, the securing means (32a, 32b, 32c, 32d, 122, 124, 226A, 226B, 226A1', 226A2', 226A3', 226B', 356, 422, 424, 1324, 1424) selected from the group consisting of hook and loop fasteners, zippers and buttons.

## Patentansprüche

1. Kompressionstherapievorrichtung (10, 100, 200, 300, 400), umfassend:
eine Primärwicklung (12, 103, 403) mit einem proximalen Ende und einem distalen Ende der Primärwicklung (12, 103, 403),
eine Sekundärwicklung (14, 104, 404) mit einem proximalen Ende und einem distalen Ende, wobei das proximale Ende der Sekundärwicklung (14, 104, 404) mit dem distalen Ende der Primärwicklung (12, 103, 403) verbunden ist, wodurch eine mindestens eine Blase (22, 102, 402) in der Primärwicklung (12, 103, 403) und/oder der Sekundärwicklung (14, 104, 404) erzeugt wird,
ein Aufblasmittel (600, 700, 810, 900, 1000, 1100), das in der Lage ist, die mindestens eine Blase (22, 102, 402) auf mehrere eingestellte Druckwerte aufzublasen, wie auf einer Skala (610, 614, 818, 1030, 1110) eines Schalters angezeigt, wobei das Aufblasmittel (600, 700, 810, 900, 1000, 1100) über einen einzigen Aufblasanschluss (24, 112, 500) an der Primärwicklung (12, 103, 403) mit der mindestens einen Blase (22, 102, 402) verbunden ist, wodurch ein geschlossenes System erzeugt wird, das zum Verhindern eines Überfüllens der mindestens einen Blase (22, 102, 402) ausgestaltet ist, wobei das Aufblasmittel (600, 700, 810, 900, 1000, 1100) mindestens zwei Rückschlagventile (630, 830, 980, 1220) aufweist, die ein Überfüllen verhindern, indem Überdruck in der mindestens einen Luftblase abgelassen wird, und der Schalter ein Überdruck-Schirmventil aufweist, das manuell oder automatisch geöffnet werden kann, um Überdruck oberhalb eines gewählten eingestellten Druckwerts auf der Skala (610, 614, 818, 1030, 1110) abzulassen und geschlossen werden kann, sobald der Überdruck abgelassen worden ist, um den eingestellten Druckwert in der mindestens einen Blase (22, 102, 402) aufrechtzuerhalten, und
wobei der einzige Aufblasanschluss (24, 112, 500) ein universeller Aufblasanschluss ist, der dazu ausgestaltet ist, universell sowohl mit einem statischen als auch einem intermittierenden Aufblasmittel verbunden werden zu können, um statische und/oder intermittierende Kompression für einen Körperteil eines Benutzers bereitzustellen, wenn die mindestens eine Blase (22, 102, 402) durch das Aufblasmittel (600, 700, 810, 900, 1000, 1100) aufgeblasen wird, wobei der einzige Aufblasanschluss (24, 112, 500) ein Rückschlagventil als Teil des geschlossenen Systems umfasst, das so ausgestaltet ist, dass das einzige universelle Aufblasanschluss-Rückschlagventil schließt und den eingestellten Druck in der mindestens einen Luftblase (22, 102, 402) aufrechterhält, bis er entweder manuell oder automatisch abgelassen wird, wenn das Aufblasmittel abgekoppelt wird.

2. Kompressionstherapievorrichtung nach Anspruch 1, wobei die Primärwicklung (12, 103, 403) (a) eine innere Folie (106), die so ausgestaltet und ausgeführt ist, dass sie im Gebrauch einer Gliedmaße zugewandt angeordnet wird, und (b) eine äußere Folie (108) aufweist, die mit der inneren Folie (106) verbunden ist, wobei die mindestens eine Blase (22, 102, 402) zwischen der inneren und der äußeren Folie (106, 108) definiert ist.

3. Kompressionstherapievorrichtung nach Anspruch 1, wobei die mindestens eine Blase (22, 102, 402) so ausgestaltet ist, dass sie eine Kammer (114, 214, 214', 214") aufweist, die zur Erzeugung eines sequentiellen Druckgradienten in der Lage ist, wenn sie mit dem Aufblasmittel (600, 700, 810, 900, 1000, 1100) verbunden ist, und wobei der Schalter ein Dreiwegeschalter ist, der so ausgestaltet ist, dass er zwischen einem ersten Ventil oder Schalter, einem zweiten Ventil oder Schalter und einem dritten Ventil oder Schalter wechseln kann, wobei das erste Ventil oder der erste Schalter so ausgestaltet ist, dass er bzw. es einen durch das Aufblasmittel (600, 700, 810, 900, 1000, 1100) in der mindestens einen Blase (22, 102, 402) erzeugten Druck halten kann, wodurch ein aufgeblasener Zustand der Blase (22, 102, 402) erzeugt wird, das zweite Ventil oder der zweite Schalter so ausgestaltet ist, dass er bzw. es einen eingestellten Druckwert in der mindestens einen Blase (22, 102, 402) ablassen kann, wodurch ein teilweise abgelassener Zustand der mindestens einen Blase (22, 102, 402) erzeugt wird, und das dritte Ventil oder der dritte Schalter so ausgestaltet ist, dass er bzw. es den gesamten Druck in der mindestens einen Blase (22, 102, 402) ablassen kann, um einen vollständig abgelassenen Zustand der mindestens einen Blase (22, 102, 402) zu erzeugen.

4. Kompressionstherapievorrichtung nach Anspruch 1, wobei das Aufblasmittel (600, 700, 810, 900, 1000, 1100) einen Echtzeit-Druckmessmechanismus aufweist.

5. Kompressionstherapievorrichtung nach Anspruch 1, ferner umfassend einen Winkelanschluss (510) und eine Ventilkappe (520), wobei die Ventilkappe (520) in der Lage ist, einen durch das Aufblasmittel (600, 700, 810, 900, 1000, 1100) in der mindestens einen Blase (22, 102, 402) erzeugten Druck abzulassen.

6. Kompressionstherapievorrichtung nach Anspruch 1, wobei das einzige universelle Aufblasanschluss-Rückschlagventil zum Schutz vor Überfüllung mit der mindestens einen Blase wirkverbunden ist.

7. Kompressionstherapievorrichtung nach Anspruch 1, ferner umfassend mindestens zwei Laschen (32a, 32b, 32c, 32d), die entweder mit dem proximalen Ende der Primärwicklung (12, 103, 403) oder dem distalen Ende der Sekundärwicklung (14, 104, 404) verbunden sind, wobei die Laschen ein Verbindungsmittel aufweisen, das dazu ausgestaltet ist, mindestens zwei Kompressionstherapievorrichtungen (10, 100, 200, 300, 400) entlang einer Ebene miteinander verbinden zu können.

8. Kompressionstherapievorrichtung nach Anspruch 1 zum Ausüben von Druck auf ein Bein des menschlichen Körpers, wobei die mindestens eine Blase (22, 102, 402) eine Fußblase (314, 314', 314") mit einer ersten aufblasbaren Kammer (314a, 314a', 314a"), die ein darin durch Aufblasen eintretendes Fluid aufnimmt, und eine Beinblase (312) mit einer zweiten aufblasbaren Kammer (312a), die das darin durch Aufblasen eintretende Fluid aufnimmt, und eine Fluidleitung (316) aufweist, die die Fußblase (314, 314', 314") und die Beinblase (312) fluidisch koppelt, so dass ein Fluid von der Fußblase (314, 314', 314") durch die Fluidleitung (316) zu der Beinblase (312) strömen kann und von der Beinblase (312) durch die Fluidleitung (316) zu der Fußblase (314, 314', 314"), wobei die Vorrichtung ferner ein erstes Mittel zum Anordnen der Beinblase (312) an dem Bein des menschlichen Körpers und ein zweites Mittel zum Anordnen der Fußblase (314, 314', 314") an einem Fuß des menschlichen Körpers aufweist.

9. Kompressionstherapievorrichtung nach Anspruch 8, wobei das erste Mittel zum Anordnen ein erstes Befestigungselement ist, das um das Bein gewickelt ist, um die Beinblase (312) an dem Bein zu befestigen, wobei das erste Befestigungselement entweder eine Vielzahl von Riemen ist, die an der Beinblase (312) befestigt sind, oder ein flexibles Element ist, das um das Bein und über die Beinblase (312) gewickelt ist, wobei die Beinblase (312) zwischen dem Bein und dem flexiblen Element angeordnet ist und wobei das zweite Mittel zum Anordnen ein zweites Befestigungselement ist, das sich um den Fuß wickelt, um die Fluidfußblase (314, 314', 314") an dem Fuß zu befestigen, und wobei das zweite Befestigungselement entweder eine Vielzahl von Riemen ist, die an der Fußblase (314, 314', 314" befestigt sind und einen Klebe- oder Klettverschluss zum Befestigen der Riemen an dem Fuß aufweisen.

10. Kompressionstherapievorrichtung nach Anspruch 9, wobei das erste Befestigungselement eine sekundäre Luftblase aufweist, das flexible Element eine umlaufende Konfiguration, die sich um das Bein wickelt, und eine offene ungewickelte Konfiguration aufweist, und die Sekundärluftblase von dem flexiblen Element in der umlaufenden Konfiguration gestützt, wobei die Sekundärluftblase in einen druckbeaufschlagten Zustand aufgeblasen werden kann, der Kompressionskräfte bereitstellt, die radial nach innen gerichtet sind, um Kompressionskräfte auf das Bein auszuüben, wenn es durch das flexible Element in der umlaufenden Konfiguration gestützt wird.

11. Kompressionstherapievorrichtung nach Anspruch 8, wobei die Fußblase (314, 314', 314") und die Beinblase (312) so angeordnet sind, dass die Beinblase (312) einen zusätzlichen Druck auf das Bein ausübt, wenn das Fluid aus der Fußblase (314, 314', 314") gedrückt wird, und wobei der zusätzliche Druck einen zusätzlichen Druck von 10 bis 20 mmHg beträgt.

12. Kompressionstherapievorrichtung nach Anspruch 1 oder 8, wobei das Aufblasmittel (600, 700, 810, 900, 1000, 1100) aus der Gruppe bestehend aus Handpumpen, statischen Pumpen, intermittierenden Pumpen, elektrischen Aufblaspumpen, Batterieaufblaspumpen, gasbetriebene Aufblaspumpen, statische pneumatische Kompressionspumpen, intermittierende pneumatische Druckpumpen und deren Kombination ausgewählt ist.

13. Kompressionstherapievorrichtung nach Anspruch 1 oder 8, wobei das einzige universelle Aufblasanschluss-Rückschlagventil entweder so eingestellt ist, dass es sich bei einem vorbestimmten Druck öffnet, oder so ausgestaltet ist, dass es sich bei einem vom Benutzer wählbaren Druck öffnet.

14. Kompressionstherapievorrichtung nach Anspruch 1 oder 8, ferner umfassend ein Entlüftungsventil, das mit mindestens einer Blase (22, 102, 312, 314, 402) zum Ablassen wirkverbunden ist.

15. Kompressionstherapievorrichtung nach Anspruch 1 oder 8, ferner umfassend ein Befestigungsmittel (32a, 32b, 32c, 32d, 122, 124, 226A, 226B, 226A1', 226A2', 226A3', 226B', 356, 422, 424, 1324, 1424), um die Kompressionstherapievorrichtung an dem Körperteil des Benutzers zu befestigen, wobei das Befestigungsmittel (32a, 32b, 32c, 32d, 122, 124, 226A, 226B, 226A1', 226A2', 226A3', 226B', 356, 422, 424, 1324, 1424) aus der Gruppe bestehend aus Klettverschlüssen, Reißverschlüssen und Knöpfen ausgewählt ist.

## Revendications

1. Appareil de compression thérapeutique (10, 100, 200, 300, 400) comprenant :
une enveloppe primaire (12, 103, 403) ayant une extrémité proximale et une extrémité distale de l'enveloppe primaire (12, 103, 403) ;
une enveloppe secondaire (14, 104, 404) ayant une extrémité proximale et une extrémité distale, l'extrémité proximale de l'enveloppe secondaire (14, 104, 404) étant reliée à l'extrémité distale de l'enveloppe primaire (12, 103, 403) créant au moins une vessie (22, 102, 402) à l'intérieur de l'enveloppe primaire (12, 103, 403) et/ou de l'enveloppe secondaire (14, 104, 404) ;
un moyen de gonflage (600, 700, 810, 900, 1000, 1100) capable de gonfler l'au moins une vessie (22, 102, 402) à de multiples quantités de pression réglées telles que reflétées sur un cadran (610, 614, 818, 1030, 1110) d'un commutateur, le moyen de gonflage (600, 700, 810, 900, 1000, 1100) étant relié à l'au moins une vessie (22, 102, 402) par l'intermédiaire d'un unique orifice de gonflage (24, 112, 500) sur l'enveloppe primaire (12, 103, 403), créant un système fermé configuré pour empêcher un surgonflage de l'au moins une vessie (22, 102, 402), le moyen de gonflage (600, 700, 810, 900, 1000, 1100) comprenant au moins deux clapets anti-retour (630, 830, 980, 1220) qui empêchent le surgonflage en relâchant la pression excessive dans l'au moins une vessie à air et le commutateur possédant un clapet parapluie de surgonflage qui est capable manuellement ou automatiquement de s'ouvrir pour relâcher la pression excessive au-dessus d'une quantité de pression réglée choisie sur le cadran (610, 614, 818, 1030, 1110) et de se fermer une fois que la pression excessive est relâchée pour maintenir la quantité de pression réglée dans l'au moins une vessie (22, 102, 402) ; et
l'orifice de gonflage unique (24, 112, 500) étant un orifice de gonflage universel configuré pour être universellement capable de se relier à la fois à un moyen de gonflage statique et intermittent afin de fournir une compression statique et/ou intermittente à une partie du corps d'un utilisateur lorsque l'au moins une vessie (22, 102, 402) est gonflée par le moyen de gonflage (600, 700, 810, 900, 1000, 1100), l'orifice de gonflage unique (24, 112, 500) comprenant un clapet anti-retour faisant partie du système fermé configuré de telle sorte que, lorsque le moyen de gonflage est déconnecté, le clapet anti-retour à orifice de gonflage universel unique se ferme et maintient la pression réglée dans l'au moins une vessie à air (22, 102, 402) jusqu'à ce qu'elle soit relâchée manuellement ou automatiquement.

2. Appareil de compression thérapeutique selon la revendication 1, l'enveloppe primaire (12, 103, 403) comprenant (a) une feuille interne (106) configurée et adaptée pour être disposée face à un membre lors de l'utilisation et (b) une feuille externe (108) jointe à la feuille interne (106) avec l'au moins une vessie (22, 102, 402) définie entre les feuilles interne et externe (106, 108).

3. Appareil de compression thérapeutique selon la revendication 1, l'au moins une vessie (22, 102, 402) étant configurée pour avoir une chambre (114, 214, 214', 214") capable d'une pression de gradient séquentielle lorsqu'elle est reliée au moyen de gonflage (600, 700, 810, 900, 1000, 1100) et le commutateur étant un commutateur à trois voies configuré pour être capable de basculer parmi une première vanne ou un premier commutateur, une deuxième vanne ou un deuxième commutateur et une troisième vanne ou un troisième commutateur, la première vanne ou le premier commutateur étant configuré(e) pour être capable de maintenir une pression créée par le moyen de gonflage (600, 700, 810, 900, 1000, 1100) au sein de l'au moins une vessie (22, 102, 402) créant un état gonflé de la vessie (22, 102, 402), la deuxième vanne ou le deuxième commutateur étant configuré(e) pour être capable de relâcher une quantité de pression réglée dans l'au moins une vessie (22, 102, 402) créant un état partiellement dégonflé de l'au moins une vessie (22, 102, 402), et la troisième vanne ou le troisième commutateur étant configuré(e) pour être capable de relâcher toute la pression à l'intérieur de l'au moins une vessie (22, 102, 402) de manière à créer un état complètement dégonflé de l'au moins une vessie (22, 102, 402).

4. Appareil de compression thérapeutique selon la revendication 1, le moyen de gonflage (600, 700, 810, 900, 1000, 1100) comportant un mécanisme de mesure de pression en temps réel.

5. Appareil de compression thérapeutique selon la revendication 1, comprenant en outre un raccord coudé (510) et un capuchon de valve (520), le capuchon de valve (520) étant capable de relâcher une pression créée par le moyen de gonflage (600, 700, 810, 900, 1000, 1100) à l'intérieur de l'au moins une vessie (22, 102, 402).

6. Appareil de compression thérapeutique selon la revendication 1, le clapet anti-retour à orifice de gonflage universel unique étant relié de manière fonctionnelle à l'au moins une vessie pour la protéger contre le surgonflage.

7. Appareil de compression thérapeutique selon la revendication 1, comprenant en outre au moins deux pattes (32a, 32b, 32c, 32d) reliées soit à l'extrémité proximale de l'enveloppe primaire (12, 103, 403), soit à l'extrémité distale de l'enveloppe secondaire (14, 104, 404), les pattes comprenant un moyen de connexion configuré pour être capable de connecter au moins deux appareils de compression thérapeutique (10, 100, 200, 300, 400) ensemble le long d'un plan.

8. Appareil de compression thérapeutique selon la revendication 1, pour appliquer une pression sur une jambe du corps humain, l'au moins une vessie (22, 102, 402) comprenant une vessie pour pied (314, 314', 314") ayant une première chambre gonflable (314a, 314a', 314a") qui reçoit un fluide entrant dans celle-ci par gonflage et une vessie pour jambe (312) ayant une deuxième chambre gonflable (312a) qui reçoit le fluide entrant dans celle-ci par gonflage et un conduit de fluide (316) couplant fluidiquement la vessie pour pied (314, 314', 314") et la vessie pour jambe (312) de telle sorte qu'un fluide peut se déplacer de la vessie pour pied (314, 314', 314") vers la vessie pour jambe (312) à travers le conduit de fluide (316), et de la vessie pour jambe (312) vers la vessie pour pied (314, 314', 314") à travers le conduit de fluide (316), l'appareil comprenant en outre un premier moyen de positionnement de la vessie pour jambe (312) sur la jambe du corps humain et un deuxième moyen de positionnement de la vessie pour pied (314, 314', 314") sur un pied du corps humain.

9. Appareil de compression thérapeutique selon la revendication 8, le premier moyen de positionnement étant un premier élément de fixation s'enroulant autour de la jambe pour fixer la vessie pour jambe (312) à la jambe, le premier élément de fixation étant soit une pluralité de sangles fixées à la vessie pour jambe (312), soit un élément flexible s'enroulant autour de la jambe et au-dessus de la vessie pour jambe (312), la vessie pour jambe (312) étant prise en sandwich entre la jambe et l'élément flexible et le deuxième moyen de positionnement étant un deuxième élément de fixation s'enroulant autour du pied pour fixer la vessie pour pied (314, 314', 314") au pied, le deuxième élément de fixation étant soit une pluralité de sangles fixées à la vessie pour pied (314, 314', 314") et possédant un adhésif ou une fermeture auto-agrippante pour fixer les sangles au pied.

10. Appareil de compression thérapeutique selon la revendication 9, le premier élément de fixation comprenant une vessie d'air secondaire, l'élément flexible ayant une configuration encerclante qui s'enroule autour de la jambe, et une configuration ouverte non enroulée, et la vessie d'air secondaire étant supportée par l'élément flexible dans la configuration encerclante, la vessie d'air secondaire étant capable de se gonfler à un état pressurisé qui fournit des forces de compression dirigées radialement vers l'intérieur pour appliquer des forces de compression à la jambe lorsqu'elle est supportée par l'élément flexible dans la configuration encerclante.

11. Appareil de compression thérapeutique selon la revendication 8, la vessie pour pied (314, 314', 314") et la vessie pour jambe (312) étant agencées de telle sorte que lorsque le fluide est expulsé de la vessie pour pied (314, 314', 314"), la vessie pour jambe (312) applique une pression supplémentaire à la jambe et la pression supplémentaire étant comprise entre 10 et 20 mm Hg.

12. Appareil de compression thérapeutique selon les revendications 1 ou 8, le moyen de gonflage (600, 700, 810, 900, 1000, 1100) étant choisi dans le groupe constitué par les pompes manuelles, les pompes statiques, les pompes intermittentes, les pompes de gonflage électriques, les pompes de gonflage à batterie, les pompes de gonflage à gaz, les pompes à compression pneumatiques statiques, les pompes à pression pneumatique intermittente et des combinaisons de celles-ci.

13. Appareil de compression thérapeutique selon la revendication 1 ou 8, le clapet anti-retour à orifice de gonflage universel unique étant soit réglé pour s'ouvrir à une pression prédéterminée, soit configuré pour s'ouvrir à une pression sélectionnable par l'utilisateur.

14. Appareil de compression thérapeutique selon la revendication 1 ou 8, comprenant en outre une soupape d'évacuation reliée de manière fonctionnelle à au moins une vessie (22, 102, 312, 314, 402) pour le dégonflage.

15. Appareil de compression thérapeutique selon la revendication 1 ou 8, comprenant en outre un moyen de fixation (32a, 32b, 32c, 32d, 122, 124, 226A, 226B, 226A1', 226A2', 226A3', 226B', 356, 422, 424, 1324, 1424) pour fixer l'appareil de compression thérapeutique sur la partie corporelle de l'utilisateur, le moyen de fixation (32a, 32b, 32c, 32d, 122, 124, 226A, 226B, 226A1', 226A2', 226A3', 226B', 356, 422, 424, 1324, 1424) étant choisi dans le groupe constitué de fermetures auto-agrippantes, de fermetures à glissière et de boutons.
